# EUROPEAN PATENT APPLICATION

(11) **EP 1 908 761 A1**
(43) Date of publication of application: **09.04.2008**
(21) Application number: 06121769.1
(22) Date of filing: 04.10.2006
(51) Int. Cl.: C07D 413/12, C07D 401/12, C07D 211/40, C07D 413/14, A61K 31/451, A61K 31/538, A61P 9/00, A61P 27/06

(54) **Organic compounds**

(71) Applicant: Speedel Experimenta AG, 4123 Allschwil (CH)
(72) Inventor: The designation of the inventor has not yet been filed
(74) Representative: Maué, Paul Georg

(57) **Abstract**

The present invention relates to substituted piperidines of the general formula (I), 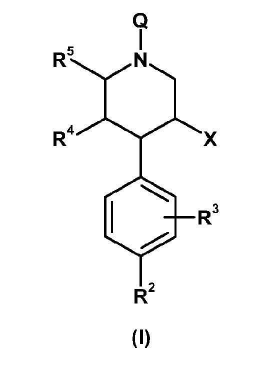 where R¹, R², R³, R⁴, R⁵, Q and X have the definitions elucidated in more detail in the description, to a process for their preparation and to the use of these compounds as medicines, in particular as renin inhibitors. Moreover, the enzymatic substrate portion of the compound is simultaneously a substrate for a membrane transporter.

## Description

### Field of the Invention

The present invention relates to substituted piperidines, to processes for their preparation and to the use of the compounds as medicaments, in particular as renin inhibitors. Moreover, a process for enhancing the membrane transportation of such compounds is provided.

### Background of the Invention

It was long thought that intestinal absorption of most drugs proceeded by passive diffusion, in which the lipid solubility of the drug molecule was the determining factor. However, many water-soluble compounds have been shown to move well across cell membranes utilizing specialized carrier-mediated transport mechanisms. These membrane transporters play a key role in determining exposure of cells or organisms to a variety of solutes including nutrients and cellular by-products, as well as drug molecules. Efforts have been made to improve drug bioavailability by using different pro-moieties targeting various active transportation systems present in the small intestine. Examples of transportation systems include peptide transporters, organic cation transporters, organic anion transporters, glucose transporters, vitamin transporters, bile acid transporters, fatty acid transporters, phosphate transporters, monocarboxylic acid transporters, bicarbonate transporters, ABC transporters, nucleoside transporters and amino acid transporters, as described by H.-C. Shi et al. in: R. Mannhold, H. Kubinyi, G. Folkers, Eds., Methods and Principles in Medicinal Chemistry, Wiley-VCH, Weinheim, 2003, pp. 245-287, herein incorporated by reference. All of these transporters are mainly located in the brush border membrane with variable distribution along the gastrointestinal tract, and show diverse substrate specificities.

The peptide transporter-1 (PEPT1) is known to play a critical role in the absorption of diverse drugs and prodrugs from the intestinal tract. PEPT1 is located in the apical enterocytic membrane of the upper small intestine where it serves as a symporter, using an electrochemical proton gradient as its driving force. Besides the intestinal tract, PEPT1 is also expressed in the pancreas, bile ducts and kidneys, where it may play a role in the re-uptake of filtered peptides. Human PEPT1 (hPEPT1) contains 708 amino acids oriented in 12 membrane-spanning domains.

Many pharmaceuticals are known to utilize the intestinal PEPT1 to gain entry into the systematic circulation. Such pharmaceuticals include β-lactam antibiotics such as penicillins and cephalosporins, ACE-inhibitors, second generation peptidic renin inhibitors, thrombin inhibitors and the dipeptide-like anti-neoplastic drug bestatin, as well as prodrugs of ganciclovir, L-Dopa and pamidronate.

Renin (EC 3.4.99.19) is a member of the well-studied family of aspartic proteinases. It controls the first and rate-limiting step of the renin-angiotensin system catalyzing the cleavage of the Leu10-Val11 peptide bond of angiotensinogen to release the decapeptide angiotensin I. A large variety of peptidic inhibitors of human renin with different transition-state analogs of the scissile peptide bond have been developed in the past 3 decades. As none of these compounds has survived all stages of drug development, newer classes (third generation) of non-peptide renin inhibitors have recently been reported, for example, in

EP 678503. Piperidine derivatives for use as medicaments are disclosed, for example, by WO 1997/09311. However, with regard especially to renin inhibition, there is still a need for highly potent active ingredients. In this context, the improvement of the pharmacokinetic properties is at the forefront. These properties directed to better bioavailability are, for example, absorption, metabolic stability, solubility or lipophilicity.

We have found unexpectedly that certain amino acid derivatives of third generation, non-peptidic piperidine-based renin inhibitors have increased bioavailabilty, in particular oral bioavailability. These derivatives are comprised of a chemical moiety attached to substituted piperidines, whereby the chemical moiety promotes uptake of the compound by a gastrointestinal tract membrane transporter, or other membrane transporter, thereby enhancing oral bioavailability. Examples of membrane transporters include peptide transporters, organic cation transporters, organic anion transporters, glucose transporters, vitamin transporters, bile acid transporters, fatty acid transporters, phosphate transporters, monocarboxylic acid transporters, bicarbonate transporters, ABC transporters, nucleoside transporters and amino acid transporters. Some of the third generation, non-peptidic piperidine-based renin inhibitors have been disclosed in WO 2006/005741.

### Detailed Description of the Invention

The invention therefore provides substituted piperidines of the general formula (I) 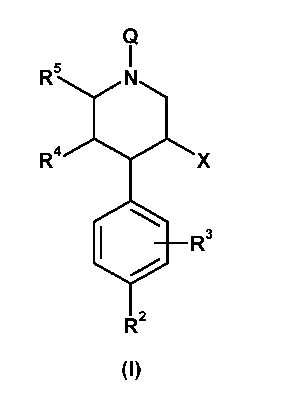 and the salts thereof, preferably the pharmaceutically acceptable salts thereof; where
R¹ is aryl or heterocyclyl, especially benzoimidazolyl, benzo[1,3]dioxolyl, benzofuranyl, benzooxazolyl, benzothiazolyl, benzo[b]thienyl, 2H-chromenyl, dihydro-2H-benzo[1,4]oxazinyl, dihydro-3H-benzo[1,4]oxazinyl, dihydro-2H-benzo[1,4]thiazinyl, 2,3-dihydroindolyl, dihydro-1H-pyrido[2,3-b][1,4]oxazinyl, imidazo[1,2-a]pyridyl, imidazo[1,5-a]pyridyl, indazolyl, indolyl, isobenzofuranyl, isoquinolyl, [1,5]naphthyridyl, phenyl, phthalazinyl, pyridyl, pyrimidinyl, 1 H-pyrrolo[2,3-b]pyridyl, 1 H-pyrrolo[2,3-c]pyridyl, 1H-pyrrolo[3,2-b]pyridyl, quinazolinyl, quinolyl, quinoxalinyl, tetrahydroimidazo[1,2-a]pyridyl, tetrahydroimidazo[1,5-a]pyridyl, tetrahydroisoquinolyl, tetrahydroquinolyl, tetrahydroquinoxalinyl, [1,2,3]triazolo[1,5-a]pyridyl or [1,2,4]triazolo[4,3-a]pyridyl, each of which is substituted by 1-4 acyl-C₁₋₈-alkoxy-C₁₋₈-alkoxy, acyl-C₁₋₈-alkoxy-C₁₋₈-alkyl, (N-acyl)-C₁₋₈-alkoxy-C₁₋₈-alkylamino, C₁₋₈-alkanoyl, C₁₋₈-alkoxy, C₁₋₈-alkoxy-C₁₋₈-alkanoyl, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, (N-C₁₋₈-alkoxy)-C₁₋₈-alkylaminocarbonyl-C₁₋₈-alkoxy, (N-C₁₋₈-alkoxy)-C₁₋₈-alkylaminocarbonyl-C₁₋₈-alkyl, C₁₋₈-alkoxy-C₁₋₈-alkyl-carbamoyl, C₁₋₈-alkoxy-C₁₋₈-alkyl-carbonylamino, C₁₋₈-alkoxy-C₁₋₈-alkyl-heterocyclyl, C₁₋₈-alkoxy-carbonyl, C₁₋₈-alkoxycarbonyl-C₁₋₈-alkoxy, C₁₋₈-alkoxycarbonyl-C₁₋₈-alkyl, C₁₋₈-alkoxycarbonylamino-C₂₋₈-alkoxy, C₁₋₈-alkoxycarbonylamino-C₁₋₈-alkyl, C₁₋₈-alkyl, (N-C₁₋₈-alkyl)-C₁₋₈-alkoxy-C₁₋₈-alkyl-carbamoyl, (N-C₁₋₈-alkyl)-C₁₋₈-alkoxy-C₁₋₈-alkylcarbonylamino, (N-C₁₋₈-alkyl)-C₁₋₈-alkoxy-carbonylamino, (N-C₁₋₈-alkyl)-C₁₋₈-alkylcarbonylamino-C₂₋₈-alkoxy, (N-C₁₋₈-alkyl)-C₁₋₈-alkylcarbonylamino-C₁₋₈-alkyl, (N-C₁₋₈-alkyl)-C₁₋₈-alkylsulfonylamino-C₂₋₈-alkoxy, (N-C₁₋₈-alkyl)-C₁₋₈-alkylsulfonylamino-C₁₋₈-alkyl, C₁₋₈-alkyl-amidinyl, C₁₋₈-alkylamino-C₂₋₈-alkoxy, di-C₁₋₈-alkylamino-C₂₋₈-alkoxy, C₁₋₈-alkylamino-C₁₋₈-alkyl, di-C₁₋₈-alkylamino-C₁₋₈-alkyl, C₁₋₈-alkylaminocarbonyl-C₁₋₈-alkoxy, di-C₁₋₈-alkylaminocarbonyl-C₁₋₈-alkoxy, C₁₋₈-alkylaminocarbonyl-C₁₋₈-alkoxy-C₁₋₈-alkyl, C₁₋₈-alkylaminocarbonyl-C₁₋₈-alkyl, di-C₁₋₈-alkylaminocarbonyl-C₁₋₈-alkyl, C₁₋₈-alkylaminocarbonylamino-C₂₋₈-alkoxy, C₁₋₈-alkylamino-carbonylamino-C₁₋₈-alkyl, C₁₋₈-alkylcarbonylamino, C₁₋₈-alkylcarbonylamino-C₂₋₈-alkoxy, C₁₋₈-alkylcarbonylamino-C₁₋₈-alkyl, C₁₋₈-alkylcarbonyloxy-C₂₋₈-alkoxy, C₁₋₈-alkylcarbonyloxy-C₁₋₈-alkyl, C₁₋₈-alkylsulfonyl, C₁₋₈-alkylsulfonyl-C₁₋₈-alkoxy, C₁₋₈-alkylsulfonyl-C₁₋₈-alkyl, C₁₋₈-alkylsulfonylamino-C₂₋₈-alkoxy, C₁₋₈-alkylsulfonylamino-C₁₋₈-alkyl, optionally N-mono- oder N,N-di-C₁₋₈-alkylated amino, aryl-C₀₋₈-alkoxy, aryl-C₀₋₈-alkyl, optionally N-mono- oder N,N-di-C₁₋₈-alkylated carbamoyl-C₀₋₈-alkoxy, carboxy-C₁₋₈-alkoxy, carboxy-C₁₋₈-alkoxy-C₁₋₈-alkyl, carboxy-C₁₋₈-alkyl, C₁₋₈-alkyl, cyano-C₂₋₈-alkoxy, cyano-C₁₋₈-alkyl, C₃₋₈-cycloalkyl-C₁₋₈-alkoxy, C₃₋₈-cycloalkyl-carbamoyl-C₀₋₈-alkyl, C₃₋₈-cycloalkylcarbonylamino-C₂₋₈-alkoxy, C₃₋₈-cyclo-alkylcarbonylamino-C₁₋₈-alkyl, O,N-dimethylhydroxylamino-C₁₋₈-alkyl, halogen, halogen-C₁₋₈-alkoxy, halogen-C₁₋₈-alkyl, heterocyclyl-C₀₋₈-alkoxy, heterocyclyl-C₀₋₈-alkyl, heterocyclyl-carbonyl, hydroxy-C₂₋₈-alkoxy-C₁₋₈-alkoxy, hydroxy-C₂₋₈-alkoxy-C₁₋₈-alkyl, hydroxy-C₁₋₈-alkyl, O-methyloximyl-C₁₋₈-alkyl, oxide or oxo;
R² is C₂₋₈-alkenyloxy, C₂₋₈-alkenyloxy-C₁₋₈-alkoxy, C₂₋₈-alkenyloxy-C₁₋₈-alkyl, C₁₋₈-alkoxy, C₁₋₈-alkoxy-C₁₋₈-alkoxy, optionally halogen-substituted C₁₋₈-alkoxy-C₁₋₈-alkoxy-C₁₋₈-alkoxy, optionally halogen-substituted C₁₋₈-alkoxy-C₁₋₈-alkoxy-C₁₋₈-alkyl, C₁₋₈-alkoxy-C₁₋₈-alkyl, C₁₋₈-alkoxy-C₂₋₈-alkylamino-C₂₋₈-alkoxy, C₁₋₈-alkoxy-C₂₋₈-alkylamino-C₁₋₈-alkyl, C₁₋₈-alkoxy-C₀₋₈-alkyl-C₃₋₈-cycloalkyl-C₀₋₈-alkoxy-C₁₋₈-alkoxy, C₁₋₈-alkoxy-C₀₋₈-alkyl-C₃₋₈-cycloalkyl-C₀₋₈-alkoxy-C₁₋₈-alkyl, C₁₋₈-alkoxy-C₂₋₈-alkylsulfanyl, C₁₋₈-alkoxy-C₂₋₈-alkylsulfanyl-C₂₋₈-alkoxy, C₁₋₈-alkoxy-C₂₋₈-alkylsulfanyl-C₁₋₈-alkyl, C₁₋₈-alkyl, C₁₋₈-alkylsulfanyl-C₂₋₈-alkoxy, C₁₋₈-alkylsulfanyl-C₂₋₈-alkoxy-C₁₋₈-alkoxy, C₁₋₈-alkylsulfanyl-C₂₋₈-alkoxy-C₁₋₈-alkyl, C₁₋₈-alkylsulfanyl-C₁₋₈-alkyl, C₁₋₈-alkylsulfonyl-C₂₋₈-alkoxy-C₁₋₈-alkoxy, C₁₋₈-alkylsulfonyl-C₂₋₈-alkoxy-C₁₋₈-alkyl, aryl-C₀₋₈-alkoxy, aryl-C₀₋₈-alkoxy-C₁₋₈-alkoxy, aryl-C₀₋₈-alkoxy-C₁₋₈-alkyl, C₃₋₈-cycloalkyl-C₀₋₈-alkoxy-C₁₋₈-alkoxy, C₃₋₈-cycloalkyl-C₀₋₈-alkoxy-C₁₋₈-alkoxy-C₁₋₈-alkoxy, C₃₋₈-cycloalkyl-C₀₋₈-alkoxy-C₁₋₈-alkoxy-C₁₋₈-alkyl, C₃₋₈-cycloalkyl-C₀₋₈-alkoxy-C₁₋₈-alkyl, heterocydyl-C₀₋₈-alkoxy, heterocyclyl-C₀₋₈-alkoxy-C₁₋₈-alkoxy, heterocyclyl-C₀₋₈-alkoxy-C₁₋₈-alkyl or heterocyclyl;
R³ is hydrogen or one or two halogen;
R⁴ is
(a) hydrogen
   or, when R⁵ is hydrogen, is
(b) optionally halogen- and/or hydroxy-substituted C₁₋₈-alkoxy, optionally halogen- and/or hydroxy-substituted C₁₋₈-alkoxy-C₁₋₈-alkoxy, optionally N-mono- or N,N-di-C₁₋₈-alkylated amino-C₂₋₈-alkoxy, optionally N-mono- or N,N-di-C₁₋₈-alkylated or optionally hydroxy-substituted amino-C₀₋₈-alkylcarbonyl-C₁₋₈-alkoxy, hydroxy-C₁₋₈-alkylcarbonyl-C₀₋₈-alkoxy, C₁₋₈-alkoxy-C₀₋₈-alkylcarbonyl-C₀₋₈-alkoxy, optionally N-C₁₋₈-alkylated C₁₋₈-alkoxycarbonylamino-C₂₋₈-alkoxy, optionally N-C₁₋₈-alkylated C₁₋₈-alkoxy-C₂₋₈-alkylamino-C₂₋₈-alkoxy, optionally N-C₁₋₈-alkylated or optionally halogen-substituted C₁₋₈-alkylcarbonylamino-C₂₋₈-alkoxy, cyano-C₂₋₈-alkoxy, optionally N-C₁₋₈-alkylated or optionally halogen-substituted C₃₋₈-cycloalkyl-C₀₋₈-alkylcarbonylamino-C₂₋₈-alkoxy, optionally N-C₁₋₈-alkylated hydroxy-C₂₋₈-alkylamino-C₂₋₈-alkoxy, heterocyclylcarbonyl-C₀₋₈-alkylamino-C₂₋₈-alkoxy, C₁₋₈-alkoxycarbonylamino-C₂₋₈-alkoxy, optionally N-C₁₋₈-alkylated or optionally halogen-substituted heterocyclyl-C₀₋₈-alkylcarbonylamino-C₂₋₈-alkoxy, C₃₋₈-cycloalkyl-C₀₋₈-alkoxy, C₃₋₈-cycloalkyloxy-C₂₋₈-alkoxy, heterocyclyl-C₀₋₈-(optionally hydroxy-substituted)alkoxy, optionally N-C₁₋₈-alkylated hetero-cydyl-C₀₋₈-alkylamino-C₀₋₈-alkylcarbonyl-C₀₋₈-alkoxy, C₁₋₈-alkylsulfonyl-C₁₋₈-alkoxy, C₃₋₈-alkynyloxy, heterocyclyl-C₃₋₈-alkynyloxy, optionally N-mono- or N,N-di-C₁₋₈-alkylated amino-C₃₋₈-alkynyloxy, N-mono- or N,N-di-C₁₋₈-alkylated aminocarbonyl-C₃₋₈-alkynyloxy, hetero-cyclylcarbonyl-C₀₋₈-alkoxy, heterocyclyloxy-C₂₋₈-alkoxy, optionally halogen- and/or hydroxy-substituted C₁₋₈-alkyl, optionally halogen- and/or hydroxy-substituted C₁₋₈-alkoxy-C₁₋₈-alkyl, optionally N-mono- or N,N-di-C₁₋₈-alkylated amino-C₁₋₈-alkyl, optionally N-mono- or N,N-di-C₁₋₈-alkylated or optionally hydroxy-substituted amino-C₀₋₈-alkylcarbonyl-C₁₋₈-alkyl, hydroxy-C₁₋₈-alkylcarbonyl-C₀₋₈-alkyl, C₁₋₈-alkoxy-C₀₋₈-alkylcarbonyl-C₀₋₈-alkyl, optionally N-C₁₋₈-alkylated C₁₋₈-alkoxycarbonylamino-C₁₋₈-alkyl, optionally N-C₁₋₈-alkylated C₁₋₈-alkoxy-C₂₋₈-alkylamino-C₁₋₈-alkyl, optionally N-C₁₋₈-alkylated or optionally halogen-substituted C₁₋₈-alkylcarbonylamino-C₁₋₈-alkyl, cyano-C₁₋₈-alkyl, optionally N-C₁₋₈-alkylated or optionally halogen-substituted C₃₋₈-cycloalkyl-C₀₋₈-alkylcarbonylamino-C₁₋₈-alkyl, optionally N-C₁₋₈-alkylated hydroxy-C₂₋₈-alkylamino-C₁₋₈-alkyl, heterocydylcarbonyl-C₀₋₈-alkylamino-C₁₋₈-alkyl, C₁₋₈-alkoxycarbonylamino-C₁₋₈-alkyl, optionally N-C₁₋₈-alkylated or optionally halogen-substituted heterocydyl-C₀₋₈-alkylcarbonylamino-C₁₋₈-alkyl, C₃₋₈-cycloalkyl-C₀₋₈-alkyl, C₃₋₈-cycloalkyloxy-C₁₋₈-alkyl, heterocyclyl-C₀₋₈-(optionally hydroxy-substituted)alkyl, optionally N-C₁₋₈-alkylated heterocydyl-C₀₋₈-alkylamino-C₀₋₈-alkylcarbonyl-C₀₋₈-alkyl, C₁₋₈-alkylsulfonyl-C₁₋₈-alkyl, C₂₋₈-alkynyl, heterocydyl-C₂₋₈-alkynyl, optionally N-mono- or N,N-di-C₁₋₈-alkylated amino-C₂₋₈-alkynyl, N-mono- or N,N-di-C₁₋₈-alkylated aminocarbonyl-C₂₋₈-alkynyl, hetero-cyclylcarbonyl-C₀₋₈-alkyl or heterocyclyloxy-C₁₋₈-alkyl,
whereby
each of the residues mentioned above for R⁴ bearing a hydroxy-group is optionally substituted at that hydroxy-group by a radical (A) whereby an ester bond is formed;
R⁵ is
(a) hydrogen
   or, when R⁴ is hydrogen, is
(b) C₂₋₈-alkenyl, C₁₋₈-alkyl, C₀₋₈-alkyl-carbonyl-amino-C₁₋₈-alkyl, C₁₋₈-alkyl-sulfonyl-C₁₋₈-alkyl, C₂₋₈-alkynyl, optionally N-mono- or N,N-di-C₁₋₈-alkylated carbamoyl-C₀₋₈-alkyl, optionally O-C₁₋₈-alkylated carboxyl-C₀₋₈-alkyl, C₃₋₈-cycloalkyl-C₀₋₈-alkyl, heterocyclylcarbonyl-C₀₋₈-alkyl or optionally N and/or N' mono-, di- or tri-C₁₋₈-alkylated ureido-C₁₋₈-alkyl, each of said radicals may be substituted, preferably by 1-4 C₁₋₈-alkoxy, C₁₋₈-alkoxy-C₁₋₈-alkoxy, C₁₋₈-alkoxycarbonyl-(N-C₁₋₈-alkyl)-amino, C₁₋₈-alkyl, C₁₋₈-alkyl-carbonyl-(N-C₁₋₈-alkyl)-amino, C₁₋₈-alkyl-sulfanyl, C₁₋₈-alkyl-sulfinyl, aryl-C₀₋₈-alkoxy, which is optionally substituted by 1 or 2 aryl or C₁₋₈-alkoxy radicals, aryl, which is optionally substituted by 1 or 2 aryl or C₁₋₈-alkoxy radicals, aryl-amino, cyano, C₃₋₈-cycloalkoxy, halogen, heterocyclyl-C₀₋₈-alkyl, heterocyclyl-C₀₋₈-alkoxy, heterocydyl-C₀₋₈-alkyl-amino, heterocyclyl-carbonyl, hydroxyl, optionally N-mono- or N,N-di-C₁₋₈-alkylated amino, optionally N-mono- or N,N-di-C₁₋₈-alkylated carbamoyl-C₀₋₈-alkyl, optionally N-mono- or N,N-di-C₁₋₈-alkylated carbamoyloxy, optionally N-mono- or N,N-di-C₁₋₈-alkylated sulfamoyl, optionally N-arylated or N-heterocyclyl-substituted carbamoyl, oxo, trifluoromethoxy or trifluoromethyl,
whereby
each of the residues mentioned above for R⁵ bearing a hydroxy-group are optionally substituted at that hydroxy-group by a radical (A) whereby an ester bond is formed;
R⁶ is hydrogen or C₁₋₈-alkyl;
R⁷ is optionally carboxy- or hydroxy-substituted C₁₋₈-alkyl or optionally carboxy- or hydroxy-substituted aryl-C₀₋₈-alkyl;
R⁸ is C₁₋₈-alkyl;
X is -Alk-R¹ -O-Alk-R¹, -Alk-O-R¹, -O-Alk-O-R¹, -S-Alk-R¹, -Alk-S-R¹, -Alk-NR⁶-R¹, -NR⁶-Alk-R¹, -C(O)-NR⁶-R¹, -Alk-C(O)-NR⁶-R¹, -C(O)-NR⁶-Alk-R¹, -Alk-C(O)-NR⁶-Alk-R¹, -NR⁶-C(O)-R¹, -Alk-NR⁶-C(O)-R¹, -NR⁶-C(O)-Alk-R¹, -Alk-NR⁶-C(O)-Alk-R¹, -O-Alk-C(O)-NR⁶-R¹, -O-Alk-NR⁶-C(O)-R¹, -S(O)₂NR⁶-R¹, -Alk-S(O)₂-NR⁶-R¹, -S(O)₂-NR⁶-Alk-R¹, -Alk-S(O)₂-NR⁶-Alk-R¹, -NR⁶-S(O)₂R¹, -Alk-NR⁶-S(O)₂R¹, -NR⁶-S(O)₂-Alk-R¹ or -Alk-NR⁶-S(O)₂Alk-R¹, where Alk represents C₁₋₈-alkylene, which is optionally substituted with halogen;
Q is hydrogen or represents a radical (A) whereby an amide bond is formed or a group of the formula -C(O)OCHR⁷OC(O)R⁸;
(A) represents a mono- or di-peptidic residue of one or two of the 20 natural amino acids, said residue being formally obtained from the mono- or di-peptide by elimination of the OH-group from the C-terminal end;
and whereby
(i) a radical (A) is present in at least one of R⁴, R⁵ or Q; or else
(ii) at least Q is a group of the formula -C(O)OCHR⁷OC(O)R⁸.

Examples of alkyl and alkoxy radicals, which may be linear or branched, are C₁₋₈-alkyl and

C₁₋₈-alkoxy radicals such as methyl, ethyl, n-propyl, isopropyl, n-butyl, isobutyl, sec-butyl, tert-butyl, pentyl, hexyl, and methoxy, ethoxy, propoxy, isopropoxy, butoxy, isobutoxy, sec-butoxy and tert-butoxy respectively, in addition C₀-alkoxy designates -O-. C₁₋₈-alkylenedioxy radicals are preferably methylenedioxy, ethylenedioxy and propylenedioxy. Examples of C₁₋₈-alkanoyl radicals, which may be linear or branched, are acetyl, propionyl and butyryl. Cycloalkyl is a saturated, cyclic hydrocarbon radical having 3-12 carbon atoms, i.e. cyclopropyl, cyclobutyl, cyclopentyl, cyclohexyl, cycloheptyl, bicyclo[2.2.1]heptyl, cyclooctyl, bicyclo[2.2.2]octyl and adamantyl. C₁₋₈-alkylene radicals, which may be linear or branched, are, for example, methylene, ethylene, 1-methylmethylene, propylene, methylethylene, 1-ethylmethylene, 1,1-dimethylmethylene, 2-methylpropylene, 2-methylbutylene, 2-methylbutyl-2-ene, butyl-2-ene, butyl-3-ene, propyl-2-ene, tetra-, penta- and hexamethylene. C₂₋₈-alkenylene radicals, which may be linear or branched, are, for example, vinylene and propenylene. C₂₋₈-alkynylene radicals, which may be linear or branched, are, for example, ethynylene; acyl radicals are alkanoyl radicals, preferably C₁₋₈-alkanoyl radicals, or aroyl radicals such as benzoyl.

Aryl denotes mono- or polycyclic aromatic radicals which may be mono- or polysubstituted, for example phenyl, substituted phenyl, naphthyl or substituted naphthyl. Examples of substituents on such aryl radicals are acetamidinyl-C₁₋₈-alkyl, acyl-C₁₋₈-alkoxy-C₁₋₈-alkyl, (N-acyl)-C₁₋₈-alkoxy-C₁₋₈-alkylamino, C₂₋₈-alkenyl, C₂₋₈-alkenyloxy, C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, (N-C₁₋₈-alkoxy)-C₁₋₈-alkylaminocarbonyl-C₁₋₈-alkoxy, (N-C₁₋₈-alkoxy)-C₁₋₈-alkylaminocarbonyl-C₁₋₈-alkyl, C₁₋₈-alkoxy-C₁₋₈-alkyl-carbamoyl, C₁₋₈-alkoxy-C₁₋₈-alkyl-carbonyl, C₁₋₈-alkoxy-C₁₋₈-alkyl-carbonylamino, C₁₋₈-alkoxy-C₁₋₈-alkyl-heterocydyl, 2-C₁₋₈-alkoxy-C₁₋₈-alkyl-4-oxo-imidazol-1-yl, 6-alkoxy-aminocarbonyl-C₁₋₈-alkoxy, C₁₋₈-alkoxy-aminocarbonyl-C₁₋₈-alkyl, C₁₋₈-alkoxycarbonyl, C₁₋₈-alkoxycarbonyl-C₁₋₈-alkoxy, C₁₋₈-alkoxycarbonyl-C₁₋₈-alkyl, C₁₋₈-alkoxycarbonylamino-C₁₋₈-alkoxy, C₁₋₈-alkoxycarbonylamino-C₁₋₈-alkyl, C₁₋₈-alkoxycarbonylphenyl, C₁₋₈-alkyl, (N-C₁₋₈-alkyl)-C₁₋₈-alkoxy-C₁₋₈-alkyl-carbamoyl, (N-C₁₋₈-alkyl)-C₁₋₈-alkoxy-C₁₋₈-alkyl-carbonylamino, (N-C₁₋₈-alkyl)-C₁₋₈-alkoxy-carbonylamino, (N-C₁₋₈-alkyl)-C₀₋₈-alkylcarbonylamino-C₁₋₈-alkoxy, (N-C₁₋₈-alkyl)-C₀₋₈-alkylcarbonylamino-C₁₋₈-alkyl, (N-C₁₋₈-alkyl)-C₁₋₈-alkylsulfonylamino-C₁₋₈-alkoxy, (N-C₁₋₈-alkyl)-C₁₋₈-alkylsulfonylamino-C₁₋₈-alkyl, C₁₋₈-alkyl-amidinyl, optionally N-mono or N,N-di-C₁₋₈-alkylated amino, C₁₋₈-alkylamino-C₂₋₈-alkoxy, di-C₁₋₈-alkylamino-C₂₋₈-alkoxy, C₁₋₈-alkylamino-C₁₋₈-alkyl, di-C₁₋₈-alkylamino-C₁₋₈-alkyl, C₁₋₈-alkylaminocarbonyl-C₁₋₈-alkoxy, di-C₁₋₈-alkylaminocarbonyl-C₁₋₈-alkoxy, C₁₋₈-alkylaminocarbonyl-C₁₋₈-alkoxy-C₁₋₈-alkyl, C₁₋₈-alkylaminocarbonyl-C₁₋₈-alkyl, di-C₁₋₈-alkylaminocarbonyl-C₁₋₈-alkyl, C₁₋₈-alkylaminocarbonyl-amino-C₁₋₈-alkoxy, C₁₋₈-alkylaminocarbonylamino-C₁₋₈-alkyl, C₁₋₈-alkyl-carbamoyl, di-C₁₋₈-alkyl-carbamoyl, C₀₋₈-alkylcarbonylamino, C₀₋₈-alkylcarbonylamino-C₁₋₈-alkoxy, C₀₋₈-alkylcarbonylamino-C₁₋₈-alkyl, C₁₋₈-alkylcarbonyloxy, C₁₋₈-alkylcarbonyloxy-C₁₋₈-alkoxy, C₁₋₈-alkylcarbonyloxy-C₁₋₈-alkyl, C₁₋₈-alkylenedioxy, C₁₋₈-alkyl-sulfonyl, C₁₋₈-alkylsulfonyl-C₁₋₈-alkoxy, C₁₋₈-alkylsulfonyl-C₁₋₈-alkyl, C₁₋₈-alkylsulfonylamino-C₁₋₈-alkoxy, C₁₋₈-alkylsulfonylamino-C₁₋₈-alkyl, aryl-C₁₋₈-alkanoyl, aryl-C₀₋₈-alkoxy, aryl-C₀₋₈-alkyl, arylamino, aryloxy, arylthio, benzoyloxy-C₁₋₈-alkoxy, benzyloxy, carbamoyl-C₀₋₈-alkoxy, carbamoyl-C₀₋₈-alkyl, carboxy, carboxy-C₁₋₈-alkoxy, carboxy-C₁₋₈-alkoxy-C₁₋₈-alkyl, carboxy-C₁₋₈-alkyl, cyano, cyano-C₁₋₈-alkoxy, cyano-C₁₋₈-alkyl, C₃₋₈-cycloalkyl-C₁₋₈-alkanoyl, C₃₋₈-cycloalkylcarbonyl-amino-C₁₋₈-alkoxy, C₃₋₈-cycloalkylcarbonylamino-C₁₋₈-alkyl, cyclopropyl-C₁₋₈-alkoxy, cyclopropyl-C₁₋₈-alkyl, O,N-dimethylhydroxylamino-C₁₋₈-alkyl, dioxolanyl-C₁₋₈-alkoxy, halogen, halogen-C₁₋₈-alkoxy, halogen-C₁₋₈-alkyl, heterocyclyl, heterocyclyl-C₁₋₈-alkanoyl, heterocyclyl-C₁₋₈-alkoxy, heterocyclyl-C₁₋₈-alkoxy-C₁₋₈-alkoxy, heterocyclyl-C₁₋₈-alkoxy-C₁₋₈-alkyl, heterocyclyl-C₁₋₈-alkyl, heterocyclylamino, heterocyclyloxy, heterocyclylthio, hydroxy, hydroxy-C₂₋₈-alkoxy, hydroxy-C₂₋₈-alkoxy-C₁₋₈-alkoxy, hydroxy-C₂₋₈-alkoxy-C₁₋₈-alkyl, hydroxy-C₁₋₈-alkyl, (N-hydroxy)-C₁₋₈-alkylaminocarbonyl-C₁₋₈-alkoxy, (N-hydroxy)-C₁₋₈-alkylaminocarbonyl-C₁₋₈-alkyl, hydroxy-C₁₋₈-alkylphenyl, (N-hydroxy)-aminocarbonyl-C₁₋₈-alkoxy, (N-hydroxy)-aminocarbonyl-C₁₋₈-alkyl, hydroxybenzyloxy, methylendioxybenzyloxy, methoxybenyloxy, O-methyl-oximyl-C₁₋₈-alkyl, nitro, 2-oxo-oxazolidinyl-C₁₋₈-alkoxy, 2-oxo-oxazolidinyl-C₁₋₈-alkyl or pyridyl-carbonylamino-C₁₋₈-alkyl.

The term heterocyclyl denotes mono- or polycyclic, saturated and unsaturated heterocyclic radicals having from 1 to 4 nitrogen and/or 1 or 2 sulfur or oxygen atoms and which may be mono- or polysubstituted, especially mono- ,di- or trisubstituted. Additionally, the term heterocyclyl includes the above mentioned oxo-substituted radicals. Heterocyclyl radicals are preferentially bound to the rest of the molecule via a carbon atom. Examples of unsaturated heterocyclyl radicals are benzo[1,3]dioxolyl, benzofuranyl, benzoimidazolyl, benzooxazolyl, benzothiazolyl, benzo[b]thienyl, quinazolinyl, quinolyl, quinoxalinyl, dihydrobenzofuranyl, 1,3-dihydro-benzoimidazol, 3,4-dihydro-2H-benzo[1,4]oxazinyl, dihydro-3H-benzo[1,4]oxazinyl, 1,4-dihydro-benzo[d][1,3]oxazin, dihydro-2H-benzo[1,4]thiazinyl, 3,4-dihydro-1H-quinazolin, 3,4-dihydro-1H-quinolin, 2,3-dihydroindolyl, dihydro-1H-pyrido[2,3-b][1,4]oxazinyl, 1,1-dioxo-dihydro-2H-benzo[1,4]thiazinyl, furyl, imidazolyl, imidazo[1,5-a]pyridinyl, imidazo[1,2-a]pyrimidinyl, indazolyl, indolyl, isobenzofuranyl, isoquinolyl, [1,5]naphthyridyl, oxazolyl, 1-oxido-pyridyl, 2-oxo-benzoimidazolyl, 3-oxo-4H-benzo[1,4]oxazinyl, 2-oxo-benzooxazolyl, 3-oxo-4H-benzo[1,4]thiazinyl, 2-oxo-dihydro-benzo[e][1,4]diazepinyl, 2-oxo-1,3-dihydro-benzoimidazol, 2-oxo-dihydro-benzo-[d][1,3]oxazinyl, 2-oxo-3,4-dihydro-1H-quinazolin, 2-oxo-3,4-dihydro-1H-quinolin, 4-oxo-dihydro-imidazolyl, 2-oxo-1,3-dihydroindolyl, 1-oxo-3H-isobenzofuranyl, 2-oxo-1 H-pyrido[2,3-b][1,4]oxazinyl, 2-oxo-1,3,4,5-tetrahydro-benzo[b]azepin, 2-oxo-tetrahydro-benzo[e][1,4]diazepinyl, 4-oxo-3H-thieno[2,3-d]pyrimidinyl, 5-oxo-4H-[1,2,4]triazinyl, phthalazinyl, pyranyl, pyrazinyl, pyrazolyl, pyridyl, pyrimidinyl, 1H-pyrrolizinyl, pyrrolo[3,2-c]pyridinyl, pyrrolo[2,3-c]pyridinyl, pyrrolo[3,2-b]pyridinyl, 1 H-pyrrolo[2,3-b]pyridyl, pyrrolyl, 1,3,4,5-tetrahydro-benzo[b]azepin, tetrahydroquinolinyl, tetrahydroquinoxalinyl, tetrahydroisoquinolinyl, thiazolyl, thienyl, triazinyl, triazolyl, 1,1,3-trioxo-dihydro-2H-1lambda*6*-benzo[1,4]thiazinyl, [1,2,3]triazolo[1,5-a]pyridinyl or [1,2,4]triazolo[4,3-a]pyridinyl.

The term saturated heterocyclyl denotes 3-16 membered mono- or bicyclic, saturated heterocyclic radicals having from 1 to 4 nitrogen and/or 1 or 2 sulfur or oxygen atoms. Prefered are 3-8 membered, especially preferred 5- or 6-membered monocyclic radicals, which may be condensed to a 3-8 membered, carbocyclic or heterocyclic ring. Another preferred group of heterocyclic radicals are bicyclic radicals possessing a spirocyclic or bridged ring skeleton. Preferred heterocyclic radicals are possessing per ring 1 nitrogen, oxygen or sulfur atom, 1-2 nitrogen atoms and 1-2 oxygen atoms or 1-2 nitrogen atoms and 1-2 sulfur atoms, whereby, per ring, at least 1 carbon atom, preferentially 1-7 carbon atoms are present.

Examples for saturated heterocyclyl radicals are azepanyl, azetidinyl, aziridinyl, 3,4-dihydroxy-pyrrolidinyl, 2,6-dimethylmorpholinyl, 3,5-dimethylmorpholinyl, dioxanyl, [1,4]dioxepanyl, dioxolanyl, 4,4-dioxothiomorpholinyl, dithianyl, dithiolanyl, 2-hydroxymethylpyrrolidinyl, 4-hydroxypiperidinyl, 3-hydroxypyrrolidinyl, 4-methylpiperazinyl, 1-methylpiperidinyl, 1-methylpyrrolidinyl, morpholinyl, oxathianyl, oxepanyl, 2-oxo-azepanyl, 2-oxo-imidazolidinyl, 2-oxo-oxazolidinyl, 2-oxo-piperidinyl, 4-oxo-piperidinyl, 2-oxo-pyrrolidinyl, 2-oxo-tetrahydro-pyrimidinyl, 4-oxo-thiomorpholinyl, piperazinyl, piperidinyl, pyrrolidinyl, tetrahydrofuranyl, tetrahydropyranyl, tetrahydrothiophenyl, tetrahydrothiopyranyl, thiepanyl or thiomorpholinyl. Examples for bicyclic heterocyclyl radicals are 2,5-dioxa-bicyclo[4.1.0]heptanyl, 2-oxa-bicyclo[2.2.1]heptanyl, 2-oxa-bicyclo[4.1.0]heptanyl, 3-oxa-bicyclo[4.1.0]heptanyl, 7-oxa-bicyclo[2.2.1]heptanyl, 2-oxa-bicyclo[3.1.0]hexanyl, 3-oxa-bicyclo[3.1.0]hexanyl, 1-oxa-spiro[2.5]octanyl, 6-oxa-spiro[2.5]octanyl or 3-oxa-bicyclo[3.3.1]nonanyl.

Heterocyclyl radicals may be unsubstituted or mono- or polysubstituted, for example mono-or disubstituted. Examples of substituents on such heterocyclyl radicals are C₁₋₈-alkanoyl, C₂₋₈-alkenyl, C₂₋₈-alkynyl, C₁₋₈-alkoxy, C₁₋₈-alkoxy-C₁₋₈-alkoxy, C₁₋₈-alkoxy-C₁₋₈-alkyl, C₁₋₈-alkoxycarbonylamino, C₁₋₈-alkyl, C₀₋₈-alkylcarbonylamino, C₁₋₈-alkylcarbonyloxy, C₁₋₈-alkylenedioxy, optionally N-mono or N,N-di-C₁₋₈-alkylated amino, aryl, optionally N-mono or N,N-di-C₁₋₈-alkylated carbamoyl, optionally esterified carboxy, cyano, C₃₋₈-cycloalkoxy, halogen, heteroaryl, heterocyclyl, hydroxy, nitro, oxid, oxo, polyhalogen-C₁₋₈-alkoxy or polyhalogen-C₁₋₈-alkyl.

The term polyhydroxyalkyl denotes C₁₋₇-alkyl radicals which may be substituted by 2-6 hydroxyl groups, for example glyceryl, arabityl, sorbityl, etc.

Halogen or halo denotes, for example, fluorine, chlorine or bromine, or a radical singly, multiply or fully substituted by fluorine, chlorine or bromine.

The radical (A) is a mono- or di--peptidic residue derived from the 20 natural amino acids, formally by elimination of the OH-group of the C-terminal end, which each, except for the residue derived from glycine only, independently of the others, may be present either in the (D)-, (L)- or racemic (D,L)- configuration, but preferably in the L-form. Examples of such amino acids (mono-peptides) are valine, glycine, alanine, leucine, isoleucine, phenylalanine, tyrosine, aspartic acid and glutamic acid. Examples of radicals (A), the asterix indicating the bond to the rest of the molecule of formula (I), are thus:

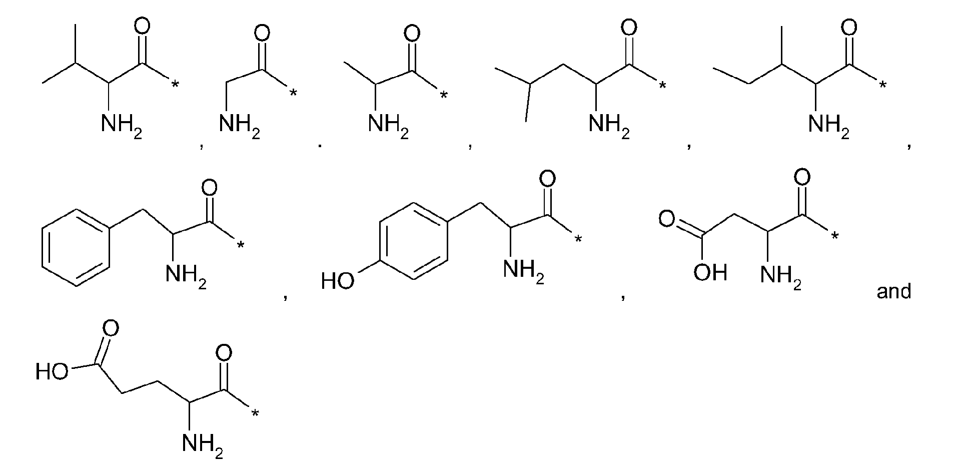

Examples of such di-peptides are glycylvaline, glycylleucine and valylvaline. Examples of radicals (A), the asterix indicating the bond to the rest of the molecule of formula (I), are thus:

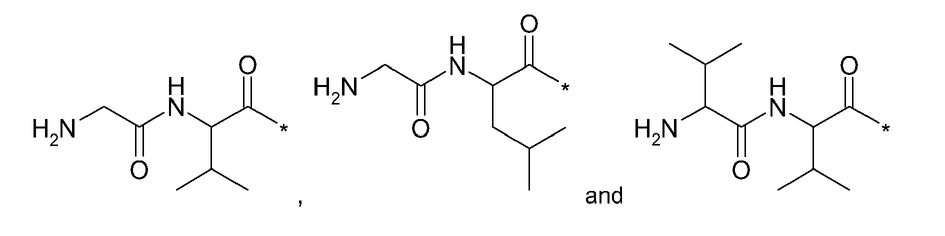

Salts are primarily the pharmaceutically usable or nontoxic salts of compounds of formula (I). The term "pharmaceutically useable salts" encompasses salts with inorganic or organic acids, such as hydrochloric acid, hydrobromic acid, nitric acid, sulfuric acid, phosphoric acid, citric acid, formic acid, maleic acid, acetic acid, succinic acid, tartaric acid, methanesulfonic acid, p-toluenesulfonic acid and the like.

Salts of compounds having salt-forming groups are in particular acid addition salts, salts with bases, or, in the presence of a plurality of salt-forming groups, in some cases also mixed salts or internal salts.

Such salts are formed, for example, from compounds of formula (I) with an acidic group, for example a carboxyl or sulfonyl group, and are, for example, the salts thereof with suitable bases such as non-toxic metal salts derived from metals of group Ia, Ib, IIa and IIb of the Periodic Table of the Elements, for example alkali metal, in particular lithium, sodium, or potassium, salts, alkaline earth metal salts, for example magnesium or calcium salts, and also zinc salts and ammonium salts, including those salts which are formed with organic amines, such as optionally hydroxy-substituted mono-, di- or trialkylamines, in particular mono-, di- or tri(lower alkyl)amines, or with quaternary ammonium bases, e.g. methyl-, ethyl-, diethyl- or triethylamine, mono-, bis- or tris(2-hydroxy(lower alkyl))amines, such as ethanol-, diethanol- or triethanolamine, tris(hydroxymethyl)methylamine or 2-hydroxy-tert-butylamine, N,N-di(lower alkyl)-N-(hydroxy(lower alkyl))amine, such as N,N-di-N-dimethyl-N-(2-hydroxyethyl)amine, or N-methyl-D-glucamine, or quaternary ammonium hydroxides such as tetrabutyl ammoniumhydroxide. The compounds of formula (I) having a basic group, for example an amino group, may form acid addition salts, for example with suitable inorganic acids, e.g. hydrohalic acid such as hydrochloric acid, hydrobromic acid, sulfuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulfonic or phosphonic acids or N-substituted sulfamic acids, e.g. acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid, isonicotinic acid, and also amino acids, for example the alpha-amino acids mentioned above, and also methanesulfonic acid, ethanesulfonic acid, 2-hydroxyethanesulfonic acid, ethane-1,2-disulfonic acid, benzenesulfonic acid, 4-methylbenzenesulfonic acid, naphthalene-2-sulfonic acid, 2- or 3-phosphoglycerate, glucose 6-phosphate, N-cyclohexylsulfamic acid (with formation of the cyclamates) or with other acidic organic compounds such as ascorbic acid. Compounds of formula (I) having acidic and basic groups may also form internal salts.

Salts obtained may be converted to other salts in a manner known per se, acid addition salts, for example, by treating with a suitable metal salt such as a sodium, barium or silver salt, of another acid in a suitable solvent in which an inorganic salt which forms is insoluble and thus separates out of the reaction equilibrium, and base salts by release of the free acid and salt reformation.

The compounds of formula (I), including their salts, may also be obtained in the form of hydrates or include the solvent used for the crystallization.

For the isolation and purification, pharmaceutically unsuitable salts may also find use.

The compounds of formula (I) also include those compounds in which one or more atoms are replaced by their stable, non-radioactive isotopes; for example a hydrogen atom by deuterium.

The compounds of formula (I) and the salts thereof may be prepared in a similar manner to the preparation processes disclosed in the literature. Preferred procedures for the preparation of optically pure piperidine scaffolds for compounds of formula (I) have been described in WO 2006/005741. Alternative procedures for the preparation of optically pure compounds of formula (I) compounds consist of the construction of the requisite multiply-substituted piperidine scaffolds and their precursors via either (i) a chiral aza-Diels-Alder reaction of an imine derivative with a diene (see Chemistry-A European Journal 2000, 6(13), 2435-2448 and references cited therein) [Scheme 1] or (ii) condensation of a chiral amino acid derivative with Meldrum's acid followed by cyclisation (see Journal of Organic Chemistry 2004, 69(1), 130-141 and references cited therein) [Scheme 2]. Details on the specific preparation variants can be taken from the examples.

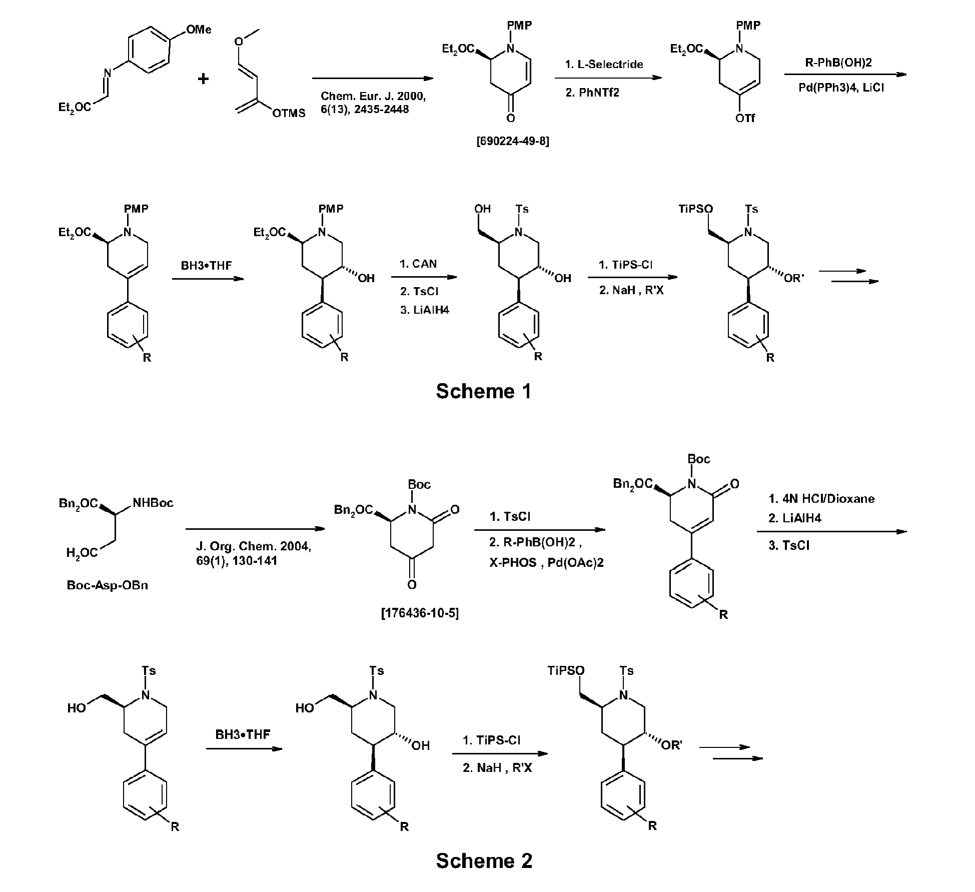

The compounds of formula (I) have at least three asymmetric carbon atoms and may therefore be in the form of optically pure diastereomers, diastereomeric mixtures, diastereomeric racemates, mixtures of diastereomeric racemates or as meso compounds. The invention encompasses all of these forms. Diastereomeric mixtures, diastereomeric racemates or mixtures of diastereomeric racemates may be separated by customary procedures, for example by column chromatography, thin-layer chromatography, HPLC and the like.

The compounds of formula (I) may also be prepared in optically pure form. The separation into antipodes can be effected by procedures known per se, either preferably at an earlier synthetic stage by salt formation with an optically active acid, for example (+)- or (-)-mandelic acid and separation of the diastereomeric salts by fractional crystallization, or preferably at a relatively late stage by derivatizing with a chiral auxiliary building block, for example (+)- or (-)-camphanoyl chloride, and separation of the diastereomeric products by chromatography and/or crystallization and subsequent cleavage of the bonds to give the chiral auxiliary. The pure diastereomeric salts and derivatives may be analysed to determine the absolute configuration of the piperidine present with common spectroscopic procedures, and X-ray spectroscopy on single crystals constitutes a particularly suitable procedure.

It is possible for the configuration at individual chiral centres in a compound of formula (I) to be inverted selectively. For example, the configuration of asymmetric carbon atoms which bear nucleophilic substituents, such as amino or hydroxyl, may be inverted by second-order nucleophilic substitution, if appropriate after conversion of the bonded nucleophilic substituent to a suitable nucleofugic leaving group and reaction with a reagent which introduces the original substituents, or the configuration at carbon atoms having hydroxyl groups can be inverted by oxidation and reduction, analogously to the process in the European patent application EP-A-0 236 734. Also advantageous is the reactive functional modification of the hydroxyl group and subsequent replacement thereof by hydroxyl with inversion of configuration.

The compound groups mentioned below are not to be regarded as closed, but rather parts of these compound groups may be exchanged with one another or with the definitions given above or omitted in a sensible manner, for example to replace general by more specific definitions. The definitions are valid in accordance with general chemical principles, such as, for example, the common valences for atoms.

Preferred compounds according to the invention are those of the general formula (IA) 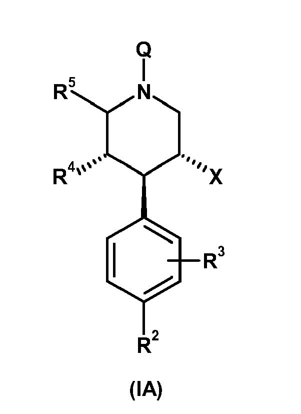 and the salts thereof, preferably the pharmaceutically acceptable salts thereof;
in which R¹, R², R³, R⁴, R⁵, Q and X have the meaning stated above for the compounds of the formula (I).

A further, preferred group of compounds of the formula (I) or more preferably of the formula (IA) and the salts thereof, preferably the pharmaceutically acceptable salts thereof; are compounds where
R¹ is aryl or heterocyclyl;
R² is C₂₋₈-alkenyloxy, C₁₋₈-alkoxy, C₁₋₈-alkoxy-C₁₋₈-alkoxy, C₁₋₈-alkoxy-C₁₋₈-alkyl, C₁₋₈-alkoxy-C₀₋₈-alkyl-C₃₋₈-cycloalkyl-C₀₋₈-alkoxy-C₁₋₈-alkoxy, C₁₋₈-alkoxy-C₀₋₈-alkyl-C₃₋₈-cycloalkyl-C₀₋₈-alkoxy-C₁₋₈-alkyl, C₁₋₈-alkoxy-C₂₋₈-alkylsulfanyl, C₁₋₈-alkoxy-C₂₋₈-alkylsulfanyl-C₂₋₈-alkoxy, C₁₋₈-alkoxy-C₂₋₈-alkylsulfanyl-C₁₋₈-alkyl, C₁₋₈-alkyl, C₁₋₈-alkylsulfanyl-C₂₋₈-alkoxy, C₁₋₈-alkylsulfanyl-C₂₋₈-alkoxy-C₁₋₈-alkoxy, C₁₋₈-alkylsulfanyl-C₂₋₈-alkoxy-C₁₋₈-alkyl, C₁₋₈-alkylsulfanyl-C₁₋₈-alkyl, aryl-C₀₋₈-alkoxy, aryl-C₀₋₈-alkoxy-C₁₋₈-alkoxy, aryl-C₀₋₈-alkoxy-C₁₋₈-alkyl, C₃₋₈-cycloalkyl-C₀₋₈-alkoxy-C₁₋₈-alkoxy, C₃₋₈-cycloalkyl-C₀₋₈-alkoxy-C₁₋₈-alkoxy, C₃₋₈-cycloalkyl-C₀₋₈-alkoxy-C₁₋₈-alkoxy-C₁₋₈-alkyl, C₃₋₈-cycloalkyl-C₀₋₈-alkoxy-C₁₋₈-alkyl, heterocydyl-C₀₋₈-alkoxy, heterocyclyl-C₀₋₈-alkoxy-C₁₋₈-alkoxy or heterocydyl-C₀₋₈-alkoxy-C₁₋₈-alkyl ;
R³ is hydrogen or 1-2 halogen;
R⁴ is
(a) hydrogen
   or, when R⁵ is hydrogen, is
(b) is optionally halogen- and/or hydroxy-substituted C₁₋₈-alkoxy, optionally halogen- and/or hydroxy-substituted C₁₋₈-alkoxy-C₁₋₈-alkoxy, optionally N-mono- or N,N-di-C₁₋₈-alkylated amino-C₂₋₈-alkoxy, optionally N-mono- or N,N-di-C₁₋₈-alkylated or optionally hydroxy-substituted amino-C₀₋₈-alkylcarbonyl-C₁₋₈-alkoxy, C₃₋₈-cycloalkyl-C₀₋₈-alkoxy, C₃₋₈-cyclo-alkyloxy-C₂₋₈-alkoxy, heterocyclyl-C₀₋₈-(optionally hydroxy-substituted)alkoxy, optionally N-C₁₋₈-alkylated heterocydyl-C₀₋₈-alkylamino-C₀₋₈-alkylcarbonyl-C₀₋₈-alkoxy, C₃₋₈-alkynyloxy, heterocyclylcarbonyl-C₀₋₈-alkoxy, heterocydyloxy-C₂₋₈-alkoxy, optionally halogen- and/or hydroxy-substituted C₁₋₈-alkyl, optionally halogen- and/or hydroxy-substituted C₁₋₈-alkoxy-C₁₋₈-alkyl, optionally N-mono- or N,N-di-C₁₋₈-alkylated amino-C₁₋₈-alkyl, optionally N-mono- or N,N-di-C₁₋₈-alkylated or optionally hydroxy-substituted amino-C₀₋₈-alkylcarbonyl-C₁₋₈-alkyl, optionally N-C₁₋₈-alkylated C₁₋₈-alkoxycarbonylamino-C₁₋₈-alkyl, optionally N-C₁₋₈-alkylated or optionally halogen-substituted C₁₋₈-alkylcarbonylamino-C₁₋₈-alkyl, optionally N-C₁₋₈-alkylated or optionally halogen-substituted C₃₋₈-cycloalkyl-C₀₋₈-alkylcarbonylamino-C₁₋₈-alkyl, C₁₋₈-alkoxycarbonylamino-C₁₋₈-alkyl, optionally N-C₁₋₈-alkylated or optionally halogen-substituted heterocydyl-C₀₋₈-alkylcarbonylamino-C₁₋₈-alkyl, C₃₋₈-cycloalkyl-C₀₋₈-alkyl, C₃₋₈-cycloalkyloxy-C₁₋₈-alkyl, heterocyclyl-C₀₋₈-(optionally hydroxy-substituted)alkyl, optionally N-C₁₋₈-alkylated heterocydyl-C₀₋₈-alkylamino-C₀₋₈-alkylcarbonyl-C₀₋₈-alkyl, heterocyclylcarbonyl-C₀₋₈-alkyl or heterocydyloxy-C₁₋₈-alkyl,
whereby
each of the residues, mentioned above for R⁴, bearing a hydroxy-group is optionally substituted at that hydroxy-group by a radical (A) whereby an ester bond is formed;
R⁵ is
(a) hydrogen
   or, when R⁴ is hydrogen, is
(b) C₁₋₈-alkyl, C₀₋₈-alkyl-carbonyl-amino-C₁₋₈-alkyl, optionally N-mono- or N,N-di-C₁₋₈-alkylated carbamoyl-C₀₋₈-alkyl, C₃₋₈-cycloalkyl-C₀₋₈-alkyl, heterocydylcarbonyl-C₀₋₈-alkyl or optionally N and/or N' mono-, di- or tri-C₁₋₈-alkylated ureido-C₁₋₈-alkyl, each of said radicals may be substituted, preferably by 1-4 C₁₋₈-alkoxy, C₁₋₈-alkoxy-C₁₋₈-alkoxy, C₁₋₈-alkoxycarbonyl-(N-C₁₋₈-alkyl)-amino, C₁₋₈-alkyl, C₁₋₈-alkyl-carbonyl-(N-C₁₋₈-alkyl)-amino, C₁₋₈-alkyl-sulfanyl, C₁₋₈-alkylsulfinyl, aryl-C₀₋₈-alkoxy, which is optionally substituted by 1 or 2 aryl or C₁₋₈-alkoxy radicals, aryl, which is optionally substituted by 1 or 2 aryl or C₁₋₈-alkoxy radicals, aryl-amino, cyano, C₃₋₈-cycloalkoxy, halogen, heterocyclyl-C₀₋₈-alkyl, heterocydyl-C₀₋₈-alkoxy, heterocydyl-C₀₋₈-alkyl-amino, heterocyclyl-carbonyl, hydroxyl, optionally N-mono- or N,N-di-C₁₋₈-alkylated amino, optionally N-mono- or N,N-di-C₁₋₈-alkylated carbamoyl-C₀₋₈-alkyl, optionally N-mono-or N,N-di-C₁₋₈-alkylated carbamoyloxy or optionally N-arylated or N-heterocyclyl-substituted carbamoyl,
whereby
each of the residues, mentioned above for R⁵, bearing a hydroxy-group is optionally substituted at that hydroxy-group by a radical (A) whereby an ester bond is formed;
R⁶ is hydrogen or C₁₋₈-alkyl;
R⁷ is optionally carboxy- or hydroxy-substituted C₁₋₈-alkyl or optionally carboxy- or hydroxy-substituted aryl-C₀₋₈-alkyl;
R⁸ is C₁₋₈-alkyl;
(A) represents a mono- or di-peptidic residue of one or two of the 20 natural amino acids, said residue being formally obtained from the mono- or di-peptide by elimination of the OH-group from the C-terminal end;
X is -Alk-R¹, -0-Alk-R¹, -O-Alk-O-R¹, -C(O)-NR⁶-R¹, -Alk-C(O)-NR⁶-R¹, -C(O)-NR⁶-Alk-R¹, - Alk-C(O)-NR⁶-Alk-R¹, -Alk-NR⁶-C(O)-R¹, -Alk-NR⁶-C(O)-Alk-R¹, -O-Alk-C(O)-NR⁶-R¹, -O-Alk-NR⁶-C(O)-R¹, -Alk-S(O)₂-NR⁶-R¹, -S(O)₂-NR⁶-Alk-R¹, -Alk-S(O)₂-NR⁶-Alk-R¹, -Alk-NR⁶-S(O)₂-R¹ or -Alk-NR⁶-S(O)₂-Alk-R¹, where Alk represents C₁₋₈-alkylene, which is optionally substituted with halogen;
Q is hydrogen or represents a radical (A); or a group of the formula -C(O)OCHR⁷OC(O)R⁸; and whereby
(i) a radical (A) is present in at least one of R⁴, R⁵ or Q; or else
(ii) at least Q is a group of the formula -C(O)OCHR⁷OC(O)R⁸.

Preferred R¹ radicals are phenyl substituted by 1-3 radicals selected from C₁₋₈-alkanoyl, C₁₋₈-alkoxy, C₁₋₈-alkoxy-C₁₋₈-alkanoyl, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkoxycarbonyl-C₁₋₈-alkoxy, C₁₋₈-alkoxycarbonyl-C₁₋₈-alkyl, C₁₋₈-alkoxycarbonylamino-C₂₋₈-alkoxy, C₁₋₈-alkoxycarbonylamino-C₁₋₈-alkyl, C₁₋₈-alkyl, (N-C₁₋₈-alkyl)-C₁₋₈-alkylcarbonylamino-C₂₋₈-alkoxy, (N-C₁₋₈-alkyl)-C₁₋₈-alkylcarbonylamino-C₁₋₈-alkyl, C₁₋₈-alkylaminocarbonyl-C₁₋₈-alkoxy, di-C₁₋₈-alkylaminocarbonyl-C₁₋₈-alkoxy, C₁₋₈-alkylaminocarbonyl-C₁₋₈-alkoxy-C₁₋₈-alkyl, C₁₋₈-alkylaminocarbonyl-C₁₋₈-alkyl, di-C₁₋₈-alkylaminocarbonyl-C₁₋₈-alkyl, C₁₋₈-alkylaminocarbonylamino-C₂₋₈-alkoxy, C₁₋₈-alkylaminocarbonylamino-C₁₋₈-alkyl, C₁₋₈-alkylcarbonylamino-C₂₋₈-alkoxy, C₁₋₈-alkylcarbonylamino-C₁₋₈-alkyl, C₁₋₈-alkylsulfonyl-C₁₋₈-alkoxy, C₁₋₈-alkylsulfonyl-C₁₋₈-alkyl, C₁₋₈-alkylsulfonylamino-C₂₋₈-alkoxy, C₁₋₈-alkylsulfonylamino-C₁₋₈-alkyl, aryl-C₀₋₈-alkoxy, aryl-C₀₋₈-alkyl, optionally N-mono- oder N,N-di-C₁₋₈-alkylated carbamoyl-C₀₋₈-alkoxy, carboxy-C₁₋₈-alkoxy, carboxy-C₁₋₈-alkoxy-C₁₋₈-alkyl, carboxy-C₁₋₈-alkyl, C₁₋₈-alkyl, cyano, cyano-C₂₋₈-alkoxy, cyano-C₁₋₈-alkyl, C₃₋₈-cycloalkyl-C₁₋₈-alkoxy, C₃₋₈-cycloalkyl-carbamoyl-C₀₋₈-alkyl, C₃₋₈-cycloalkylcarbonylamino-C₂₋₈-alkoxy, C₃₋₈-cycloalkylcarbonylamino-C₁₋₈-alkyl, halogen, halogen-C₁₋₈-alkoxy, halogen-C₁₋₈-alkyl, heterocydyl-C₀₋₈-alkoxy, heterocydyl-C₀₋₈-alkyl, hydroxy-C₂₋₈-alkoxy-C₁₋₈-alkoxy or hydroxy-C₂₋₈-alkoxy-C₁₋₈-alkyl.

R¹ radicals which are likewise preferred are benzofuranyl, benzoimidazolyl, 4H-benzo[1,4]oxazinyl, benzooxazolyl, 2- and 5-benzo[b]thienyl, 3,4-dihydro-2H-benzo[1,4]oxazinyl, 1,3-dihydroindolyl, 2,3-dihydroindolyl, indazolyl, indolyl,1H-quinolinyl, 2H-chromenyl, 1,1a,2,7b-tetrahydro-cyclopropa[c]chromenyl, 1a,7b-dihydro-1H-cyclo-propa[c]chromenyl, 6- and 7-isoquinolyl, pyridyl, pyrimidinyl, 6- and 7-quinazolinyl, 6- and 7-quinolyl, 6-quinoxalinyl, 6- and 7-tetrahydroisoquinolyl and 6- and 7-tetrahydroquinolyl, each of which is substituted by 1-3 radicals selected from C₁₋₈-alkanoyl, C₁₋₈-alkoxy, C₁₋₈-alkoxy-C₁₋₈-alkanoyl, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkoxycarbonyl-C₁₋₈-alkoxy, C₁₋₈-alkoxycarbonyl-C₁₋₈-alkyl, C₁₋₈-alkoxycarbonylamino-C₂₋₈-alkoxy, C₁₋₈-alkoxycarbonylamino-C₁₋₈-alkyl, C₁₋₈-alkyl, (N-C₁₋₈-alkyl)-C₁₋₈-alkylcarbonylamino-C₂₋₈-alkoxy, (N-C₁₋₈-alkyl)-C₁₋₈-alkylcarbonylamino-C₁₋₈-alkyl, C₁₋₈-alkylaminocarbonyl-C₁₋₈-alkoxy, di-C₁₋₈-alkylaminocarbonyl-C₁₋₈-alkoxy, C₁₋₈-alkylaminocarbonyl-C₁₋₈-alkoxy-C₁₋₈-alkyl, C₁₋₈-alkylaminocarbonyl-C₁₋₈-alkyl, di-C₁₋₈-alkylaminocarbonyl-C₁₋₈-alkyl, C₁₋₈-alkyl-aminocarbonylamino-C₂₋₈-alkoxy, C₁₋₈-alkylaminocarbonylamino-C₁₋₈-alkyl, C₁₋₈-alkylcarbonylaminO-C₂₋₈-alkoxy, C₁₋₈-alkylcarbonylamino-C₁₋₈-alkyl, C₁₋₈-alkylsulfonyl-C₁₋₈-alkoxy, C₁₋₈-alkylsulfonyl-C₁₋₈-alkyl, C₁₋₈-alkylsulfonylamino-C₂₋₈-alkoxy, C₁₋₈-alkylsulfonylamino-C₁₋₈-alkyl, aryl-C₀₋₈-alkoxy, aryl-C₀₋₈-alkyl, optionally N-mono- oder N,N-di-C₁₋₈-alkylated carbamoyl-C₀₋₈-alkoxy, carboxy-C₁₋₈-alkoxy, carboxy-C₁₋₈-alkoxy-C₁₋₈-alkyl, carboxy-C₁₋₈-alkyl, C₁₋₈-alkyl, cyano, cyano-C₂₋₈-alkoxy, cyano-C₁₋₈-alkyl, C₃₋₈-cycloalkyl-C₁₋₈-alkoxy, C₃₋₈-cycloalkyl-carbamoyl-C₀₋₈-alkyl, C₃₋₈-cycloalkylcarbonylamino-C₂₋₈-alkoxy, C₃₋₈-cycloalkylcarbonylamino-C₁₋₈-alkyl, halogen, halogen-C₁₋₈-alkoxy, halogen-C₁₋₈-alkyl, heterocydyl-C₀₋₈-alkoxy, heterocydyl-C₀₋₈-alkyl, hydroxy-C₂₋₈-alkoxy-C₁₋₈-alkoxy, hydroxy-C₂₋₈-alkoxy-C₁₋₈-alkyl, oxide or oxo.

Examples of particularly preferred R¹ radicals are phenyl, pyridyl, 3-C₁₋₈-alkylindolyl, benzofuranyl, 4H-benzo[1,4]oxazin-3-onyl, 3,4-dihydro-2H-benzo[1,4]oxazinyl, 3,4-dihydro-2H-benzo[1,4]thiazinyl, 3,3-di-C₁₋₈-alkyl-1,3-dihydroindol-2-onyl, 3,3-di-C₁₋₈-alkyl-1,3-dihydroindolyl, indazolyl, indolyl, 1H-quinolinyl,2H-chromenyl, 1,1a,2,7b-tetrahydro-cyclo-propa[c]chromenyl, 1a,7b-dihydro-1H-cyclopropa[c]chromenyl, and spiro[cyclopropane-1,3']-2,3-dihydro-1H-indolyl which are substituted as specified above, especially by at least one substituent selected from halogen, oxide, oxo, C₁₋₈-alkoxy, C₁₋₈-alkoxy-C₁₋₈-alkoxy, C₁₋₈-alkoxy-C₁₋₈-alkyl, C₁₋₈-alkoxycarbonylamino-C₁₋₈-alkoxy, C₁₋₈-alkoxycarbonylamino-C₁₋₈-alkyl, C₁₋₈-alkyl, C₀₋₆-alkylcarbonylamino-C₁₋₈-alkoxy, C₀₋₆-alkylcarbonylamino-C₁₋₈-alkyl, N-acetyl-C₁₋₈-alkoxy-C₁₋₈-alkylamino, C₁₋₈-alkanoylamido-C₁₋₈-alkyl, N-C₁₋₈-alkyl-C₁₋₈-alkanoylamido-C₁₋₈-alkyl, C₁₋₈-alkoxy-C₁₋₈-alkoxy-C₁₋₈-alkyl, triazol-1-yl-C₁₋₈-alkyl, tetrazol-1-yl-C₁₋₈-alkyl, tetrazol-2-yl-C₁₋₈-alkyl, tetrazol-5-yl-C₁₋₈-alkyl, C₁₋₈-alkoxycarboxyl-C₁₋₈-alkyl, pyrrolidinonyl-C₁₋₈-alkyl, imidazolyl-C₁₋₈-alkyl, cyano-C₁₋₈-alkyl, carboxy-C₁₋₈-alkyl, carboxy-C₁₋₈-alkoxy, C₁₋₈-alkoxycarbonyl-C₀₋₆-alkyl, C₁₋₈-alkylsulfonamidyl-C₁₋₈-alkyl, C₁₋₈-alkoxy-C₁₋₈-alkanoylamido, C₁₋₈-alkoxy-C₁₋₈-alkanoylamido-C₁₋₈-alkyl, N-(C₁₋₈-alkyl)-C₁₋₈-alkoxy-C₁₋₈-alkanoylamido, N-C₁₋₈-alkylcarbamoyl-C₁₋₈-alkyl, C₃₋₈-cycloalkanoylamido-C₁₋₈-alkyl, C₁₋₈-alkylaminocarbonyl-amino-C₁₋₈-alkyl, C₁₋₈-alkanoylamidomethylpyrrolidinyl, N-(C₁₋₈-alkoxy-C₁₋₈-alkyl)carbamoyl, N-(C₁₋₈-alkoxy-C₁₋₈-alkyl)-N-(C₁₋₈-alkyl)carbamoyl, N-(C₁₋₈-alkoxy-C₁₋₈-alkyl)imidazol-2-yl, hydroxy-C₁₋₈-alkyl, hydroxy-C₁₋₈-alkoxy, hydroxy-C₁₋₈-alkoxy-C₁₋₈-alkyl, C₁₋₈-alkoxycarbonyl-amido-C₁₋₈-alkyl, amino-C₁₋₈-alkyl and C₁₋₈-alkylamino-C₁₋₈-alkyl.

Examples of very particularly preferred R¹ radicals are phenyl, pyridyl, and a bicyclic heterocyclyl, consisting of a phenyl-ring annulated to a 5-6 membered, saturated or unsaturated, heteocyclic ring, having from 1 to 3 nitrogen and/or 1 sulfur or oxygen atoms, such as 4H-benzo[1,4]oxazin-3-on-6-yl, 3,4-dihydro-2H-benzo[1,4]oxazin-6-yl, 3,4-dihydro-2H-benzo[1,4]thiazin-6-yl, 2,2-dimethyl-3,4-dihydro-2H-benzo[1,4]oxazin-6-yl, 2,2-dimethyl-4H-benzo[1,4]oxazin-3-on-6-yl, 4,4-dimethyl-1,4-dihydrobenzo[d][1,3]oxazin-2-on-8-yl, 3,3-dimethyl-1,3-dihydroindol-2-on-6-yl, 3,3-dimethyl-1,3-dihydroindol-2-on-7-yl, 3,3-dimethyl-1,3-dihydroindol-6-yl, 3-methylindol-6-yl, 1-methyl-indazol-5-yl, 3,4-dihydro-1 H-quinolin-2-on-7-yl, 4,4-dimethyl-3,4-dihydro-1 H-quinolin-2-on-8-yl, 1 H-quinolinyl, 2H-chromenyl, 1,1 a,2,7b-tetrahydro-cyclopropa[c]chromenyl, 1a,7b-dihydro-1 H-cyclopropa[c]chromenyl, and spiro[cyclopropane-1,3']-2,3-dihydro-1 H-indol-6-yl, which are substituted as specified above, especially by at least one substituent selected from C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkoxycarbonylamino-C₁₋₈-alkoxy, C₁₋₈-alkoxycarbonylamino-C₁₋₈-alkyl, C₁₋₈-alkyl, C₀₋₆-alkylcarbonylamino-C₁₋₈-alkoxy, C₀₋₆-alkylcarbonylamino-C₁₋₈-alkyl, halogen, oxide, triazol-1-yl-C₁₋₈-alkyl, and oxo, and, preferably, where, in the case that R¹ is a bicyclic heterocyclyl, the phenyl-ring of the bicyclic system is bonded to the rest of the molecule and at least the non-phenyl-ring is substituted as specified.

Examples of further very particularly preferred R¹ radicals are phenyl, 3,4-dihydro-2H-benzo[1,4]oxazin-6-yl and 3,3-dimethyl-1,3-dihydroindol-6-yl, which are substituted as specified above, especially by at least one substituent selected from C₁₋₈-alkoxy, C₁₋₈-alkoxy-C₁₋₈-alkoxy, C₁₋₈-alkoxy-C₁₋₈-alkyl, C₁₋₈-alkyl, C₀₋₆-alkylcarbonylamino-C₁₋₈-alkoxy, C₀₋₆-alkylcarbonylamino-C₁₋₈-alkyl and halogen, and, preferably, where, in the case that R¹ is a bicyclic heterocyclyl, the phenyl-ring of the bicyclic system is bonded to the rest of the molecule and at least the non-phenyl-ring is substituted as specified.

Examples of preferred X radicals are -Alk-R¹, -O-Alk-, -O-Alk-O-R¹, -C(O)-NR⁶-R¹ or -O-Alk-C(O)-NR⁶-R¹, where Alk represents C₁₋₈-alkylene, which is optionally substituted with halogen. Particularly preferred for X is -O-Alk-R¹.

The invention further provides a process for enhancing the membrane transportation of compounds of formula (I), or preferably of the formula (IA), and their pharmaceutically useable salts characterized in that
(i) a radical (A) is present in at least one of R⁴, R⁵ or Q; or else
(ii) at least Q is a group of the formula -C(O)OCHR⁶OC(O)R⁷.

The compounds of formula (I), or preferably of the formula (IA), and their pharmaceutically useable salts have inhibiting action on the natural enzyme renin. The latter passes from the kidneys into the blood and there brings about the cleavage of angiotensinogen to form the decapeptide angiotensin l which is then cleaved in the lung, the kidneys and other organs to the octapeptide angiotensin II. Angiotensin II increases the blood pressure both directly by arterial constriction and indirectly by the release of the hormone aldosterone which inhibits the release of the sodium ion from the adrenal glands, which is associated with a rise in the extracellular liquid volume. This rise can be attributed to the action of angiotensin II itself or of the heptapeptide angiotensin III formed therefrom as a cleavage product. Inhibitors of the enzymatic activity of renin bring about a reduction in the formation of angiotensin I and, as a consequence thereof, the formation of a smaller amount of angiotensin II. The reduced concentration of this active peptide hormone is the immediate cause of the hypotensive action of renin inhibitors.

One experimental procedure of detecting the action of transporters, in particular PEPT1, is by means of *in vitro* tests, in which inhibition of [¹⁴C]Gly-Sar uptake is measured. One *in vitro* test which is used is the one according to Faria et al. J. Med. Chem. 49(12), 3636-3644, (2006).

This assay uses a stably transfected cell line which expresses the rPepT1 transporter. [¹⁴C]Gly-Sar (10 µM) is added together with an excess of an unlabelled competitor (0.1 - 9 mM) onto the monolayer cell culture grown in microtiter plates. The uptake of [¹⁴ C]Gly-Sar is determined in a subsequent radioimmunoassay measuring the [14C]Gly-Sar content in the solubilized cells. The IC₅₀ is defined as the concentration of the particular competitor which reduces the uptake of [¹⁴C]Gly-Sar by 50%. The IC₅₀ values are estimated by nonlinear regression analysis. Michaelis-Menten-like kinetic parameters (Kₘ, Vₘₐₓ) are determined by nonlinear curve fitting of specific uptake data to the following equation: V₀ = Vₘₐₓ[S]/(Kₘ+[S]), where V₀ is the initial uptake velocity, Vₘₐₓ is the maximal uptake velocity at saturating substrate concentrations, Kₘ is a constant analogous to the Michaelis-Menten constant and S is the substrate concentration. The compounds of the present invention which are substrates of PEPT1 show inhibitory effects in the in vitro system at minimal concentrations of about 10⁻² to about 10⁻⁵ mol/l.

The pharmacokinetic properties of the compounds described herein can be tested *in vivo* using the following protocol: The investigations take place in pre-catheterized (carotid artery) male rats (300 g ±20%) that can move freely throughout the study. The compound is administered intravenously and orally (gavage) in separate sets of animals. The applied doses for oral administration may range from 0.5 to 50 mg/kg body weight; the doses for intravenous administration may range from 0.5 to 20 mg/kg body weight. Blood samples are collected through the catheter before compound administration and over the subsequent 24-hour period using an automated sampling device (AccuSampler, DiLab Europe, Lund, Sweden). Plasma levels of the compound are determined using a validated LC-MS analytical method. The pharmacokinetic analysis is performed on the plasma concentration-time curves after averaging all plasma concentrations across time points for each route of administration. Typical pharmacokinetics parameters to be calculated include: maximum concentration (Cₘₐₓ), time to maximum concentration (tₘₐₓ), area under the curve from 0 hours to the time point of the last quantifiable concentration (AUC₀₋ₜ), area under the curve from time 0 to infinity (AUC_{0-inf}), elimination rate constant (K), terminal half-life (t_{½}), absolute oral bioavailability or fraction absorbed (F), clearance (CL), and volume of distribution during the terminal phase (Vd). The compounds of the present invention effectively increase the concentration of parent compound in plasma in the *in vivo* test described at doses of about 0.3 to about 30 mg/kg p.o.

In salt-depleted animals, renin inhibitors bring about a blood pressure decrease. Human renin differs from renin of other species. To test inhibitors of human renin, primates (marmosets, Callithrixjacchus) are used, because human renin and primate renin are substantially homologous in the enzymatically active region. One in vivo test which is used is as follows: the test compounds are tested on normotensive marmosets of both genders and having a body weight of about 350 g which are conscious, able to move freely and in their normal cages. Blood pressure and heart rate are measured using a catheter in the descending aorta and recorded radiometrically. The endogenous release of renin is stimulated by the combination of a 1-week low-salt diet with a single intra-muscular injection of furosemide (5-(aminosulfonyl)-4-chloro-2-[(2-furanylmethyl)amino]benzoic acid) (5 mg/kg). 16 hours after the injection of furosemide, the test substances are administered either directly into the femoral artery by means of an injection cannular or into the stomach by gavage as a suspension or solution, and their effect on blood pressure and heart rate was evaluated. The compounds of the present invention effectively reduce blood pressure in the in vivo test described at doses of about 0.003 to about 0.3 mg/kg i.v. and at doses of about 0.3 to about 30 mg/kg p.o.

The compounds of formula (I), or preferably of the formula (IA), and their pharmaceutically useable salts may find use as medicaments, for example in the form of pharmaceutical preparations. The pharmaceutical preparations may be administered enterally, such as orally, for example in the form of tablets, coated tablets, sugar-coated tablets, hard and soft gelatine capsules, solutions, emulsions or suspensions, nasally, for example in the form of nasal sprays, rectally, for example in the form of suppositories, or transdermally, for example in the form of ointments or patches. The administration may also be parenteral, such as intramuscular or intravenous, for example in the form of injection solutions.

To prepare tablets, coated tablets, sugar-coated tablets and hard gelatine capsules, the compounds of formula (I), or preferably of the formula (lA), and pharmaceutically useable salts thereof, may be processed with pharmaceutically inert, inorganic or organic excipients. Such excipients used, for example for tablets, coated tablets and hard gelatine capsules, may be lactose, corn starch, or derivatives thereof, talc, stearic acid or salts thereof etc. Suitable excipients for soft gelatine capsules are, for example, vegetable oils, waxes, fats, semisolid and liquid polyols, etc.

Suitable excipients for preparing solutions and syrups are, for example, water, polyols, sucrose, invert sugar, glucose, etc.

Suitable excipients for injection solutions are, for example, water, alcohols, polyols, glycerol, vegetable oils, bile acids, lecithin, etc.

Suitable excipients for suppositories are, for example, natural or hardened oils, waxes, fats, semisolid or liquid polyols, etc.

The pharmaceutical preparations may additionally also comprise preservatives, solubilizers, viscosity-increasing substances, stabilizers, wetting agents, emulsifiers, sweeteners, colorants, flavourings, salts for altering the osmotic pressure, buffers, coatings or antioxidants. They may also comprise other therapeutically valuable substances.

The present invention further provides the use of the compounds of formula (I), or preferably of the formula (IA), and the pharmaceutically useable salts thereof, in the treatment or prevention of hypertension and heart failure, and also glaucoma, cardiac infarction, kidney failure, restenoses and stroke of mammals, especially of human beings.

The compounds of formula (I), or preferably of the formulae (IA), and the pharmaceutically useable salts thereof, may also be administered in combination with one or more agents having cardiovascular action, for example α- and β-blockers such as phentolamine, phenoxybenzamine, prazosin, terazosin, tolazine, atenolol, metoprolol, nadolol, propranolol, timolol, carteolol etc.; vasodilators such as hydralazine, minoxidil, diazoxide, nitroprusside, flosequinan etc.; calcium antagonists such as amrinone, bencyclan, diltiazem, fendiline, flunarizine, nicardipine, nimodipine, perhexilene, verapamil, gallopamil, nifedipine etc.; ACE inhibitors such as cilazapril, captopril, enalapril, lisinopril etc.; potassium activators such as pinacidil; anti-serotoninergics such as ketanserin; thromboxane-synthetase inhibitors; neutral endopeptidase inhibitors (NEP inhibitors); angiotensin II antagonists; and also diuretics such as hydrochlorothiazide, chlorothiazide, acetazolamide, amiloride, bumetanide, benzthiazide, ethacrynic acid, furosemide, indacrinone, metolazone, spironolactone, triamteren, chlorthalidone etc.; sympatholytics such as methyldopa, clonidine, guanabenz, reserpine; and other agents which are suitable for the treatment of hypertension, heart failure or vascular diseases in humans and animals which are associated with diabetes or renal disorders such as acute or chronic renal failure. Such combinations may be employed separately or in preparations which comprise a plurality of components.

Further substances which can be used in combination with the compounds of formula (I), or preferably of the formula (IA), are the compounds of classes (i) to (ix) on page 1 of

WO 02/40007 (and also the preferences and examples further listed therein) and the substances specified on pages 20 and 21 of WO 03/027091.

The dose may vary within wide limits and has of course to be adapted to the individual circumstances in each individual case. In general, for oral administration, a daily dose of about 3 mg to about 3 g, preferably about 10 mg to about 1 g, for example about 300 mg, per adult (70 kg), divided into preferably 1-3 individual doses which may, for example, be of equal size, may be appropriate, although the upper limit specified may also be exceeded if this should be found to be appropriate; typically, children receive a lower dose according to their age and body weight.

### Examples

The examples which follow illustrate the present invention. All temperatures are reported in degrees Celsius, pressures in mbar. Unless stated otherwise, the reactions take place at room temperature. The abbreviation "Rf = xx (A)" means, for example, that the Rf value xx is obtained in the solvent system A. The ratio of the solvents relative to one another is always reported in parts by volume. Chemical names of end products and intermediates were obtained with the aid of the program AutoNom 2000 (Automatic Nomenclature).

HPLC gradient on Hypersil BDS C-18 (5 µm); column: 4 x 125 mm
90% water*/10% acetonitrile* to 0% water*/100% acetonitrile* in 5 minutes + 2.5 minutes (1.5 ml/min); *: containing 0.1 % trifluoroacetic acid

The following abbreviations are used:
- Rf: ratio of distance which a substance travels to distance of the eluent front from the start point in thin layer chromatography
- Rt: retention time of a substance in HPLC (in minutes)
- m.p.: melting point (temperature)

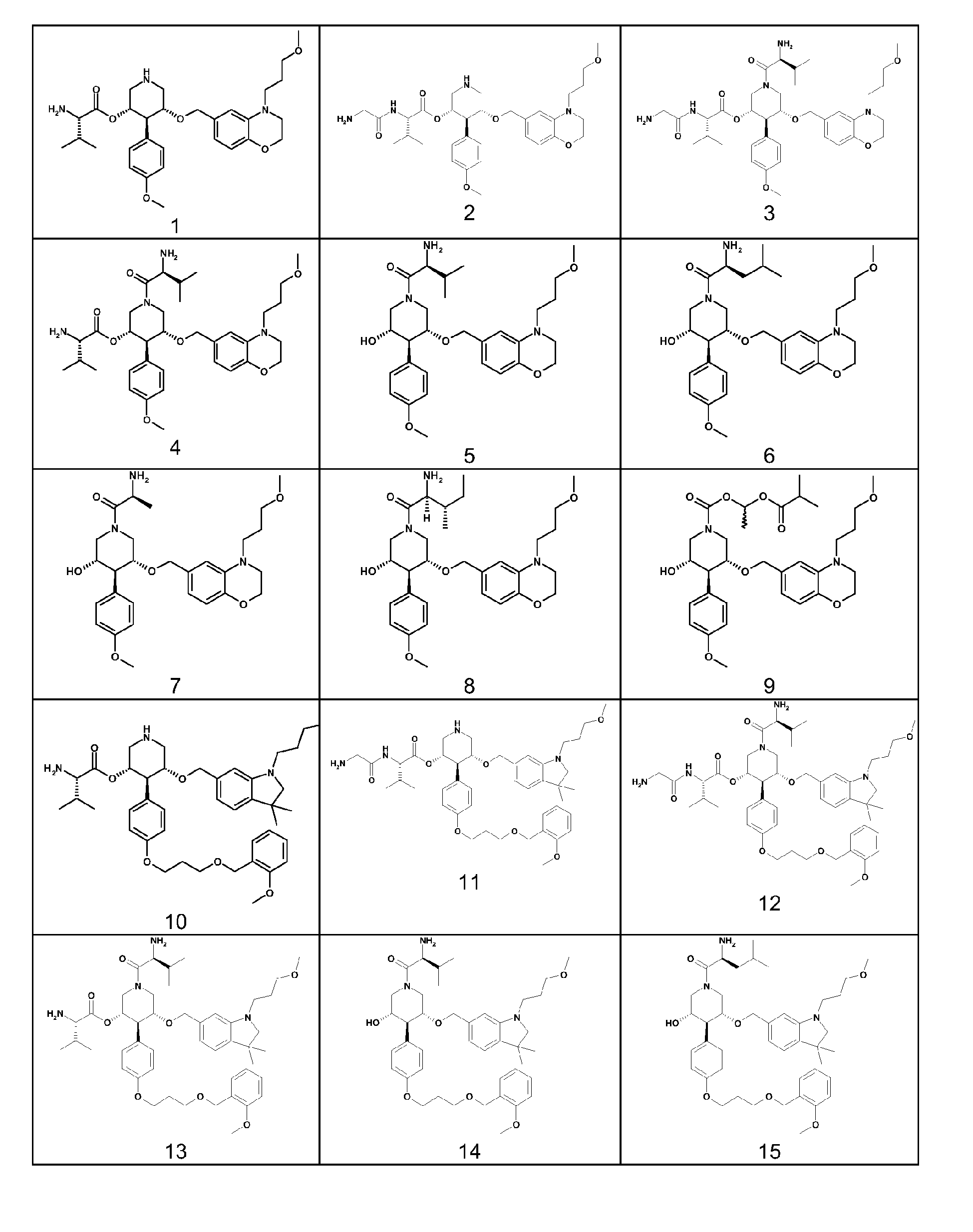

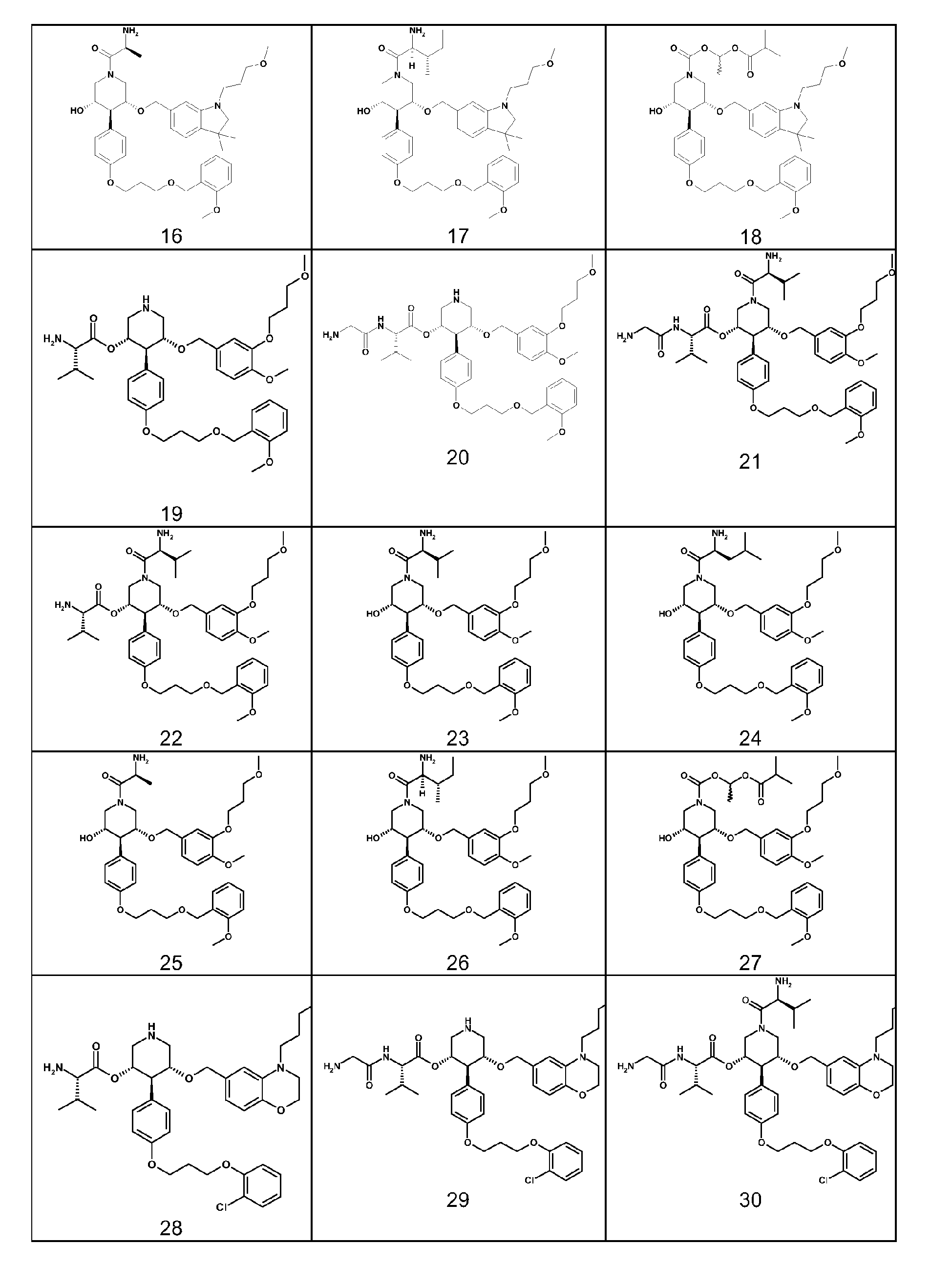

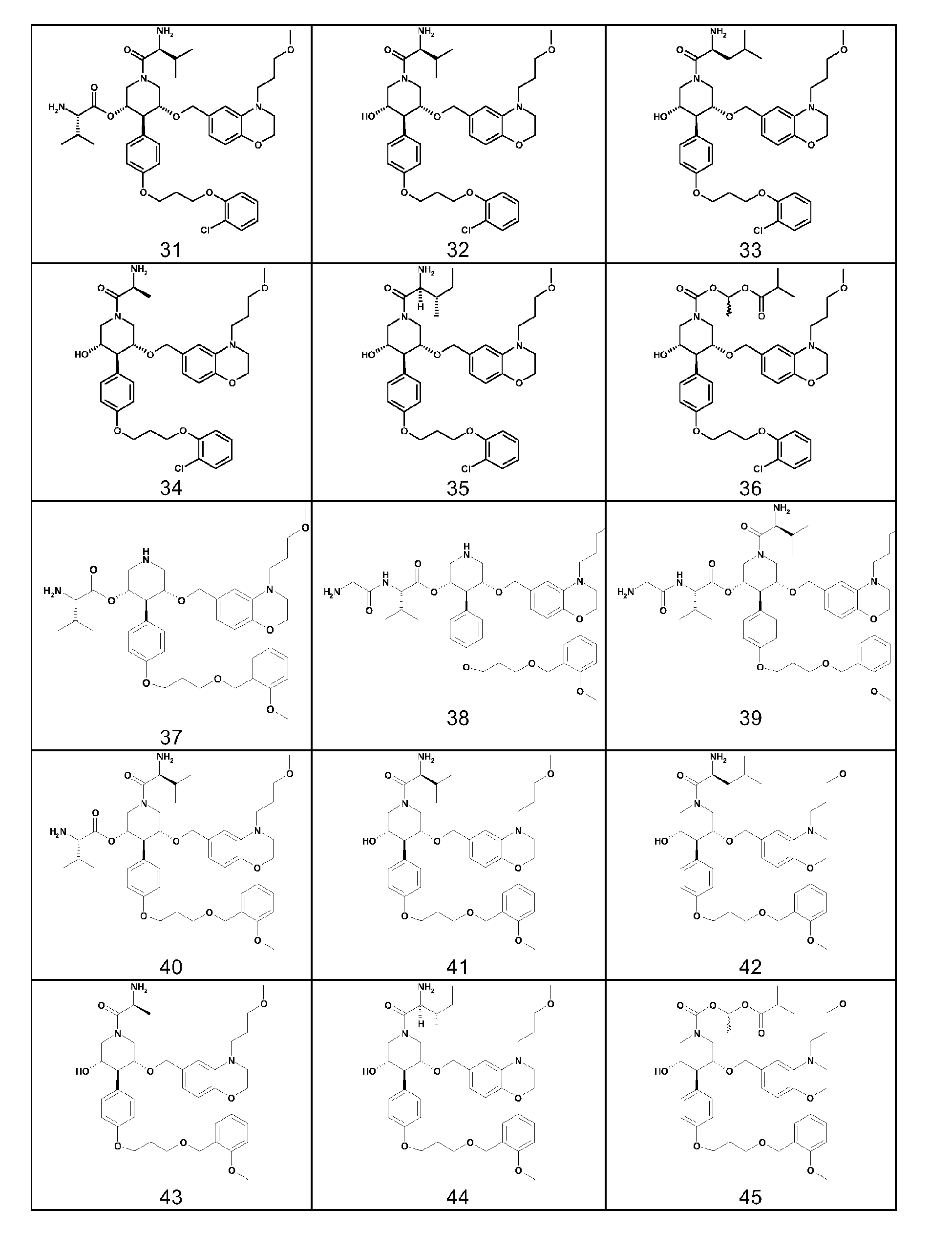

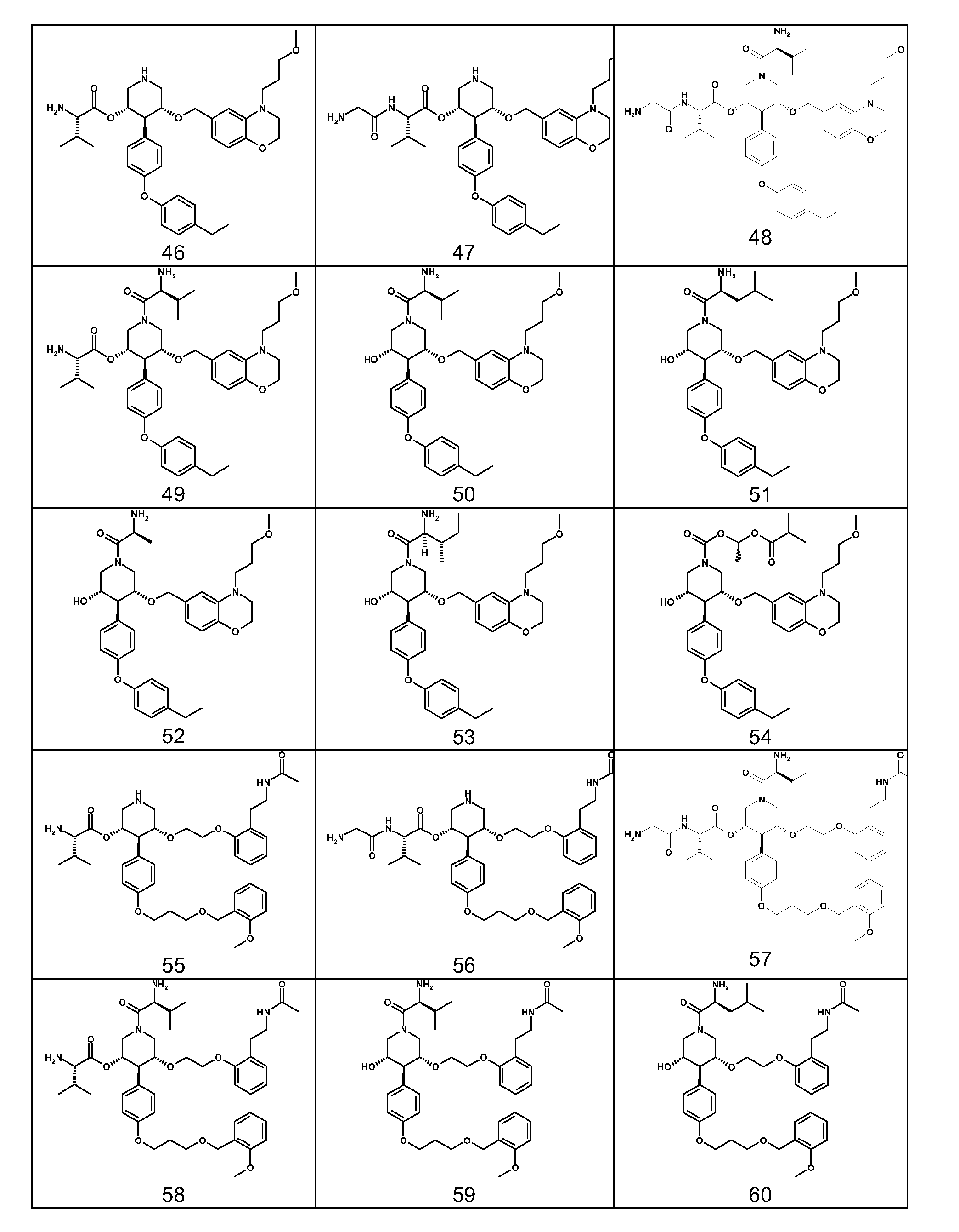

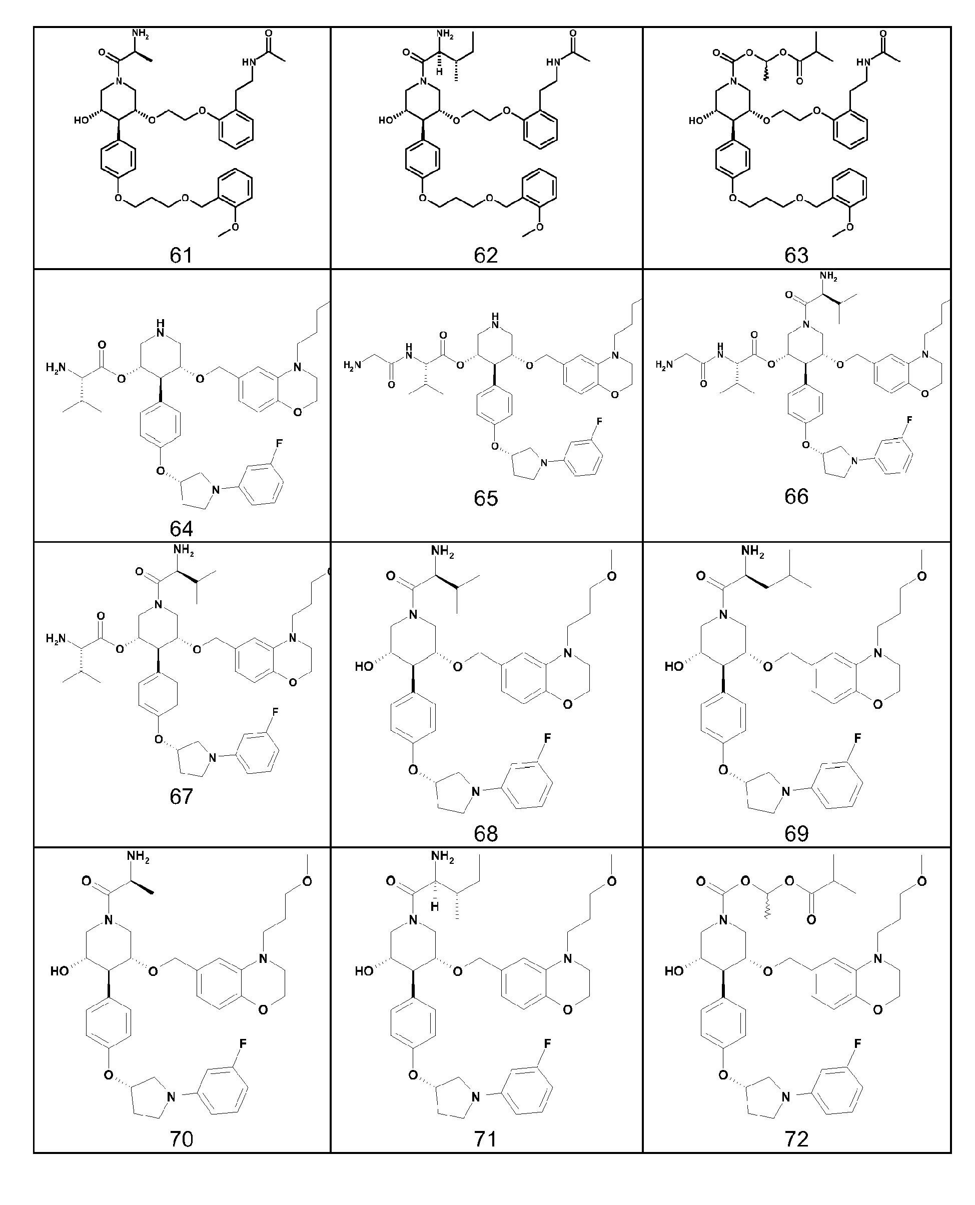

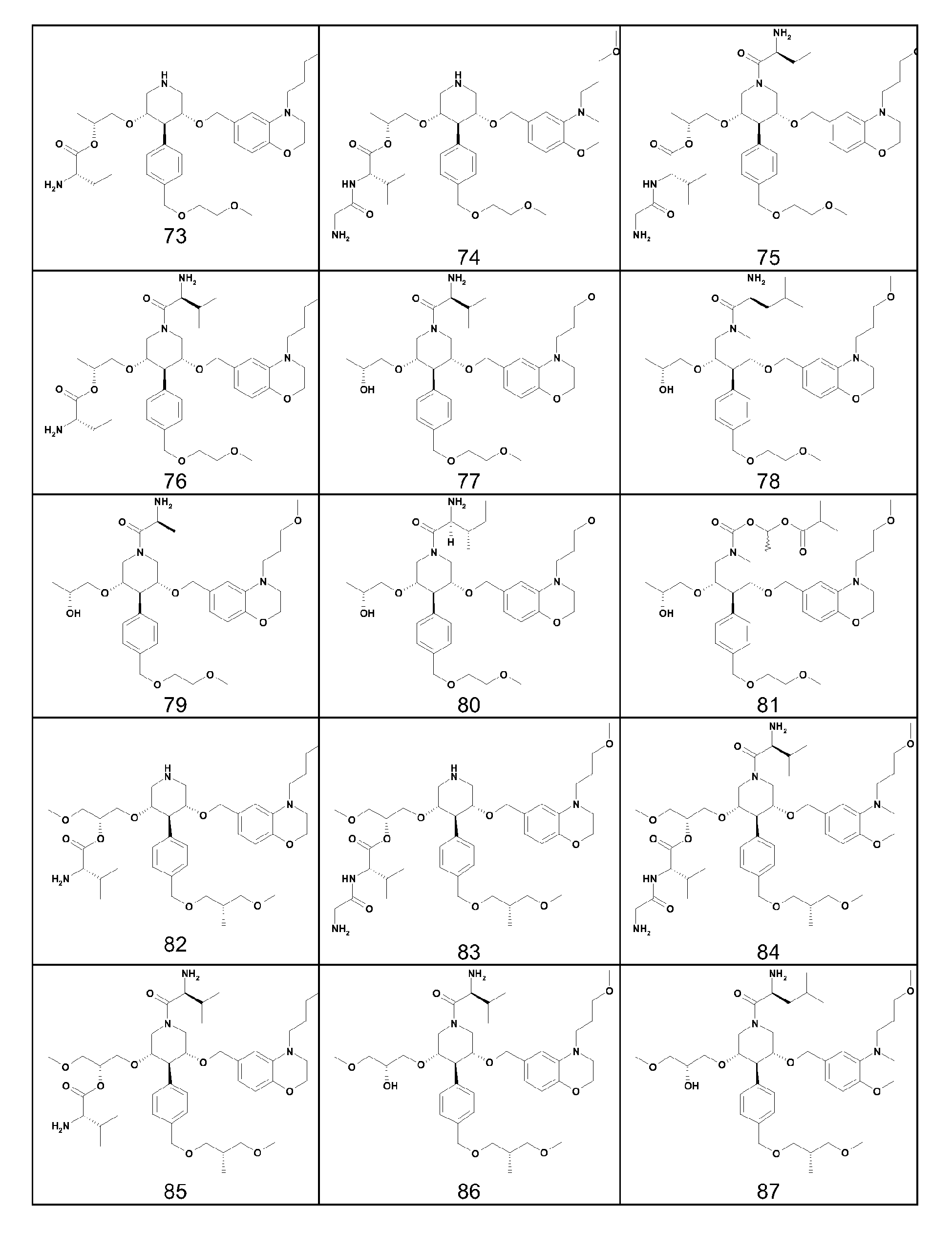

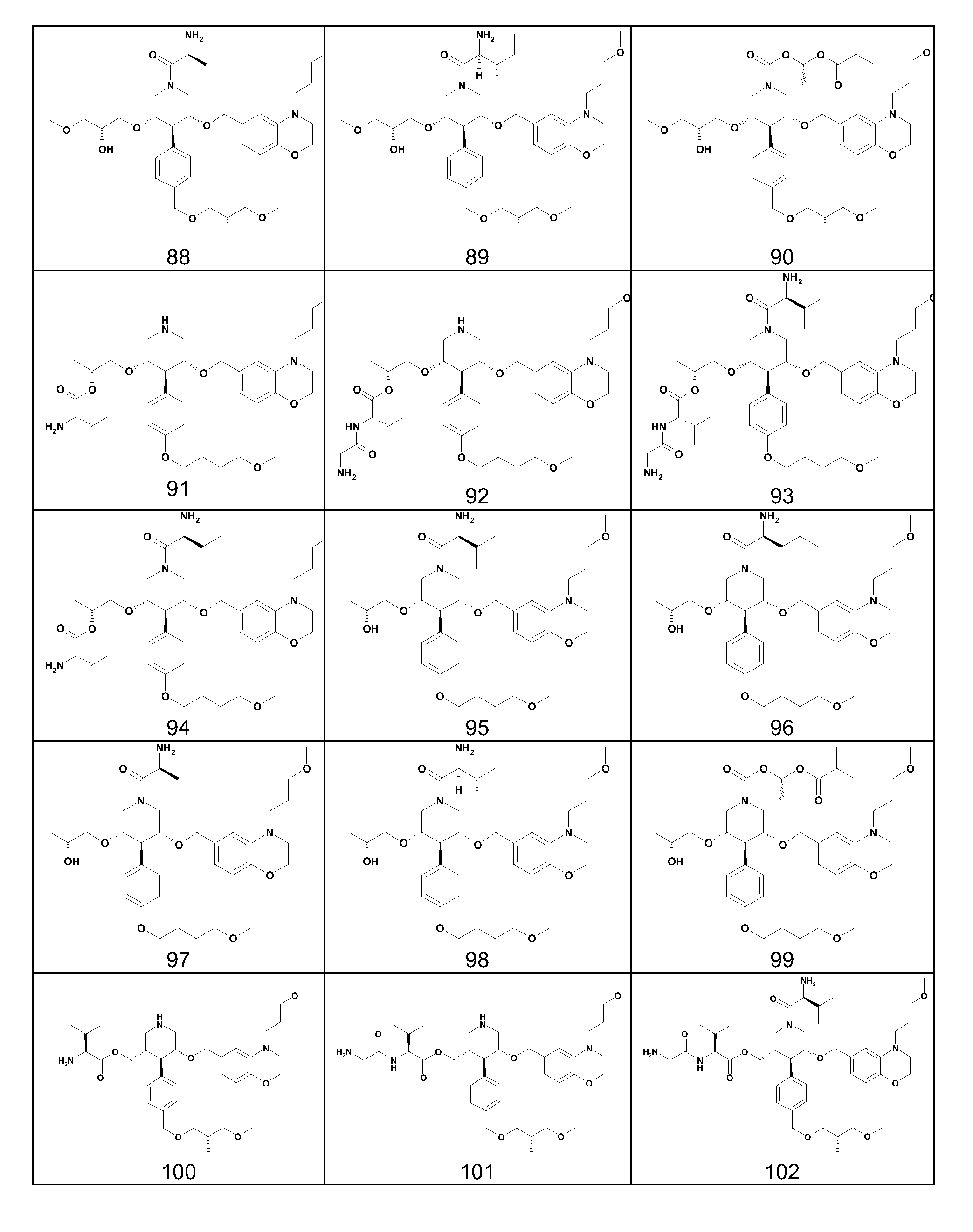

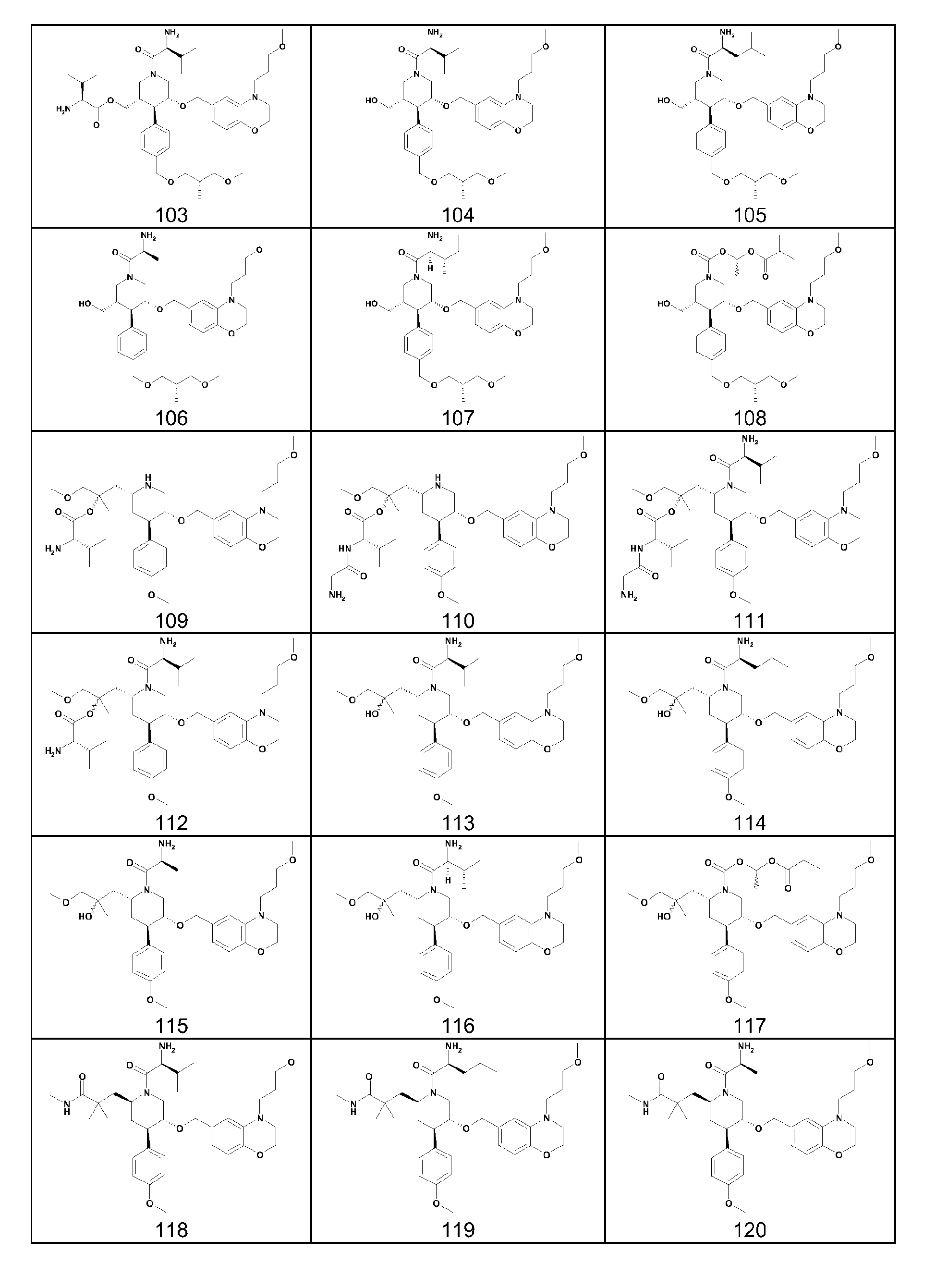

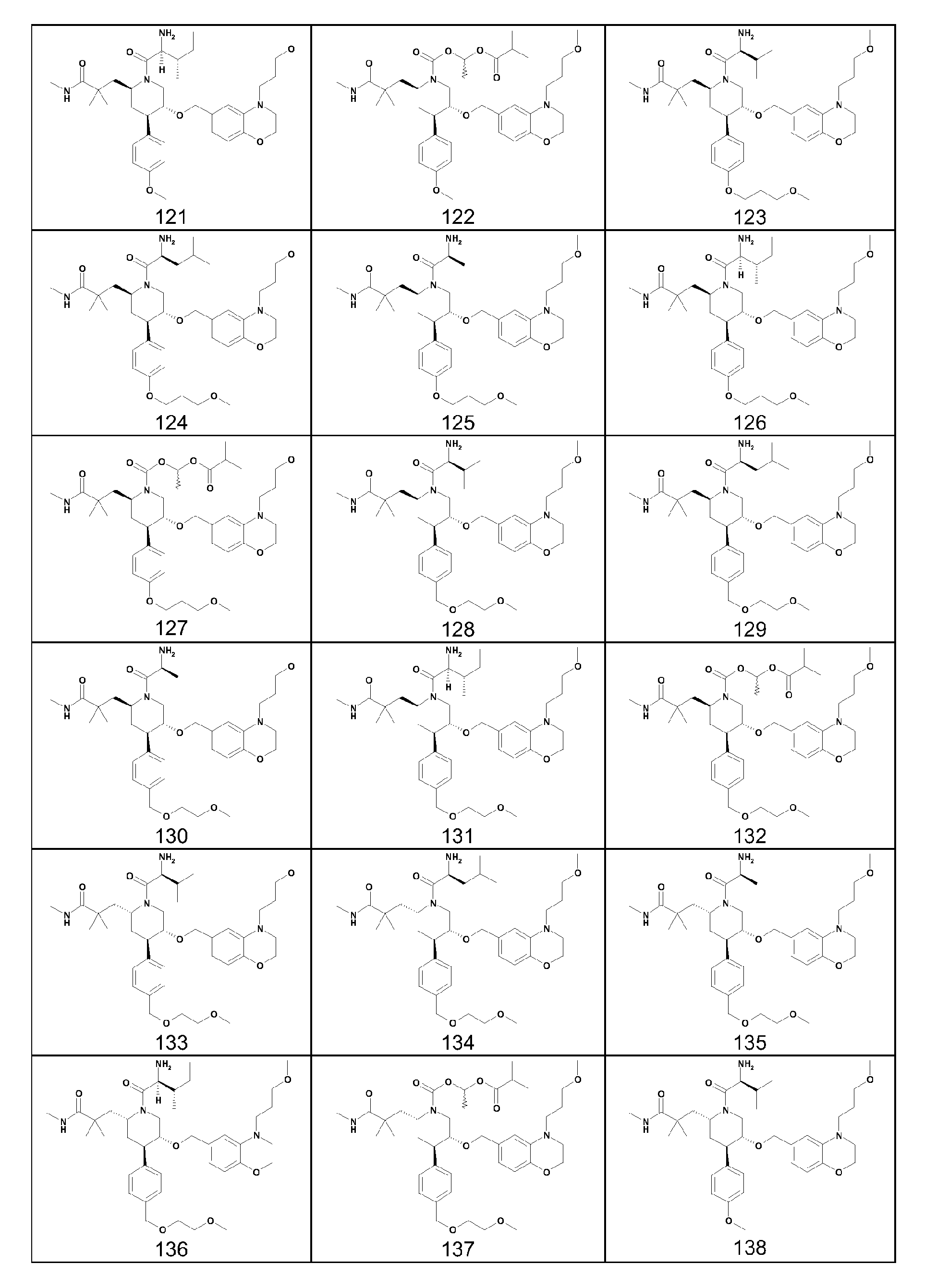

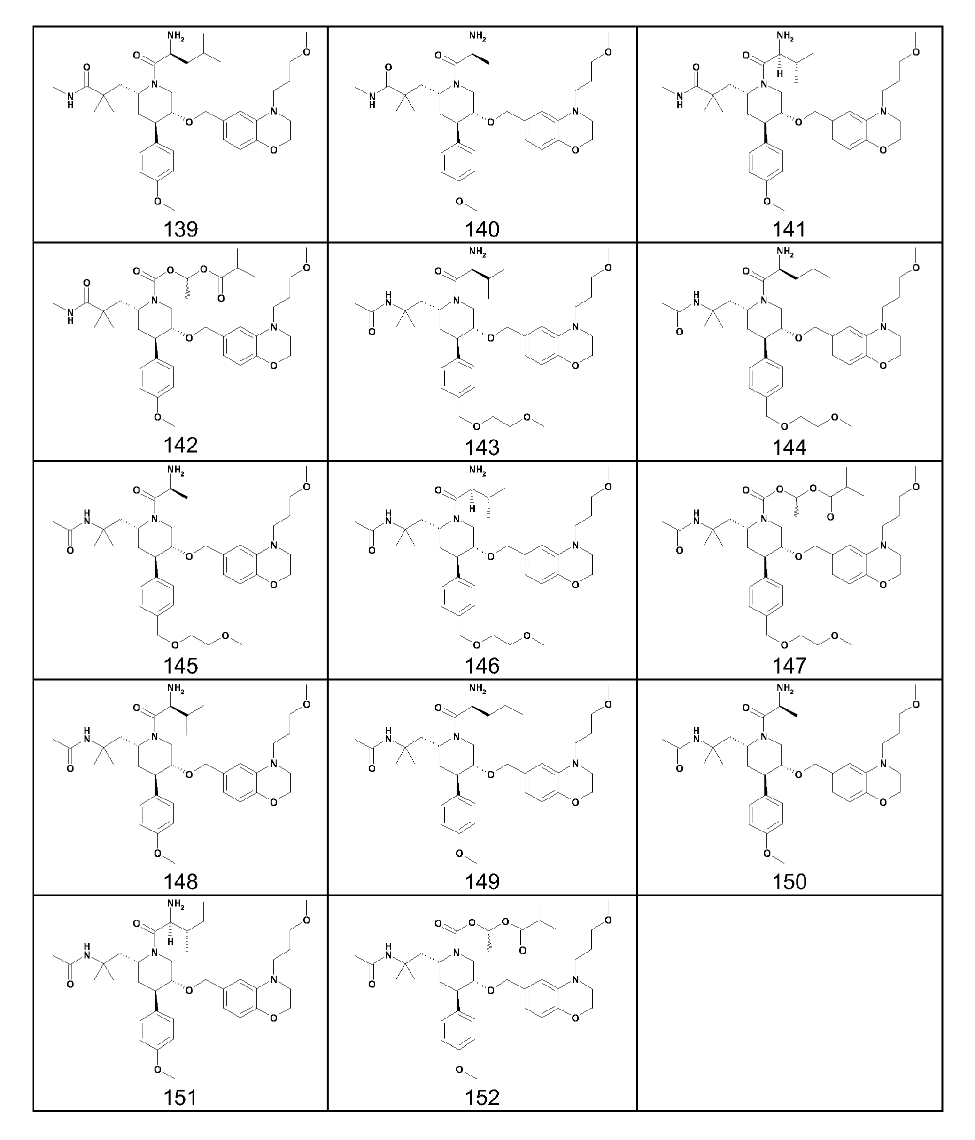

### General procedure A (N-Tos-deprotection)

To a stirred solution of 0.09 mmol "tosylamide" in 10 ml of methanol are added 0.44 mmol sodium dihydrogenphosphate and 0.90 mmol of sodium amalgam (10% Na) at room temperature. The reaction mixture is stirred for 2-18 hours, diluted with water and extracted with ethyl acetate. The organic phases are combined, washed with brine and dried over sodium sulfate. The solvent is concentrated under reduced pressure and the residue is purified by flash chromatography (SiO₂ 60F) to afford the title compound.

### General procedure B: (N-p-methoxyphenyl-deprotection)

To a stirred solution of 0.5 mmol "p-methoxyaniline" in 10 ml of acetonitrile/water (1:1) is added a solution of 1.43 mol of ceric ammonium nitrate in 5.0 ml of water at 0°C. The mixture is stirred for 30 min, followed by addition of 1.0 g of sodium sulfite. After additional 30 min, the mixture is diluted with water and extracted with tert-butyl methyl ether. The organic phase is dried and concentrated under reduced pressure. The residue is purified by flash chromatography (SiO₂ 60F) to afford the title compound.

### General procedure C: (BH₃-reduction)

To a stirred solution of 1 mmol of "lactam" in 3 ml of tetrahydrofuran is admixed with 2-4 mmol of borane tetrahydrofuran (1M in tetrahydrofuran) and heated to 50°C for 2-8 hours. The reaction mixture is quenched by addition of 10 ml of methanol and concentrated under reduced pressure. The title compound is obtained from the residue by means of flash chromatography (SiO₂ 60F).

### General procedure D: (amide-formation I)

To a stirred solution of 1.0 mmol of "acid" and "1.0 mmol of "amine" in 20 ml of dichloromethane are added 5.0 mmol of triethylamine and 1.0 mmol of tri-propylphosphonic acid cyclic anhydride [68957-94-8] (50% in ethyl acetate) at room temperature. The reaction mixture is stirred for 1-3 hours, diluted with dichloromethane, washed with 1 N hydrochloric acid and brine. The organic phases are combined, dried over sodium sulfate and the solvent is concentrated under reduced pressure. The residue is purified by flash chromatography (SiO₂ 60F) to afford the title compound.

### General procedure E: (N-Cbz-deprotection, N-(1-phenyl-ethyl)-deprotection or N-benzyl-deprotection)

To a stirred solution of 1 mmol of "N-protected derivative" in 15 ml of tetrahydrofuran are added 0.1 mmol Pd/C 10% and the reaction mixture is hydrogenated at room temperature. The reaction mixture is filtered and concentrated under reduced pressure. The residue is purified by flash chromatography (SiO₂ 60F) to afford the title compound.

### General procedure F: (mesylation)

To a stirred solution of 1 mmol of "alcohol" in 10 ml of dichloromethane are added 5 mmol of triethylamine and 2 mmol of methanesulfonyl chloride at 0°C. The reaction mixture is allowed to stir for 1 hour, diluted with dichloromethane, washed with 1 N hydrochloric acid and dried over sodium sulfate. The solvent is concentrated under reduced pressure and the residue is purified by flash chromatography (SiO₂ 60F) to afford the title compound or is directly used in the next step without any further purification.

### General procedure G: (amide-formation II)

To a stirred solution of 1.0 mmol of "amine" in 40 ml of dichloromethane are added 1.5 mmol of "acid" and 0.2 mmol of N,N-dimethylaminopyridine The reaction mixture is cooled to 0°C and 1.5 mmol of N-ethyl-N'-(3-dimethylaminopropyl)carbodiimid (EDC) is added. The reaction mixture is stirred at 0°C for 3-8 hours, washed with 1M aqueous ammonium chloride solution. The aqueous phase is re-extracted with dichloromethane. The combined organic phases are washed with water, dried over sodium sulfate and the solvent is concentrated under reduced pressure. The residue is purified by flash chromatography (SiO₂ 60F) to afford the title compound.

### General procedure H: (N-Cbz-protection)

To a stirred solution of 1 mmol of "amine" in 30 ml of ethyl acetate and 15 ml saturated aqueous sodium carbonate solution at 0°C is treated with 1.1 mmol benzyl chloroformate. The reaction mixture is allowed to reach room temperature and stirred for 8 hours at room temperature. The phases are separated and the aqueous phase is re-extracted with ethyl acetate. The combined organic phases are dried over sodium sulfate and the solvent is concentrated under reduced pressure. The residue is purified by flash chromatography (SiO₂ 60F) to afford the title compound.

### General procedure I (alcohol-desilylation)

A solution of 1 mmol of "silyl ether" in 5 ml of tetrahydrofuran is treated with 1.5 - 2.0 mmol of tetrabutylammoniumfluorid (1M solution in tetrahydrofuran) and the solution is stirred for 1 - 2 hours at room temperature. The solution is then diluted with water and extracted with tert-butyl methyl ether (2X). The combined organic phases are dried over sodium sulfate and the solvent is concentrated under reduced pressure. The residue is purified by flash chromatography (SiO₂ 60F) to afford the title compound.

### General procedure J (borane-reduction)

A solution of 1 mmol of "substrat" in 3 ml of tetrahydrofuran is treated with 3.0 - 6.0 mmol of borane-tetrahydrofuran-complex (1M in tetrahydrofuran) and stirred for 1 - 3 hours at room temperature (reaction control by HPLC or TLC). The reaction mixture is treated with 3.0 - 6.0 mmol of methanol and evaporated. The residue is purified by flash chromatography (SiO₂ 60F) to afford the title compound.

### General procedure K: (O-alkylation)

A solution of 1 mmol of "alcohol" and 1.0 - 2.0 mmol of "halide" in 2.0 ml of N,N-dimethylformamide is stirred at -10°C and treated with 1.1 mmol of sodium hydrid (60% dispersion in oil). The reaction mixture is stirred for 1 hour at -10°C and then for 18 hours at room temperature. The mixture is poured on 1M aqueous sodium bicarbonate solution and extracted with tert-butyl methyl ether (2X). The combined organic layers are washed with water (1X) and brine (1X) and dried over sodium sulfate. The solvent is concentrated under reduced pressure and the residue is purified by flash chromatography (SiO₂ 60F) to afford the title compound.

### General procedure L: (phenol-alkylation)

A suspension of 1 mmol of "tosylate" or "halide", 2 mmol of "phenol", 2 mmol of potassium carbonate and 20 ml of acetonitrile is stirred at 90°C for 24 hours. The reaction mixture is concentrated under reduced pressure and the residue is extracted with ethyl acetate (2X). The organic phases are washed with brine, dried over sodium sulfate and concentrated under reduced pressure. The residue is purified by flash chromatography (SiO₂ 60F) to afford the title compound.

### General procedure M: (tosylation)

A solution of 12 mmol of p-toluenesulfonylchloride in 15 ml of dichloromethane is added drop wise to a solution of 10 mmol of "alcohol" or "amine", 15 mmol of triethylamine and 1 mmol of 4-dimethylaminopyridine in 90 ml of dichloromethane at 0°C. The reaction mixture is stirred for 2-18 hours at room temperature, then diluted with dichloromethane and washed with water and brine and dried over sodium sulfate. The solvent is concentrated under reduced pressure. The residue is purified by flash chromatography (SiO₂ 60F) to afford the title compound.

### General procedure N (O-alkylation II)

A solution of 1 mmol of "secondary alcohol" in 5 ml of tetrahydrofuran is treated with 1 mmol of methyl magnesiumbromid (35% solution in diethyl ether) at room temperature. The reaction mixture is heated to reflux for 5 minutes and then treated with a solution of 2.2 mmol of "oxirane" in 1 ml of tetrahydrofuran. The reaction mixture is heated to reflux for 1-5 hours, then poured on saturated aqueous sodium bicarbonate solution and extracted with tert-butyl-methyl ether (3X). The combined organic phases are dried over sodium sulfate and the solvent is concentrated under reduced pressure. The residue is purified by flash chromatography (SiO₂ 60F) to afford the title compound.

### Example 1

### (S)-2-Amino-3-methyl-butyric acid (3S,4R,5R)-4-(4-methoxy-phenyl)-5-[4-(3-methoxy-propyl)-3,4-dihydro-2H-benzo[1,4]oxazin-6-ylmethoxy]-piperidin-3-yl ester

According to general procedure E, (3S,4R,5R)-3-((S)-2-benzyloxycarbonylamino-3-methyl-butyryloxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester is used to afford the title compound, which is identified based on its Rf value.

The starting materials are prepared as follows:
a) (3S,4R,5R)-3-((S)-2-Benzyloxycarbonylamino-3-methyl-butyryloxy)-4-(4-methoxyphenyl)-5-[4-(3-methoxy-propyl)-3,4-dihydro-2H-benzo[1,4]oxazin-6-ylmethoxy]piperidine-1-carboxylic acid benzyl ester
   According to general procedure G, (3S,4S,5R)-3-hydroxy-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester and (S)-2-benzyloxycarbonylamino-3-methyl-butyric acid [1149-26-4] are used to afford the title compound, which is identified based on its Rf value.
b) (3S,4S,5R)-3-Hydroxy-4-(4-methoxy-phenyl)-5-[4-(3-methoxy-propyl)-3,4-dihydro-2H-benzo[1,4]oxazin-6-ylmethoxyl-piperidine-1-carboxylic acid benzyl ester
   According to general procedure H, (3S,4S,5R)-4-(4-methoxy-phenyl)-5-[4-(3-methoxy-propyl)-3,4-dihydro-2H-benzo[1,4]oxazin-6-ylmethoxy]-piperidin-3-ol [873945-55-2] is used to afford the title compound, which is identified based on its Rf value.

According to the procedure described in example 1, the following compounds are prepared in analogous manner:
10 (S)-2-Amino-3-methyl-butyric acid (3S,4R,5R)-4-{4-[3-(2-methoxy-benzyloxy)-propoxyl-phenyl}-5-[1-(3-methoxy-propyl)-3,3-dimethyl-2,3-dihydro-1H-indol-6-ylmethoxy]-piperidin-3-yl ester
   starting from (3S,4S,5R)-3-hydroxy-4-{4-[3-(2-methoxy-benzyloxy)-propoxy]-phenyl}-5-[1-(3-methoxy-propyl)-3,3-dimethyl-2,3-dihydro-1H-indol-6-ylmethoxy]-piperidine-1-carboxylic acid benzyl ester.

The starting materials are prepared as follows:
a) (3S,4S,5R)-3-Hydroxy-4-{4-[3-(2-methoxy-benzyloxy)-propoxy]-phenyl}-5-[1-(3-methoxy-propyl)-3,3-dimethyl-2,3-dihydro-1H-indol-6-ylmethoxy]-piperidine-1-carboxylic acid benzyl ester
   According to general procedure I, (3R,4R,5S)-4-{4-[3-(2-methoxy-benzyloxy)-propoxy]-phenyl}-3-[1-(3-methoxy-propyl)-3,3-dimethyl-2,3-dihydro-1 H-indol-6-ylmethoxy]-5-triisopropylsilanyloxy-piperidine-1-carboxylic acid benzyl ester is used to afford the title compound, which is identified based on its Rf value.
b) (3R,4R,5S)-4-{4-[3-(2-Methoxy-benzyloxy)-propoxy]-phenyl}-3-[1-(3-methoxy-propyl)-3,3-dimethyl-2,3-dihydro-1H-indol-6-ylmethoxy]-5-triisopropylsilanyloxy-piperidine-1-carboxylic acid benzyl ester
   According to general procedure J, (3R,4R,5S)-4-{4-[3-(2-methoxy-benzyloxy)-propoxy]-phenyl}-3-[1-(3-methoxy-propyl)-3,3-dimethyl-2-oxo-2,3-dihydro-1H-indol-6-ylmethoxy]-5-triisopropylsilanyloxy-piperidine-1-carboxylic acid benzyl ester is used to afford the title compound, which is identified based on its Rf value.
c) (3R,4R,5S)-4-{4-[3-(2-Methoxy-benzyloxy)-propoxy]-phenyl}-3-[1-(3-methoxy-propyl)-3,3-dimethyl-2-oxo-2,3-dihydro-1H-indol-6-ylmethoxy]-5-triisopropy]silanyloxy-piperidine-1-carboxylic acid benzyl ester
   According to general procedure K, (3R,4R,5S)-3-hydroxy-4-{4-[3-(2-methoxy-benzyloxy)-propoxy]-phenyl}-5-triisopropylsilanyloxy-piperidin-1-carboxylic acid benzyl ester [873945-54-1] and 6-bromomethyl-1-(3-methoxy-propyl)-3,3-dimethyl-1,3-dihydro-indol-2-one [857274-09-0] are used to afford the title compound, which is identified based on its Rf value.

### 19 (S)-2-Amino-3-methyl-butyric acid (3S,4R,5R)-4-{4-[3-(2-methoxy-benzyloxy)-propoxyl-phenyl}-5-[4-methoxy-3-(3-methoxy-propoxy)-benzyloxyl-piperidin-3-yl ester

starting from (3S,4S,5R)-3-hydroxy-4-{4-[3-(2-methoxy-benzyloxy)-propoxy]-phenyl}-5-[4-methoxy-3-(3-methoxy-propoxy)-benzyloxy]-piperidine-1-carboxylic acid benzyl ester

The starting materials are prepared as follows:
a) (3S,4S,5R)-3-Hydroxy-4-{4-[3-(2-methoxy-benzvloxy)-propoxy]-phenyl}-5-[4-methoxy-3-(3-methoxy-propoxy)-benzyloxy]-piperidine-1-carboxylic acid benzyl ester
   According to general procedure I, (3R,4R,5S)-4-{4-[3-(2-methoxy-benzyloxy)-propoxy]-phenyl}-3-[4-methoxy-3-(3-methoxy-propoxy)-benzyloxy]-5-triisopropylsilanyloxy-piperidine-1-carboxylic acid benzyl ester is used to afford the title compound, which is identified based on its Rf value.
b) (3R,4R,5S)-4-{4-[3-(2-Methoxy-benzyloxy)-propoxy]-phenyl}-3-[4-methoxy-3-(3-methoxy-propoxy)-benzyloxy]-5-triisopropylsilanyloxy-piperidine-1-carboxylic acid benzyl ester
   According to general procedure K, (3R,4R,5S)-3-hydroxy-4-{4-[3-(2-methoxy-benzyloxy)-propoxy]-phenyl}-5-triisopropylsilanyloxy-piperidin-1-carboxylic acid benzyl ester [873945-54-1] and 4-bromomethyl-1-methoxy-2-(3-methoxy-propoxy)-benzene [172900-73-1] are used to afford the title compound, which is identified based on its Rf value.

### 28 (S)-2-Amino-3-methyl-butyric acid (3S,4R,5R)-4-{4-[3-(2-chloro-phenoxy)-propoxy]-phenyl}-5-[4-(3-methoxy-propyl)-3,4-dihydro-2H-benzo[1,4]oxazin-6-ylmethoxy]-piperidin-3-yl ester

starting from (3S,4S,5R)-4-{4-[3-(2-chloro-phenoxy)-propoxy]-phenyl}-3-hydroxy-5-[4-(3-methoxy-propyl)-3,4-dihydro-2H-benzo[1,4]oxazin-6-ylmethoxy]-piperidine-1-carboxylic acid benzyl ester [873945-67-6].

### 37 (S)-2-Amino-3-methyl-butyric acid (3S,4R,5R)-4-{4-[3-(2-methoxy-benzyloxy)-propoxy]-phenyl}-5-[4-(3-methoxy-propyl)-3,4-dihydro-2H-benzo[1,4]oxazin-6-ylmethoxy]-piperidin-3-yl ester

starting from (3S,4S,5R)-3-hydroxy-4-{4-[3-(2-methoxy-benzyloxy)-propoxy]-phenyl}-5-[4-(3-methoxy-propyl)-3,4-dihydro-2H-benzo[1,4]oxazin-6-ylmethoxy]-piperidine-1-carboxylic acid benzyl ester [873945-51-8].

### 46 (S)-2-Amino-3-methyl-butyric acid (3S,4R,5R)-4-[4-(4-ethyl-phenoxy)-phenyl]-5-[4-(3-methoxy-propyl)-3,4-dihydro-2H-benzo[1,4]oxazin-6-ylmethoxy]-piperidin-3-yl ester

starting from (3S,4S,5R)-4-[4-(4-ethyl-phenoxy)-phenyl]-3-hydroxy-5-[4-(3-methoxy-propyl)-3,4-dihydro-2H-benzo[1,4]oxazin-6-ylmethoxy]-piperidine-1-carboxylic acid benzyl ester.

The starting materials are prepared as follows:
a) (3S,4S,5R)-4-[4-(4-Ethyl-phenoxy)-phenyl]-3-hydroxy-5-[4-(3-methoxy-propyl)-3,4-dihydro-2H-benzo[1,4]oxazin-6-ylmethoxy]-piperidine-1-carboxylic acid benzyl ester
   According to general procedure I, (3R,4R,5S)-4-[4-(4-ethyl-phenoxy)-phenyl]-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester is used to afford the title compound as a yellow oil. Rf = 0.19 (EtOAc-heptane 1:1); Rt = 5.73 (gradient I).
b) (3R,4R,5S)-4-[4-(4-Ethyl-phenoxy)-phenyl]-3-[4-(3-methoxy-propyl)-3,4-dihydro-2H-benzo[1,4]oxazin-6-ylmethoxy]-5-triisopropylsilanyloxy- piperidine-1-carboxylic acid benzyl ester
   A solution of 1 mmol of (3R,4R,5S)-4-(4-hydroxy-phenyl)-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester, 3 mmol of 4-ethyl-phenylboronic acid [63139-21-9], 2.2 mmol of copper(II)-acetate, 5 mmol of triethylamine and 700 mg of molecular sieves (4A, powdered) in 10 ml of anhydrous dichloromethane is stirred for 24 hours at room temperature. The mixture is filtered and the filtrate is evaporated. The residue is purified by flash chromatography (SiO₂ 60F) to afford the title compound as a yellow oil. Rf = 0.34 (EtOAc-heptane 1:2); Rt = 5.78 (gradient I).
c) (3R,4R,5S)-4-(4-Hydroxy-phenyl)-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester
   According to general procedure J (the reaction is carried out at 70°C and the mixture is cooled before addition of methanol), (3R,4R,5S)-4-(4-hydroxy-phenyl)-3-[4-(3-methoxy-propyl)-3-oxo-3,4-dihydro-2H-benzo[1,4]oxazin-6-ylmethoxy]-5-triisopropylsilanyloxy-piperidine-1-carboxylic acid benzyl ester is used to afford the title compound as a yellow oil. Rf = 0.10 (EtOAc-heptane 1:2); Rt =29.65 (gradient II).
d) (3R,4R,5S)-4-(4-Hydroxy-phenyl)-3-[4-(3-methoxy-propyl)-3-oxo-3,4-dihydro-2H-benzo[1,4]oxazin-6-ylmethoxy]-5-triisopropylsilanyloxy-piperidine-1-carboxylic acid benzyl ester
   A solution of 5.019 mmol of (3R,4R,5S)-4-(4-allyloxy-phenyl)-3-[4-(3-methoxy-propyl)-3-oxo-3,4-dihydro-2H-benzo[1,4]oxazin-6-ylmethoxy]-5-triisopropylsilanyloxy-piperidine-1-carboxylic acid benzyl ester in 80 ml of methanol under argon atmosphere is treated with 0.376 mmol of tetrakis(triphenylphosphine)palladium(0). After 5 minutes, 5.057 mmol of potassium carbonate are added and the reaction mixture is stirred for 4 hours at room temperature. The solvent is evaporated and water is added to the residue, which is then extracted with tert-butyl methyl ether (2X). The combined organic layers are washed with water (1X) and brine (1X) and dried over sodium sulfate. The solvent is concentrated under reduced pressure and the residue is purified by flash chromatography (SiO₂ 60F) to afford the title compound as yellow oil. Rf = 0.22 (EtOAc-heptane 1:1); Rt = 6.43 (gradient I).
e) (3R,4R,5S)-4-(4-Allyloxy-phenyl)-3-[4-(3-methoxy-propyl)-3-oxo-3,4-dihydro-2H-benzo[1,4]oxazin-6-ylmethoxy]-5-triisopropylsilanyloxy-piperidine-1-carboxylic acid benzyl ester
   According to general procedure K, (3R,4R,5S)-4-(4-allyloxy-phenyl)-3-hydroxy-5-triisopropylsilanyloxy-piperidine-1-carboxylic acid benzyl ester and 6-chloromethyl-4-(3-methoxy-propyl)-4H-benz[1,4]-oxazin-3-one [857272-02-7] are used to afford the title compound as a yellow oil. Rf = 0.24 (EtOAc-heptane 1:2); Rt = 7.17 (gradient I).
f) (3R,4R,5S)-4-(4-Allyloxy-phenyl)-3-hydroxy-5-triisopropylsilanyloxy-piperidine-1-carboxylic acid benzyl ester
   A mixture of 9.875 mmol of (3R,4R,5S)-3-hydroxy-4-(4-hydroxy-phenyl)-5-triisopropylsilanyl-oxy-piperidine-1-carboxylic acid benzyl ester in 63 ml of N,N-dimethylformamide is treated with 12.838 mmol of potasssium carbonate and 12.838 mmol of allylbromide and stirred for 24 hours at 60°C. The mixture is filtered and the filtrate is evaporated. Water is added to the residue, which is then extracted with ethyl acetate (2X). The combined organic layers are washed with brine (1X) and dried over sodium sulfate. The solvent is concentrated under reduced pressure and the residue is purified by flash chromatography (SiO₂ 60F) to afford the title compound as a red brown oil. Rf = 0.28 (EtOAc-heptane 1:2); Rt = 6.61 (gradient I).
g) (3R,4R,5S)-3-Hydroxy-4-(4-hydroxy-phenyl)-5-triisopropylsilanyloxy-piperidine-1-carboxylic acid benzyl ester
   To a solution of 172.323 mmol of (3R,4R,5S)-4-(4-hydroxy-phenyl)-5-triisopropylsilanyloxy-piperidin-3-ol in 1000 ml of ethyl acetate is added 1000 ml of a saturated sodium bicarbonate solution. The biphasic mixture is cooled to 0°C, and slowly treated with 173.185 mmol of benzylchloroformate. The reaction mixture is stirred for 2 hours at room temperature, and then extracted with a mixture of ethyl acetate and tetrahydrofuran. The combined organic layers are evaporated and the title compound is obtained as a white solid from the residue by crystallization (ethyl acetate-heptane). Rf = 0.38 (EtOAc-heptane 1:1); Rt = 5.77 (gradient I).
h) (3R,4R,5S)-4-(4-Hydroxy-phenyl)-5-triisopropylsilanyloxy-piperidin-3-ol
   According to general procedure E, (3R,4R,5S)-4-(4-benzyloxy-phenyl)-1-((R)-1-phenyl-ethyl)-5-triisopropylsilanyloxy-piperidin-3-ol is used to afford the title compound as a colourless solid. Rf = 0.19 (dichloromethane-methanol-ammonia conc. 25% = 200:20:1); Rt = 3.80 (gradient I).
i) (3R,4R,5S)-4-(4-Benzyloxy-phenyl)-1-((R)-1-phenyl-ethyl)-5-triisopropylsilanyloxypiperidin-3-ol
   To a solution of 18.085 mmol of (S)-4-(4-benzyloxy-phenyl)-1-((R)-1-phenyl-ethyl)-3-triisopropylsilanyloxy-1,2,3,4-tetrahydro-pyridine in 140 ml of 1,2-dimethoxyethane at 0°C is dropwise added 72.340 mmol of borane-tetrahydrofuran-complex (1M in tetrahydrofuran). The reaction mixture is stirred for 3 hours at 30°C, then cooled to room temperature and hydrolysed with 70 ml of water. The mixture is stirred for 5 minutes, and then treated with 86.808 mmol of sodium percarbonate. The resulting suspension is stirred for 1 hour at 50°C, then poured on water and extracted with ethyl acetate (2X). The combined organic phases are washed with water, dried over sodium sulfate and the solvent is concentrated under reduced pressure. The residue is purified by flash chromatography (SiO₂ 60F) to afford the title compound as yellow oil. Rf = 0.23 (EtOAc-heptane 1:2); Rt = 5.75 (gradient I).
j) (S)-4-(4-Benzyloxy-phenyl)-1-((R)-1-phenyl-ethyl)-3-triisopropylsilanyloxy-1,2,3,6-tetrahydro-pyridine
   A suspension of 38.131 mmol of 4-(4-benzyloxy-phenyl)-1-(1(R)-phenyl-ethyl)-1,2,3,6-tetrahydro-pyridin-3(S)-ol [257928-45-3] in 250 ml of dichloromethane is treated with 57.197 mmol of 2,6-lutidine and cooled to 0°C. 45.757 mmol of trifluoromethanesulfonic acid triisopropylsilyl ester are added dropwise and the reaction mixture is stirred for 1 hour at 0°C. The reaction mixture is poured on water and the phases are separated. The aqueous phase is extracted with dichloromethane and the combined organic phases are dried over sodium sulfate and the solvent is concentrated under reduced pressure. The residue is purified by flash chromatography (SiO₂ 60F) to afford the title compound as a yellow-brown oil. Rf = 0.66 (EtOAc-heptane 1:2); Rt = 5.83 (gradient I).

### 55 (S)-2-Amino-3-methyl-butyric acid (3S,4R,5R)-5-{2-[2-(2-acetylamino-ethyl)-phenoxyl] ethoxy}-4-{4-[3-(2-methoxy-benzyloxy)-propoxy]-phenyl}-piperidin-3-yl ester

starting from (3R,4S,5S)-3-{2-[2-(2-acetylamino-ethyl)-phenoxy]-ethoxy}-5-hydroxy-4-{4-[3-(2-methoxy-benzyloxy)-propoxy]-phenyl}-piperidine-1-carboxylic acid benzyl ester.

The starting materials are prepared as follows:
a) (3R,4S,5S)-3-{2-[2-(2-Acetylamino-ethyl)-phenoxy]-ethoxy}-5-hydroxy-4-{4-[3-(2-methoxy-benzyloxy)-propoxy]-phenyl}-piperidine-1-carboxylic acid benzyl ester
   According to general procedure I, (3R,4R,5S)-3-{2-[2-(2-acetylamino-ethyl)-phenoxy]-ethoxy}-4-{4-[3-(2-methoxy-benzyloxy)-propoxy]-phenyl}-5-triisopropylsilanyloxy-piperidine-1-carboxylic acid benzyl ester is used to afford the title compound, which is identified based on its Rf value.
b) (3R,4R,5S)-3-{2-[2-(2-Acetylamino-ethyl)-phenoxyl-ethoxyl-4-{4-[3-(2-methoxy-benzyloxy)-propoxy]-phenyl}-5-triisopropylsilanyloxy-piperidine-1-carboxylic acid benzyl ester
   According to general procedure L, (3R,4R,5S)-4-{4-[3-(2-methoxy-benzyloxy)-propoxy]-phenyl}-3-[2-(toluene-4-sulfonyloxy)-ethoxy]-5-triisopropylsilanyloxy-piperidine-1-carboxylic acid benzyl ester and N-[2-(2-hydroxy-phenyl)-ethyl]-acetamide are used to afford the title compound, which is identified based on its Rf value.
c) (3R,4R,5S)-4-{4-[3-(2-Methoxy-benzyloxy)-propoxy]-phenyl}-3-[2-(toluene-4-sulfonyloxy)-ethoxy]-5-triisopropylsilanyloxy-piperidine-1-carboxylic acid benzyl ester
   According to general procedure M, (3R,4R,5S)-3-(2-hydroxy-ethoxy)-4-{4-[3-(2-methoxy-benzyloxy)-propoxy]-phenyl}-5-triisopropylsilanyloxy-piperidine-1-carboxylic acid benzyl ester is used to afford the title compound, which is identified based on its Rf value.
d) (3R,4R,5S)-3-(2-Hydroxy-ethoxy)-4-{4-[3-(2-methoxy-benzyloxy)-propoxy]-phenyl}-5-triisopropylsilanyloxy-piperidine-1-carboxylic acid benzyl ester
   To a stirred mixture of 1 mmol of (3R,4R,5S)-3-allyloxy-4-{4-[3-(2-methoxy-benzyloxy)-propoxy]-phenyl}-5-triisopropylsilanyloxy-piperidine-1-carboxylic acid benzyl ester in a mixture of tetrahydrofuran/water 3:1 (4 ml) is treated with 0.01 mmol of osmiumtetroxide (2.5 wt% in tert-butanol). 2.54 mmol of sodium periodate are added portionwise. The reaction mixture is stirred for 2 hours at room temperature and then evaporated. The residue is suspended in a mixture of dichloromethane/methanol 1:1 (12 ml), cooled to 5°C and treated portionwise with 6.08 mmol of sodium borohydride. The reaction mixture is stirred for 1.5 hours at 5°C and then concentrated under reduced pressure. The residue is stirred with dichloromethane and filtered, the filter cake is washed with dichloromethane. The filtrate is washed with a mixture of brine/water 1:1, filtered, dried over sodium sulfate and concentrated under reduced pressure. The residue is purified by flash chromatography (SiO₂ 60F) to afford the title compound, which is identified based on its Rf value.
e) (3R,4R,5S)-3-Allyloxy-4-{4-[3-(2-methoxy-benzyloxy)-propoxy]-phenyl}-5-triisopropylsilanyloxy-piperidine-1-carboxylic acid benzyl ester
   According to general procedure K, (3R,4R,5S)-3-hydroxy-4-{4-[3-(2-methoxy-benzyloxy)-propoxy]-phenyl}-5-triisopropylsilanyloxy-piperidine-1-carboxylic acid benzyl ester [873945-54-1] and allylbromide are used to afford the title compound, which is identified based on its Rf value.

### 64 (S)-2-Amino-3-methyl-butyric acid (3S,4R,5R)-4-{4-[(S)-1-(3-fluoro-phenyl)-pyrrolidin-3-yloxy]-phenyl}-5-[4-(3-methoxy-propyl)-3,4-dihydro-2H-benzo[1,4]oxazin-6-ylmethoxy]-piperidin-3-yl ester

starting from (3S,4S,5R)-4-{4-[(S)-1-(3-fluoro-phenyl)-pyrrolidin-3-yloxy]-phenyl}-3-hydroxy-5-[4-(3-methoxy-propyl)-3,4-dihydro-2H-benzo[1,4]oxazin-6-ylmethoxy]-piperidine-1-carboxylic acid benzyl ester [873945-20-1].

### 73 (S)-2-Amino-3-methyl-butyric acid (R)-2-{(3S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-3-yloxy}-1-methyl-ethyl ester

starting from (R)-1-{(3S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-3-yloxy}-propan-2-ol.

The starting materials are prepared as follows:
a) (R)-1-{(3S,4R,5R)-4-[4-(2-Methoxy-ethoxymethyl)-phenyl]-5-[4-(3-methoxy-propyl)-3,4-dihydro-2H-benzo[1,4]oxazin-6-ylmethoxy]-piperidin-3-yloxy}-propan-2-ol
   According to general procedure E, (3R,4R,5S)-4-[4-(2-methoxy-ethoxymethyl)-phenyl]-3-[4-(3-methoxy-propyl)-3,4-dihydro-2H-benzo[1,4]oxazin-6-ylmethoxy]-5-((R)-1-oxiranyl-methoxy)-piperidine-1-carboxylic acid benzyl ester is used to afford the title compound as a colourless glass. Rf = 0.23 (dichloromethane-methanol-25% ammonia conc. 200:20:1); Rt = 3.31 (gradient I).
b) (3R,4R,5S)-4-[4-(2-Methoxy-ethoxymethyl)-phenyl]-3-[4-(3-methoxy-propyl)-3,4-dihydro-2H-benzo[1,4]oxazin-6-ylmethoxy]-5-((R)-1-oxiranylmethoxy)-piperidine-1-carboxylic acid benzyl ester
   A solution of 0.493 mmol of (3S,4S,5R)-3-hydroxy-4-[4-(2-methoxy-ethoxymethyl)-phenyl]-5-[4-(3-methoxy-propyl)-3,4-dihydro-2H-benzo[1,4]oxazin-6-ylmethoxy]-piperidine-1-carboxylic acid benzyl ester in 2 ml of tetrahydrofuran is treated with 0.789 mmol of sodium hydride (60% dispersion in oil). The mixture is stirred for 45 minutes at 40°C, then a stirred solution of 0.986 mmol of toluene-4-sulfonic acid (R)-1-oxiranymethyl ester [113826-06-5] in 1.5 ml of tetrahydrofuran is added and the reaction mixture is stirred for 3 hours at 50°C. The reaction mixture is poured on saturated aqueous sodium bicarbonate solution and extracted with tert-butyl methyl ether (3X). The combined organic phases are washed with water, dried over sodium sulfate and the solvent is concentrated under reduced pressure. The residue is purified by flash chromatography (SiO₂ 60F) to afford the title compound as a yellow oil.
   Rf = 0.15 (EtOAc-heptane 2:1); Rt = 5.04 (gradient I).
c) (3S,4S,5R)-3-Hydroxy-4-[4-(2-methoxy-ethoxymethyl)-phenyl]-5-[4-(3-methoxy-propyl)-3,4-dihydro-2H-benzo[1,4]oxazin-6-ylmethoxy]-piperidine-1-carboxylic acid benzyl ester
   According to general procedure 1, (3R,4R,5S)-4-[4-(2-methoxy-ethoxymethyl)-phenyl]-3-[4-(3-methoxy-propyl)-3,4-dihydro-2H-benzo[1,4]oxazin-6-ylmethoxy]-5-triisiopropylsilanyloxypiperidine-1-carboxylic acid benzyl ester is used to afford the title compound as a yellowish oil. Rf = 0.30 (EtOAc-heptane 2:1); Rt = 4.63 (gradient I).
d) (3R,4R,5S)-4-[4-(2-Methoxy-ethoxymethyl)-phenyl]-3-[4-(3-methoxy-propyl)-3,4-dihydro-2H-benzo[1,4]oxazin-6-ylmethoxy]-5-triisiopropylsilanyloxy-piperidine-1-carboxylic acid 2-methoxy-ethyl ester
   and
   (3R,4R,5S)-4-[4-(2-Methoxy-ethoxymethyl)-phenyl]-3-[4-(3-methoxy-propyl)-3,4-dihydro-2H-benzo[1,4]oxazin-6-ylmethoxy]-5-triisiopropylsilanyloxy-piperidine-1-carboxylic acid benzyl ester
   According to general procedure K, (3R,4R,5S)-4-(4-chloromethyl-phenyl)-3-[4-(3-methoxy-propyl)-3,4-dihydro-2H-benzo[1,4]oxazin-6-ylmethoxy]-5-triisiopropylsilanyloxy-piperidine-1-carboxylic acid benzyl ester is used to afford the title compounds.
   (3R,4R,5S)-4-[4-(2-methoxy-ethoxymethyl)-phenyl]-3-[4-(3-methoxy-propyl)-3,4-dihydro-2H-benzo[1,4]oxazin-6-ylmethoxy]-5-triisiopropylsilanyloxy-piperidine-1-carboxylic acid 2-methoxy-ethyl ester:
   Yellowish oil; Rf = 0.26 (EtOAc-heptane 1:1); Rt = 29.90 (gradient II).
   (3R,4R,5S)-4-[4-(2-methoxy-ethoxymethyl)-phenyl]-3-[4-(3-methoxy-propyl)-3,4-dihydro-2H-benzo[1,4]oxazin-6-ylmethoxy]-5-triisiopropylsilanyloxy-piperidine-1-carboxylic acid benzyl ester:
   Yellowish oil; Rf = 0.36 (EtOAc-heptane 1:1); Rt = 31.96 (gradient II).
e) (3R,4R,5S)-4-(4-Chloromethyl-phenyl)-3-[4-(3-methoxy-propyl)-3,4-dihydro-2H-benzo[1,4]oxazin-6-ylmethoxy]-5-triisiopropylsilanyloxy-piperidine-1-carboxylic acid benzyl ester
   A solution of 7.407 mmol of (3R,4R,5S)-4-(4-hydroxymethyl-phenyl)-3-[4-(3-methoxy-propyl)-3,4-dihydro-2H-benzo[1,4]oxazin-6-ylmethoxy]-5-triisiopropylsilanyloxy-piperidine-1-carboxylic acid benzyl ester [873946-12-4] in 100 ml of dichloromethane is cooled to 0°C and treated dropwise with 88.884 mmol of 1-chloro-N,N-2-trimethyl-propenylamine. The reaction mixture is stirred for 16 hours at 20°C, then quenched with tert-butyl methyl ether and water. The phases are separated and the organic phase is washed with brine, dried over sodium sulfate and the solvent is concentrated under reduced pressure. The residue is purified by flash chromatography (SiO₂ 60F) to afford the title compound as a yellow oil. Rf = 0.39 (EtOAc-heptane 1:2).

### 82 (S)-2-Amino-3-methyl-butyric acid (R)-1-methoxymethyl-2-{(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxyl-piperidin-3-yloxy}-ethyl ester

starting from (R)-1-methoxy-3-{(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-yloxy}-propan-2-ol

The starting materials are prepared as follows:
a) (R)-1-Methoxy-3-{(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxyl-piperidin-3-yloxy}-propan-2-ol
   According to general procedure A, (R)-1-methoxy-3-[(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-3-yloxy]-propan-2-ol is used to afford the title compound as a colourless oil. Rf = 0.34 (dichloromethane-methanol-25% ammonia conc. = 200:20:1); Rt = 3.73 (gradient I).
b) (R)-1-Methoxy-3-[(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-3-yloxy]-propan-2-ol
   According to general procedure N, (3S,4S,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-3-ol and S-(+)-glycidyl methyl ether [64491-68-5] are used to afford the title compound as a colourless oil. Rf = 0.25 (EtOAc-heptane 2:1); Rt = 5.26 (gradient I).
c) (3S,4S,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-3-ol
   According to general procedure I, 6-[(3R,4R,5S)-4-[4-((S)-3-methoxy-2-methyl-propoxymethyl)-phenyl]-1-(toluene-4-sulfonyl)-5-triisopropylsilanyloxy-piperidin-3-yloxymethyl]-4-(3-methoxy-propyl)-3,4-dihydro-2H-benzo[1,4]oxazine is used to afford the title compound as a yellowish oil. Rf = 0.24 (EtOAc-heptane 2:1); Rt = 5.13 (gradient I).
d) 6-[(3R,4R,5S)-4-[4-((S)-3-Methoxy-2-methyl-propoxymethyl)-phenyl]-1-(toluene-4-sulfonyl)-5-triisopropylsilanyloxy-piperidin-3-yloxymethyl]-4-(3-methoxy-propyl)-3,4-dihydro-2H-benzo[1,4]oxazine
   A mixture of 2 mmol of 6-[(3R,4R,5S)-4-(4-chloromethyl-phenyl)-1-(toluene-4-sulfonyl)-5-triisopropylsilanyloxy-piperidin-3-yloxymethyl]-4-(3-methoxy-propyl)-3,4-dihydro-2H-benzo[1,4]oxazine and 3 mmol of (R)-3-methoxy-2-methyl-propan-1-ol in 30 ml of N,N-dimethylformamide at 0°C is treated with 4 mmol sodium hydride (60% dispersion in oil). The reaction mixture is stirred at room temperature for 22 hours, then poured on saturated aqueous sodium bicarbonate solution and extracted with tert-butyl-methyl ether (3X). The combined organic phases are washed with brine, dried over sodium sulfate and the solvent is concentrated under reduced pressure. The residue is purified by flash chromatography (SiO₂ 60F) to afford the title compound as a yellow oil. Rf = 0.26 (EtOAc-heptane 1:2).
e) 6-[(3R,4R,5S)-4-(4-Chloromethyl-phenyl)-1-(toluene-4-sulfonyl)-5-triisopropylsilanyloxy-piperidin-3-yloxymethyl]-4-(3-methoxy-propyl)-3,4-dihydro-2H-benzo[1,4]oxazine
   A mixture of 5.12 mmol of {4-[(3R,4R,5S)-3-[4-(3-methoxy-propyl)-3,4-dihydro-2H-benzo[1,4]oxazin-6-ylmethoxy]-1-(toluene-4-sulfonyl)-5-triisopropylsilanyloxy-piperidin-4-yl]-phenyl}-methanol, 8.20 mmol of triethylamine and 0.51 mmol of tetrabutylammonium chloride in 75 ml of dichloromethane at room temperature is treated with 6.15 mmol of methanesulfonyl chloride. After 66 hours, the reaction mixture is diluted with tert-butyl methyl ether, washed with water and brine, dried over sodium sulfate and the solvent is concentrated under reduced pressure. From the residue, the crude title compound is obtained as yellow oil. Rf = 0.26 (EtOAc-heptane 1:2).
f) {4-[(3R,4R,5S)-3-[4-(3-Methoxy-propyl)-3,4-dihydro-2H-benzo[1,4]oxazin-6-ylmethoxy]-1-(toluene-4-sulfonyl)-5-triisopropylsilanyloxy-piperidin-4-yl]-phenyl}-methanol
   A mixture of 5.44 mmol of (4-{(3R,4R,5S)-3-[4-(3-methoxy-propyl)-3,4-dihydro-2H-benzo[1,4]oxazin-6-ylmethoxy]-5-triisopropylsilanyloxy-piperidin-4-yl}-phenyl)-methanol in 100 ml of ethyl acetate and 100 ml of 2N sodium carbonate solution at room temperature is treated with 5.99 mmol of toluenesulfonyl chloride. After 14 hours, the phases are separated and the aqueous phase is extracted with ethyl acetate (3X). The combined organic phases are washed with brine, dried over sodium sulfate and the solvent is concentrated under reduced pressure. From the residue, the title compound is obtained as orange oil. Rf = 0.30 (EtOAc-heptane 1:1); Rt = 29.47 (gradient II).
g) (4-{(3R,4R,5S)-3-[4-(3-Methoxy-propyl)-3,4-dihydro-2H-benzo[1,4]oxazin-6-ylmethoxy]-5-triisopropylsilanyloxy-piperidin-4-yl}-phenyl)-methanol
   According to general procedure E (using tetrahydrofuran as solvent), (3R,4R,5S)-4-(4-hydroxymethyl-phenyl)-3-[4-(3-methoxy-propyl)-3,4-dihydro-2H-benzo[1,4]oxazin-6-ylmethoxy]-5-triisiopropylsilanyloxy-piperidine-1-carboxylic acid benzyl ester [873946-12-4] is used to afford the title compound as an orange oil. Rt = 4.66 (gradient I).
h) (R)-3-Methoxy-2-methyl-propan-1-ol
   According to general procedure I, triisopropyl-(3-methoxy-2(S)-methyl-propoxy)-silane is used to afford the title compound as a yellow liquid. Rf = 0.15 (EtOAc-heptane 1:4).
i) Triisopropyl-((S)-3-methoxy-2-methyl-propoxy)-silane
   A solution of 10.060 mmol of (S)-2-methyl-3-triisopropylsilanyloxy-propan-1-ol [256643-28-4] and 50.300 mmol of methyl iodide in 50 ml of tetrahydrofuran at 0°C is treated with 50.300 mmol of sodium hydride (60% dispersion in oil). The reaction mixture is stirred at room temperature for 60 hours, and then diluted with tert-butyl methyl ether, washed with water and brine, dried over sodium sulfate and the solvent is concentrated under reduced pressure. The residue is purified by flash chromatography (SiO₂ 60F) to afford the title compound as a yellow oil. Rf = 0.41 (EtOAc-heptane 1:10).

### 91 (S)-2-Amino-3-methyl-butyric acid (R)-2-{(3S,4R,5R)-4-[4-(4-methoxy-butoxy)-phenyl]-5-[4-(3-methoxy-propyl)-3,4-dihydro-2H-benzo[1,4]oxazin-6-ylmethoxy]-piperidin-3-yloxy}-1-methyl-ethyl ester

starting from (R)-1-{(3S,4R,5R)-4-[4-(4-methoxy-butoxy)-phenyl]-5-[4-(3-methoxy-propyl)-3,4-dihydro-2H-benzo[1,4]oxazin-6-ylmethoxy]-piperidin-3-yloxy}-propan-2-ol.

The starting materials are prepared as follows:
a) (R)-1-{(3S,4R,5R)-4-[4-(4-Methoxy-butoxy)-phenyl]-5-[4-(3-methoxy-propyl)-3,4-dihydro-2H-benzo[1,4]oxazin-6-ylmethoxy]-piperidin-3-yloxy}-propan-2-ol
   According to general procedure E, (3R,4R,5S)-4-[4-(4-methoxy-butoxy)-phenyl]-3-[4-(3-methoxy-propyl)-3,4-dihydro-2H-benzo[1,4]oxazin-6-ylmethoxy]-5-((R)-1-oxiranylmethoxy)-piperidine-1-carboxylic acid benzyl ester is used to afford the title compound as a colourless oil. Rf = 0.27 (dichloromethane-methanol-25% ammonia conc. 200:20:1); Rt = 3.65 (gradient I).
b) (3R,4R,5S)-4-[4-(4-Methoxy-butoxy)-phenyl]-3-[4-(3-methoxy-propyl)-3,4-dihydro-2H-benzo[1,4]oxazin-6-ylmethoxy]-5-((R)-1-oxiranylmethoxy)-piperidine-1-carboxylic acid benzyl ester
   According to the procedure described in example 73b, (3S,4S,5R)-3-hydroxy-4-[4-(4-methoxy-butoxy)-phenyl]-5-[4-(3-methoxy-propyl)-3,4-dihydro-2H-benzo[1,4]oxazin-6-ylmethoxy]-piperidine-1-carboxylic acid benzyl ester is used to afford the title compound as a yellowish oil. Rf = 0.30 (EtOAc-heptane 3:1); Rt = 5.36 (gradient I).
c) (3S,4S,5R)-3-Hydroxy-4-[4-(4-methoxy-butoxy)-phenyl]-5-[4-(3-methoxy-propyl)-3,4-dihydro-2H-benzo[1,4]oxazin-6-ylmethoxy]-piperidine-1-carboxylic acid benzyl ester
   A solution of 1.379 mmol of (3R,4R,5S)-4-[4-(4-methoxy-butoxy)-phenyl]-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester in 10 ml of tetrahydrofuran at room temperature is treated with 2.758 mmol of tetrabutylammonium fluoride trihydrate. After 1 hour, the reaction mixture is quenched with 0.5 ml of water and concentrated under reduced pressure. The residue is purified by flash chromatography (SiO₂ 60F) to afford the title compound as a turbid oil. Rf = 0.60 (EtOAc); Rt = 4.94 (gradient I).
d) (3R,4R,5S)-4-[4-(4-Methoxy-butoxy)-phenyl]-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester
   According to general procedure J, (3R,4R,5S)-4-[4-(4-methoxy-butoxy)-phenyl]-3-[4-(3-methoxy-propyl)-3-oxo-3,4-dihydro-2H-benzo[1,4]oxazin-6-ylmethoxy]-5-triisopropylsilanyl-oxy-piperidine-1-carboxylic acid benzyl ester is used to afford the title compound as a colourless oil. Rf = 0.19 (EtOAc-heptane 1:1).
e) (3R,4R,5S)-4-[4-(4-Methoxy-butoxy)-phenyl]-3-[4-(3-methoxy-propyl)-3-oxo-3,4-dihydro-2H-benzo[1,4]oxazin-6-ylmethoxy]-5-triisopropylsilanyloxy-piperidine-1-carboxylic acid benzyl ester
   According to general procedure K, (3R,4R,5S)-3-hydroxy-4-[4-(4-methoxy-butoxy)-phenyl]-5-triisopropylsilanyloxy-piperidine-1-carboxylic acid benzyl ester and 6-chloromethyl-4-(3-methoxy-propyl)-4H-benz[1,4]-oxazin-3-one [857272-02-7] are used to afford the title compound as a yellowish oil. Rt = 7.24 (gradient I).
f) (3R,4R,5S)-3-Hydroxy-4-[4-(4-methoxy-butoxy)-phenyl]-5-triisopropylsilanyloxy-piperidine-1-carboxylic acid benzyl ester
   According to general procedure K, (3R,4R,5S)-3-hydroxy-4-[4-hydroxy-phenyl]-5-triiso-propylsilanyloxy-piperidine-1-carboxylic acid benzyl ester [873945-27-8] and 1-bromo-4-methoxy-butane [4457-67-4] are used to afford the title compound as a yellow oil. Rt = 6.63 (gradient I).

### 100 (S)-2-Amino-3-methyl-butyric acid (3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-ylmethyl ester

starting from (3S,4R,5R)-3-hydroxymethyl-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidine-1-carboxylic acid benzyl ester

The starting materials are prepared as follows:
a) (3S,4R,5R)-3-Hydroxymethyl-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidine-1-carboxylic acid benzyl ester
   According to general procedure J, (3S,4R,5R)-3-hydroxymethyl-4-[4-((S)-3-methoxy-2-methyl-propoxymethyl)-phenyl]-5-[4-(3-methoxy-propyl)-3-oxo-3,4-dihydro-2H-benzo[1,4]oxazin-6-ylmethoxy]-piperidine-1-carboxylic acid benzyl ester is used to afford the title compound as a yellow oil. Rf = 0.40 (EtOAc-heptane = 4:1); Rt = 5.21 (gradient I).
b) (3S,4R,5R)-3-Hydroxymethyl-4-[4-((S)-3-methoxy-2-methyl-propoxymethyl)-phenyl]-5-[4-(3-methoxy-propyl)-3-oxo-3,4-dihydro-2H-benzo[1,4]oxazin-6-ylmethoxy]-piperidine-1-carboxylic acid benzyl ester
   A solution of 1.919 mmol (3R,4R,5S)-4-[4-((S)-3-methoxy-2-methyl-propoxymethyl)-phenyl]-3-[4-(3-methoxy-propyl)-3-oxo-3,4-dihydro-2H-benzo[1,4]oxazin-6-ylmethoxy]-5-trityloxy-methyl-piperidine-1-carboxylic acid benzyl ester in 20 ml of methanol and 4 ml of tetrahydrofuran is treated with 7.960 mmol of p-toluenesulfonic acid monohydrate stirred for 1.5 hours at room temperature. The reaction mixture is poured onto saturated aqueous sodium bicarbonate solution and extracted with dichloromethane (3X). The combined organic layers are dried over sodium sulfate. The solvent is concentrated under reduced pressure to afford the crude title compound as yellow oil. Rt = 4.81 (gradient I).
c) (3R,4R,5S)-4-[4-((S)-3-Methoxy-2-methyl-propoxymethyl)-phenyl]-3-[4-(3-methoxy-propyl)-3-oxo-3,4-dihydro-2H-benzo[1,4]oxazin-6-ylmethoxy]-5-trityloxymethyl-piperidine-1-carboxylic acid benzyl ester
   According to general procedure K (carried out at 0°C, with addition of 0.1 equivalents of tetrabutylammonium iodide), (3R,4R,5S)-3-hydroxy-4-[4-((S)-3-methoxy-2-methyl-propoxymethyl)-phenyl]-5-trityloxymethyl-piperidine-1-carboxylic acid benzyl ester and 6-chloromethyl-4-(3-methoxy-propyl)-4H-benz[1,4]-oxazin-3-one [857272-02-7] are used to afford the title compound as a colourless oil. Rf = 0.19 (EtOAc-heptane = 1:2); Rt = 6.57 (gradient I).
d) (3R,4R,5S)-3-Hydroxy-4-[4-((S)-3-methoxy-2-methyl-propoxymethyl)-phenyl]-5-trityloxymethyl-piperidine-1-carboxylic acid benzyl ester
   According to general procedure H, (3R,4R,5S)-3-hydroxy-4-[4-((S)-3-methoxy-2-methyl-propoxymethyl)-phenyl]-5-trityloxymethyl-piperidinium (S)-carboxy-phenyl-methanolate is used to afford the title compound as a white foam. Rt = 6.14 (gradient I).
e) (3R,4R,5S)-3-Hydroxy-4-[4-((S)-3-methoxy-2-methyl-propoxymethyl)-phenyl]-5-trityloxymethyl-piperidinium (S)-carboxy-phenyl-methanolate
   According to general procedure E (using methanol as solvent), (3R,4R,5S)-1-benzyl-3-hydroxy-4-[4-((S)-3-methoxy-2-methyl-propoxymethyl)-phenyl]-5-trityloxymethyl-piperidinium (S)-carboxy-phenyl-methanolate is used to afford the title compound as a white foam. Rt = 4.90 (gradient I).
f) (3R,4R,5S)-1-Benzyl-3-hydroxy-4-[4-((S)-3-methoxy-2-methyl-propoxymethyl)-phenyl]-5-trityloxymethyl-piperidinium (S)-carboxy-phenyl-methanolate
   A solution of 23.84 mmol of 1-benzyl-4-[4-((S)-3-methoxy-2-methyl-propoxymethyl)-phenyl]-5-trityloxymethyl-piperidin-3-ol (diastereomeric mixture) in 171 ml of tetrahydrofuran at 65°C is treated with 11.92 mmol of L-(+)-mandelic acid. After 1 minute, 158 ml of hexane are added. The solution is slowly cooled to room temperature, 23 ml of hexane is added and the mixture is stirred for 15 hours at room temperature. The white suspension is cooled to 10°C and filtered. The white powder is washed with 40 ml of tetrahydrofuran-hexane 1:3 and dried. The crystals possess an isomeric purity of about 80% (by chiral HPLC). In order to enhance the isomeric purity, the above described procedure is repeated using 0.75 equivalents of L-(+)-mandelic acid.
g) 1-Benzyl-4-[4-((S)-3-methoxy-2-methyl-propoxymethyl)-phenyl]-5-trityloxymethyl-piperidin-3-ol (diastereomeric mixture)
   To a solution of 44.3 mmol of 1-benzyl-4-[4-((S)-3-methoxy-2-methyl-propoxymethyl)-phenyl]-3-trityloxymethyl-1,2,3,6-tetrahydro-pyridine (diastereomeric mixture) in 220 ml of tetrahydrofuran at 0°C are added drop wise 88.6 mmol of borane-tetrahydrofuran complex (1M in tetrahydrofuran). The reaction mixture is stirred for 18 hours at room temperature and then cooled to 10°C. The mixture is treated with a solution of 256.8 mmol of KOH in 20 ml of water and then hydrolyzed with a solution of hydrogen peroxide (30% in water). The reaction mixture is stirred for 4 hours at 60°C, then cooled to room temperature and diluted with 300 ml of tert-butyl methyl ether. The mixture is washed with 150 ml of water and 200 ml of brinewater 1:1. The combined aqueous phases are extracted with 100 ml of tert-butyl methyl ether. The combined organic phases are dried over sodium sulfate and concentrated under reduced pressure. The residue is purified by flash chromatography (SiO₂ 60F) to afford the title compound as a yellowish foam. Rf = 0.09 (EtOAc-heptane-triethylamine 100:100:1);
   Rt = 4.89 (gradient I).
h) 1-Benzyl-4-[4-((S)-3-methoxy-2-methyl-propoxymethyl)-phenyl]-3-trityloxymethyl-1,2,3,6-tetrahydro-pyridine (diastereomeric mixture)
   A solution of 85.53 mmol 1-benzyl-4-[4-((S)-3-methoxy-2-methyl-propoxymethyl)-phenyl]-3-trityloxymethyl-piperidin-4-ol (diastereomeric mixture) in 250 ml of pyridine at 0°C is treated drop wise with 102.64 mmol of thionylchloride. The brown reaction mixture is stirred for 5 minutes at 0°C, quenched with 50 ml of 4N NaOH and concentrated under reduced pressure. The residue is suspended in 750 ml of ethyl acetate and washed sequentially with 500 ml of saturated aqueous sodium bicarbonate solution, 500 ml of water und 100 ml of brine. The combined aqueous phases are extracted with 250 ml ethyl acetate. The combined organic phases are dried over sodium sulfate and concentrated under reduced pressure. The residue is purified by flash chromatography (SiO₂ 60F) to afford the title compound as a yellow oil.
   Rf = 0.28 (EtOAc-heptane-triethylamine 100:200:1); Rt = 5.64 (gradient I).
i) 1-Benzyl-4-[4-((S)-3-methoxy-2-methyl-propoxymethyl)-phenyl]-3-trityloxymethyl-piperidin-4-ol (diastereomeric mixture)
   A suspension of 151.5 mmol of magnesium turnings in 20 ml of tetrahydrofuran under Aratmosphere is treated with 0.125 mmol of 1,2-dibromoethane and heated to 60°C till gas evolution is detected. A solution of 151.5 mmol of 1-bromo-4-((S)-3-methoxy-2-methyl-propoxymethyl)-benzene in 170 ml of tetrahydrofuran is added drop wise. The orange reaction mixture is stirred for 1 hour at reflux, cooled to room temperature and treated drop wise with a solution of 125.2 mmol of 1-benzyl-3-trityloxymethyl-piperidin-4-one [234757-27-8] in 170 ml of tetrahydrofuran. The temperature of the reaction mixture is kept below 40° C. The reaction mixture is then stirred for 16 hours at room temperature and quenched by addition of 300 ml of saturated aqueous ammonium chloride solution and 300 ml of ethyl acetate. The organic phase is separated and washed with 2X100 ml of water and 100 ml of brine. The combined aqueous phases are extracted with 100 ml ethyl acetate and the combined organic phases are dried over sodium sulfate and concentrated under reduced pressure. The residue is purified by flash chromatography (SiO₂ 60F) to afford the title compound as a yellowish oil. Rf = 0.30 (EtOAc-heptane-triethylamine 100:100:1); Rt = 5.43 (gradient I).
j) 1-Bromo-4-((S)-3-methoxy-2-methyl-propoxymethyl)-benzene
   A suspension of 360.13 mmol of sodium hydride (60% dispersion in oil) in 100 ml of N,N-dimethylformamide under Ar atmosphere is cooled to 0°C and treated dropwise with a solution of 261.09 mmol (R)-3-methoxy-2-methyl-propan-1-ol (example 82h) in 50 ml of N,N-dimethylformamide. The reaction mixture is stirred for 20 minutes at 0°C-10°C, and then treated with 180.0 mmol of 1-bromo-4-chloromethyl-benzene [158328-45-1] over 30 minutes. The reaction mixture is stirred for 75 minutes at 0°C-10°C, diluted with 400 ml tert-butyl methyl ether and poured onto 400 ml of saturated aqueous sodium bicarbonate solution. The organic phase is separated, the aqueous phase is extracted with tert-butyl methyl ether (3 X 500 ml). The combined organic phases are washed with 350 ml of water and 350 ml of brine, dried over sodium sulfate and the solvent is concentrated under reduced pressure. The residue is purified by flash chromatography (SiO₂ 60F) to afford the title compound as a yellow oil. Rf = 0.44 (EtOAc-heptane 1:6); Rt = 5.29 (gradient I).

### 109 (S)-2-Amino-3-methyl-butyric acid (R,S)-1-methoxymethyl-2-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-1-methyl-ethyl ester (diastereomeric mixture)

starting from (R,S)-1-Methoxy-3-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2-methyl-propan-2-ol (diastereomeric mixture)

The starting materials are prepared as follows:
a) (R,S)-1-Methoxy-3-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2-methyl-propan-2-ol (diastereomeric mixture)
   According to general procedure A, (R,S)-1-methoxy-3-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2-methyl-propan-2-ol (diastereomeric mixture) is used to afford the title compound.
b) (R,S)-1-Methoxy-3-[(2R,4R,5R)-4-(4-methoxy-phenyl)-5-[4-(3-methoxy-propyl)-3,4-dihydro-2H-benzo[1,4]oxazin-6-ylmethoxyl-1-(toluene-4-sulfonyl)-piperidin-2-yl]-2-methyl-propan-2-ol (diastereomeric mixture)
   To a stirred solution of 1.0 g of (R,S)-1-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-propan-2-one in 15 ml of methanol are added 360 mg of trimethylsulfoxonium iodide and 273 mg of sodium hydroxide and the reaction mixture is heated to 70°C for 1 day. The reaction mixture is concentrated under reduced pressure and the residue is taken up in water and dichloromethane. The organic phase is dried and concentrated under reduced pressure. The residue is purified by flash chromatography (SiO₂ 60F) to afford the title compound as a yellow oil. Rf = 0.39 (EtOAc-heptane 1:1); Rt = 5.09 (gradient I).
c) 1-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-propan-2-one
   To a stirred solution of 0.79 g of N-methoxy-2-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-N-methyl-acetamide in 10 ml of tetrahydrofuran are added 1.3 ml of a 3M solution of methyl magnesium bromide in tetrahydrofuran at 0°C. The reaction mixture is stirred for 1 hour, diluted with an aqueous solution of 1 N potassium hydrogen sulfate, extracted with tert-butyl methyl ether. The organic phases are combined, dried over sodium sulfate and concentrated under reduced pressure. The residue is purified by flash chromatography (SiO₂ 60F) to afford the title compound as a yellow oil. Rf = 0.20 (EtOAc-heptane 1:1); Rt = 5.14 (gradient I).
d) N-Methoxy-2-[(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-N-methyl-acetamide
   According to general procedure D, [(2R,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-acetic acid and N,O-dimethylhydroxylamine hydrochloride are used to afford the title compound as a colourless foam. Rf = 0.44 (EtOAc-heptane 5:1); Rt = 5.00 (gradient I).
e) [(2R,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-acetic acid
   A stirred solution of 0.5 g of [(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-acetonitrile in 15 ml of ethanol and 15 ml of 2N sodium hydroxide is heated to 85°C overnight. The reaction mixture is allowed to cool to room temperature, acidified by addition of 1 N hydrochloric acid solution and extracted with ethyl acetate. The organic phase is dried over sodium sulfate and concentrated under reduced pressure. The residue is purified by flash chromatography (SiO₂ 60F) to afford the title compound as a green foam. Rf = 0.33 (EtOAc-heptane 5:1); Rt = 4.76 (gradient I).
f) [(2S,4R,5R)-4-(4-Methoxy-phenyl)-5-[4-(3-methoxy-propyl)-3,4-dihydro-2H-benzo[1,4]oxazin-6-ylmethoxy]-1-(toluol-4-sulfonyl)-piperidin-2-yl]-acetonitril
   To a stirred solution of 0.5 g of methanesulfonic acid (2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-ylmethyl ester in 10 ml of dimethylsulfoxide are added 0.25 g of sodium cyanide and the mixture is heated to 50°C for 4 hours. The reaction mixture is diluted with water, extracted with ethyl acetate, dried over sodium sulfate and concentrated under reduced pressure. The residue is purified by flash chromatography (SiO₂ 60F) to afford the title compound. Rf = 0.26 (EtOAc-heptane 1:1); Rt = 5.20 (gradient I).
g) Methanesulfonic acid (2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-ylmethyl ester
   According to general procedure F, [(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-methanol (from example 24b) is used to afford the title compound. Rf = 0.45 (EtOAc-heptane 1:1); Rt = 5.15 (gradient I).
h) [(2S,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-methanol
   To a stirred solution of 0.45 g of (2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidine-2-carboxylic acid ethyl ester in 35 ml of tetrahydrofuran are added 58.0 mg of lithium aluminium hydride at 0°C. The reaction mixture is stirred for 1 hour at this temperature, diluted with tert-butyl methyl ether and quenched by addition of 7 ml of aqueous saturated sodium sulfate solution. The mixture is allowed to warm to room temperature, filtered and dried over sodium sulfate. The solvent is concentrated under reduced pressure and the residue is purified by flash chromatography (SiO₂ 60F) to afford the title compound as a yellow oil. Rf = 0.3 (EtOAc-heptane 2:1); Rt = 4.82 (gradient I).
i) (2S,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidine-2-carboxylic acid ethyl ester
   To a solution of 1.51 g of (2S,4R,5R)-4-(4-methoxy-phenyl)-5-[4-(3-methoxy-propyl)-3,4-dihydro-2H-benzo[1,4]oxazin-6-ylmethoxy]-piperidine-2-carboxylic acid ethyl ester in 25 ml of dichloromethane are added 1.97 ml of triethylamine, 3.5 mg of 4-dimethylaminopyridine and 0.57 g of 4-toluenesulfonylchloride at 0°C. The reaction mixture is allowed to warm to room temperature overnight, diluted with water and 1 N hydrochloric acid and the organic phase is separated and dried over sodium sulfate. The organic phase is concentrated under reduced pressure and the residue is purified by flash chromatography (SiO₂ 60F) to afford the title compound as a colourless oil. Rf = 0.50 (EtOAc-heptane 1:1); Rt = 5.44.
j) (2S,4R,5R)-4-(4-Methoxy-phenyl)-5-[4-(3-methoxy-propyl)-3,4-dihydro-2H-benzo[1,4]oxazin-6-ylmethoxy]-piperidine-2-carboxylic acid ethyl ester
   According to general procedure C, (2S,4R,5R)-4-(4-methoxy-phenyl)-5-[4-(3-methoxy-propyl)-3-oxo-3,4-dihydro-2H-benzo[1,4]oxazin-6-ylmethoxy]-piperidine-2-carboxylic acid ethyl ester is used to afford the title compound as a yellow oil. Rf = 0.55 (dichlormethane-methanol-25% ammonia conc. 200:20:1); Rt = 3.81 (gradient I).
k) (2S,4R,5R)-4-(4-Methoxy-phenyl)-5-[4-(3-methoxy-propyl)-3-oxo-3,4-dihydro-2H-benzo[1,4]oxazin-6-ylmethoxy]-piperidine-2-carboxylic acid ethyl ester
   According to general procedure B, (2S,4R,5R)-1,4-bis-(4-methoxy-phenyl)-5-[4-(3-methoxy-propyl)-3-oxo-3,4-dihydro-2H-benzo[1,4]oxazin-6-ylmethoxy]-piperidine-2-carboxylic acid ethyl ester is used to afford the title compound as a yellow oil. Rf = 0.10 (dichlormethane-methanol-25% ammonia conc. 200:10:1); Rt = 3.53 (gradient I).
l) (2S,4R,5R)-1,4-Bis-(4-methoxy-phenyl)-5-[4-(3-methoxy-propyl)-3-oxo-3,4-dihydro-2H-benzo[1,4]oxazin-6-ylmethoxy]-piperidine-2-carboxylic acid ethyl ester
   To a stirred solution of 5.20 g of (2S,4R,5R)-5-hydroxy-1,4- bis(4-methoxy-phenyl)piperidine-2-carboxylic acid ethyl ester and 5.62 g of 2,2,2-trichloro-acetimidic acid 4-(3-methoxy-propyl)-3-oxo-3,4-dihydro-2H-benzo[1,4]oxazin-6-ylmethyl ester in 150 ml of dichloromethane is added 1.1 ml of trifluoromethanesulfonic acid at -30°C. The reaction mixture is allowed to stir for another hour at this temperature and then allowed to warm to 0°C within 2 hours. The mixture is quenched by addition of 2N sodium hydroxide, extracted with dichloromethane, dried over sodium sulfate and concentrated under reduced pressure. The residue is purified by flash chromatography (SiO₂ 60F) to afford the title compound as a brown oil. Rf = 0.24 (EtOAc-heptane 1:1); Rt = 4.73 (gradient I).
m) (2S,4R,5R)-5-Hydroxy-1,4-bis(4-methoxy-phenyl)piperidine-2-carboxylic acid ethyl ester
   To a stirred solution of 19.5 g of (S)-1,4-bis-(4-methoxy-phenyl)-1,2,3,6-tetrahydro-pyridine-2-carboxylic acid ethyl ester in 400 ml of tetrahydrofuran are added 56 ml of a solution of borane-tetrahydrofuran (1 M) at room temperature. The reaction mixture is heated to 40°C for 2-6 hours, cooled to room temperature, and quenched by the addition of 50 ml of 2N sodium hydroxide solution and 100 ml of 30% hydrogen peroxide solution. The organic phase is separated, dried over sodium sulfate and concentrated under reduced pressure. The residue is purified by flash chromatography (SiO₂ 60F) to afford the title compound as a yellow solid. Rf = 0.33 (EtOAc-heptane 1:1); Rt = 3.58 (gradient I).
n) (S)-1,4-Bis-(4-methoxy-phenyl)-1,2,3,6-tetrahydro-pyridine-2-carboxylic acid ethyl ester
   To a suspension of 38.0 g of (S)-1-(4-methoxy-phenyl)-4-trifluoromethanesulfonyloxy-1,2,3,6-tetrahydropyridine-2-carboxylic acid ethyl ester, 18.1 g of 4-methoxyphenylboronic acid, 6.45 g of lithium chloride in 300 ml of dimethoxyethane are added 100 ml of ethanol, 160 ml of 2N sodium carbonate solution and 2.22 g of tetrakis(triphenylphosphine)palladium. The reaction mixture is heated to 75°C for 3-6 hours and allowed to cool to room temperature overnight. The reaction mixture is filtered and the filter cake is re-dissolved in dichloromethane and water. The suspension is filtered again and the organic phase is concentrated under reduced pressure to afford the title compound as a colourless solid. Rf = 0.25 (EtOAc-heptane 1:4);
   Rt = 5.00 (gradient I).
o) (S)-1-(4-Methoxy-phenyl)-4-trifluoromethanesulfonyloxy-1,2,3,6-tetrahydropyridine-2-carboxylic acid ethyl ester
   To a stirred solution of 0.94 g (S)-1-(4-methoxy-phenyl)-4-oxo-1,2,3,4-tetrydro-pyridine-2-carboxylic acid ethyl ester [690224-49-8] in 20 ml tetrahydrofuran is added a solution of 3.7 ml of L-selectride (0.5M, Aldrich 17,849-7) at -78°C. The mixture is stirred for 1 hour and a solution of 1.18 g N-phenyltrifluoromethanesulfonimide (Fluka 78175) in 8 ml of tetrahydrofuran is added and the mixture is allowed to warm to room temperature overnight. The reaction mixture is concentrated under reduced pressure and the residue is purified by flash chromatography (aluminium oxide, Fluka 06290) to afford the title compound as a brown solid. Rf = 0.2 (EtOAc-heptane 1:9); Rt = 5.14 (gradient I).
p) 2,2,2-Trichloro-acetimidic acid 4-(3-methoxy-propyl)-3-oxo-3,4-dihydro-2H-benzo[1,4]oxazin-6-ylmethyl ester
   To a stirred solution of 8.50 g of 6-hydroxymethyl-4-(3-methoxy-propyl)-4H-benzo[1,4]oxazin-3-one [857272-03-8] in 200 ml of diethyl ether is added 1.28 g of sodium hydride at 0°C. The mixture is stirred for 1 hour and concentrated to dryness under reduced pressure to afford a brown oil, which is not further purified and directly used in the next step. Rf = 0.5 (EtOAc-heptane 1:1).

### Example 2

### (S)-2-(2-Amino-acetylamino)-3-methyl-butyric acid (3S,4R,5R)-4-(4-methoxy-phenyl)-5-[4-(3-methoxy-propyl)-3,4-dihydro-2H-benzo[1,4]oxazin-6-ylmethoxy]-piperidin-3-yl ester

According to the process described in example 1, 1 a and 1 b, using (S)-2-(2-benzyloxycarbonylamino-acetylamino)-3-methyl-butyric acid [33912-87-7] in step a, the title compound is obtained starting from (3S,4S,5R)-4-(4-methoxy-phenyl)-5-[4-(3-methoxy-propyl)-3,4-dihydro-2H-benzo[1,4]oxazin-6-ylmethoxy]-piperidin-3-ol [873945-55-2], and is identified based on its Rf value.

According to the procedure described in example 2, the following compounds are prepared in analogous manner:
11 (S)-2-(2-Amino-acetylamino)-3-methyl-butyric acid (3S,4R,5R)-4-{4-[3-(2-methoxy-benzyloxy)-propoxy]-phenyl}-5-[1-(3-methoxy-propyl)-3,3-dimethyl-2,3-dihydro-1H-indol-6-ylmethoxyl-piperidin-3-yl ester
   starting from (3S,4S,5R)-3-hydroxy-4-{4-[3-(2-methoxy-benzyloxy)-propoxy]-phenyl}-5-[1-(3-methoxy-propyl)-3,3-dimethyl-2,3-dihydro-1H-indol-6-ylmethoxy]-piperidine-1-carboxylic acid benzyl ester (example 10a).
20 (S)-2-(2-Amino-acetylamino)-3-methyl-butyric acid (3S,4R,5R)-4-{4-[3-(2-methoxy-benzyloxy)-propoxy]-phenyl}-5-[4-methoxy-3-(3-methoxy-propoxy)-benzyloxy]-piperidin-3-yl ester
   starting from (3S,4S,5R)-3-hydroxy-4-{4-[3-(2-methoxy-benzyloxy)-propoxy]-phenyl}-5-[4-methoxy-3-(3-methoxy-propoxy)-benzyloxy]-piperidine-1-carboxylic acid benzyl ester (example 19a).
29 (S)-2-(2-Amino-acetylamino)-3-methyl-butyric acid (3S,4R,5R)-4-{4-[3-(2-chloro-phenoxy)-propoxy]-phenyl}-5-[4-(3-methoxy-propyl)-3,4-dihydro-2H-benzo[1,4]oxazin-6-ylmethoxy]-piperidin-3-yl ester
   starting from (3S,4S,5R)-4-{4-[3-(2-chloro-phenoxy)-propoxy]-phenyl}-3-hydroxy-5-[4-(3-methoxy-propyl)-3,4-dihydro-2H-benzo[1,4]oxazin-6-ylmethoxy]-piperidine-1-carboxylic acid benzyl ester [873945-67-6].
38 (S)-2-(2-Amino-acetylamino)-3-methyl-butyric acid (3S,4R,5R)-4-{4-[3-(2-methoxy-benzyloxy)-propoxy]-phenyl}-5-[4-(3-methoxy-propyl)-3,4-dihydro-2H-benzo[1,4]oxazin-6-ylmethoxy]-piperidin-3-yl ester
   starting from (3S,4S,5R)-3-hydroxy-4-{4-[3-(2-methoxy-benzyloxy)-propoxy]-phenyl}-5-[4-(3-methoxy-propyl)-3,4-dihydro-2H-benzo[1,4]oxazin-6-ylmethoxy]-piperidine-1-carboxylic acid benzyl ester [873945-51-8].
47 (S)-2-(2-Amino-acetylamino)-3-methyl-butyric acid (3S,4R,5R)-4-[4-(4-ethyl-phenoxy)-phenyl]-5-[4-(3-methoxy-propyl)-3,4-dihydro-2H-benzo[1,4]oxazin-6-ylmethoxyl-piperidin-3-yl ester
   starting from (3S,4S,5R)-4-[4-(4-ethyl-phenoxy)-phenyl]-3-hydroxy-5-[4-(3-methoxy-propyl)-3,4-dihydro-2H-benzo[1,4]oxazin-6-ylmethoxy]-piperidine-1-carboxylic acid benzyl ester (example 46a).
56 (S)-2-(2-Amino-acetylamino)-3-methyl-butyric acid (3S,4R,5R)-5-{2-[2-(2-acetylaminoethyl)-phenoxy]-ethoxy}-4-{4-[3-(2-methoxy-benzyloxy)-propoxy]-phenyl}-piperidin-3-yl ester
   starting from (3R,4S,5S)-3-{2-[2-(2-acetylamino-ethyl)-phenoxy]-ethoxy}-5-hydroxy-4-{4-[3-(2-methoxy-benzyloxy)-propoxy]-phenyl}-piperidine-1-carboxylic acid benzyl ester (example 55a).
65 (S)-2-(2-Amino-acetylamino)-3-methyl-butyric acid (3S,4R,5R)-4-{4-[(S)-1-(3-fluorophenyl)-pyrrolidin-3-yloxyl-phenyl}-5-[4-(3-methoxy-propyl)-3,4-dihydro-2H-benzo[1,4]oxazin-6-ylmethoxy]-piperidin-3-yl ester
   starting from (3S,4S,5R)-4-{4-[(S)-1-(3-fluoro-phenyl)-pyrrolidin-3-yloxy]-phenyl}-3-hydroxy-5-[4-(3-methoxy-propyl)-3,4-dihydro-2H-benzo[1,4]oxazin-6-ylmethoxy]-piperidine-1-carboxylic acid benzyl ester [873945-20-1].
74 (S)-2-(2-Amino-acetylamino)-3-methyl-butyric acid (R)-2-{(3S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-3-yloxy}-1-methyl-ethyl ester
   starting from (R)-1-{(3S,4R,5R)-4-[4-(2-methoxy-ethoxymethyl)-phenyl]-5-[4-(3-methoxypropyl)-3,4-dihydro-2H-benzo[1,4]oxazin-6-ylmethoxy]-piperidin-3-yloxy}-propan-2-ol (example 73a).
83 (S)-2-(2-Amino-acetylamino)-3-methyl-butyric acid (R)-1-methoxymethyl-2-{(3S,4R,5R)-4-[4-((S)-3-methoxy-2-methyl-propoxymethyl)-phenyl]-5-[4-(3-methoxypropyl)-3,4-dihydro-2H-benzo[1,4]oxazin-6-ylmethoxy]-piperidin-3-yloxy}-ethyl ester
   starting from (R)-1-methoxy-3-{(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-yloxy}-propan-2-ol (example 82a).
92 (S)-2-(2-Amino-acetylamino)-3-methyl-butyric acid (R)-2-{(3S,4R,5R)-4-[4-(4-methoxy-butoxy)-phenyl]-5-[4-(3-methoxy-propyl)-3,4-dihydro-2H-benzo[1,4]oxazin-6-ylmethoxy]-piperidin-3-yloxy}-1-methyl-ethyl ester
   starting from (R)-1-{(3S,4R,5R)-4-[4-(4-methoxy-butoxy)-phenyl]-5-[4-(3-methoxy-propyl)-3,4-dihydro-2H-benzo[1,4]oxazin-6-ylmethoxy]-piperidin-3-yloxy}-propan-2-ol (example 91 a).
101 (S)-2-(2-Amino-acetylamino)-3-methyl-butyric acid (3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-ylmethyl ester
   starting form (3S,4R,5R)-3-hydroxymethyl-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidine-1-carboxylic acid benzyl ester (example 100a).
110 (S)-2-(2-Amino-acetylamino)-3-methyl-butyric acid 1-methoxymethyl-2-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-1-methyl-ethyl ester (diastereomeric mixture)
   starting from (R,S)-1-methoxy-3-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2-methyl-propan-2-ol (diastereomeric mixture) (example 109a).

### Example 3

### (S)-2-(2-Amino-acetylamino)-3-methyl-butyric acid (3S,4R,5R)-1-((S)-2-amino-3-methylbutyryl)-4-(4-methoxy-phenyl)-5-[4-(3-methoxy-propyl)-3,4-dihydro-2H-benzo[1,4]oxazin-6-ylmethoxy]-piperidin-3-yl ester

According to general procedure E, (S)-2-(2-benzyloxycarbonylamino-acetylamino)-3-methylbutyric acid (3S,4R,5R)-1-((S)-2-benzyloxycarbonylamino-3-methyl-butyryl)-4-(4-methoxyphenyl)-5-[4-(3-methoxy-propyl)-3,4-dihydro-2H-benzo[1,4]oxazin-6-ylmethoxy]-piperidin-3-yl ester is used to afford the title compound, which is identified based on its Rf value.

The starting materials are prepared as follows:
a) (S)-2-(2-Benzyloxycarbonylamino-acetylamino)-3-methyl-butyric acid (3S,4R,5R)-1-((S)-2-benzyloxycarbonylamino-3-methyl-butyryl)-4-(4-methoxy-phenyl)-5-[4-(3-methoxy-propyl)-3,4-dihydro-2H-benzo[1,4]oxazin-6-ylmethoxy]-piperidin-3-yl ester
   According to general procedure G, ((S)-1-{(3S,4S,5R)-3-hydroxy-4-(4-methoxy-phenyl)-5-[4-(3-methoxy-propyl)-3,4-dihydro-2H-benzo[1,4]oxazin-6-ylmethoxy]-piperidine-1-carbonyl}-2-methyl-propyl)-carbamic acid benzyl ester and (S)-2-(2-benzyloxycarbonylamino-acetylamino)-3-methyl-butyric acid [33912-87-7] are used to afford the title compound, which is identified based on its Rf value.
b) ((S)-1-{(3S,4S,5R)-3-Hydroxy-4-(4-methoxy-phenyl)-5-[4-(3-methoxy-propyl)-3,4-dihydro-2H-benzo[1,4]oxazin-6-ylmethoxy]-piperidine-1-carbonyl}-2-methyl-propyl}-carbamic acid benzyl ester
   According to general procedure D, (3S,4S,5R)-4-(4-methoxy-phenyl)-5-[4-(3-methoxy-propyl)-3,4-dihydro-2H-benzo[1,4]oxazin-6-ylmethoxy]-piperidin-3-ol [873945-55-2] and (S)-2-benzyloxycarbonylamino-3-methyl-butyric acid [1149-26-4] are used to afford the title compound, which is identified based on its Rf value.

According to the procedure described in example 3, the following compounds are prepared in analogous manner:
12 (S)-2-(2-Amino-acetylamino)-3-methyl-butyric acid (3S,4R,5R)-1-((S)-2-amino-3-methyl-butyryl)-4-{4-[3-(2-methoxy-benzyloxy)-propoxy]-phenyl}-5-[1-(3-methoxy-propyl)-3,3-dimethyl-2,3-dihydro-1H-indol-6-ylmethoxy]-piperidin-3-yl ester
   starting from (3S,4S,5R)-4-{4-[3-(2-methoxy-benzyloxy)-propoxy]-phenyl}-5-[1-(3-methoxy-propyl)-3,3-dimethyl-2,3-dihydro-1 H-indol-6-ylmethoxy]-piperidin-3-ol.

The starting material is prepared as follows:
a) (3S,4S,5R)-4-{4-[3-(2-Methoxy-benzyloxy)-propoxy]-phenyl}-5-[1-(3-methoxy-propyl)-3,3-dimethyl-2,3-dihydro-1H-indol-6-ylmethoxy]-piperidin-3-ol
   According to general procedure E, (3S,4S,5R)-3-hydroxy-4-{4-[3-(2-methoxy-benzyloxy)-propoxy]-phenyl}-5-[1-(3-methoxy-propyl)-3,3-dimethyl-2,3-dihydro-1H-indol-6-ylmethoxy]-piperidine-1-carboxylic acid benzyl ester (example 10a) is used to afford the title compound, which is identified based on its Rf value.

### 21 (S)-2-(2-Amino-acetylamino)-3-methyl-butyric acid (3S,4R,5R)-1-((S)-2-amino-3-methyl-butyryl)-4-{4-[3-(2-methoxy-benzyloxy)-propoxy]-phenyl}-5-[4-methoxy-3-(3-methoxy-propoxy)-benzyloxy]-piperidin-3-yl ester

starting from (3S,4S,5R)-4-{4-[3-(2-methoxy-benzyloxy)-propoxy]-phenyl}-5-[4-methoxy-3-(3-methoxy-propoxy)-benzyloxy]-piperidin-3-ol.

The starting material is prepared as follows:
a) (3S,4S,5R)-4-{4-[3-(2-Methoxy-benzyloxy)-propoxyl-phenyl}-5-[4-methoxy-3-(3-methoxy-propoxy)-benzyloxy]-piperidin-3-ol
   According to general procedure E, (3S,4S,5R)-3-hydroxy-4-{4-[3-(2-methoxy-benzyloxy)-propoxy]-phenyl}-5-[4-methoxy-3-(3-methoxy-propoxy)-benzyloxy]-piperidine-1-carboxylic acid benzyl ester (example 19a) is used to afford the title compound, which is identified based on its Rf value.

### 30 (S)-2-(2-Amino-acetylamino)-3-methyl-butyric acid (3S,4R,5R)-1-((S)-2-amino-3-methyl-butyryl)-4-{4-[3-(2-chloro-phenoxy)-propoxy]-phenyl}-5-[4-(3-methoxy-propyl)-3,4-dihydro-2H-benzo[1,4]oxazin-6-ylmethoxy]-piperidin-3-yl ester

starting from (3S,4S,5R)-4-{4-[3-(2-chloro-phenoxy)-propoxy]-phenyl}-5-[4-(3-methoxy-propyl)-3,4-dihydro-2H-benzo[1,4]oxazin-6-ylmethoxy]-piperidin-3-ol [873945-66-5].

### 39 (S)-2-(2-Amino-acetylamino)-3-methyl-butyric acid (3S,4R,5R)-1-((S)-2-amino-3-methyl-butyryl)-4-{4-[3-(2-methoxy-benzyloxy)-propoxy]-phenyl}-5-[4-(3-methoxy-propyl)-3,4-dihydro-2H-benzo[1,4]oxazin-6-ylmethoxy]-piperidin-3-yl ester

starting from (3S,4S,5R)-4-{4-[3-(2-methoxy-benzyloxy)-propoxy]-phenyl}-5-[4-(3-methoxy-propyl)-3,4-dihydro-2H-benzo[1,4]oxazin-6-ylmethoxy]-piperidin-3-ol [873945-50-7].

### 48 (S)-2-(2-Amino-acetylamino)-3-methyl-butyric acid (3S,4R,5R)-1-((S)-2-amino-3-methyl-butyryl)-4-[4-(4-ethyl-phenoxy)-phenyl]-5-[4-(3-methoxy-propyl)-3,4-dihydro-2H-benzo[1,4]oxazin-6-ylmethoxy]-piperidin-3-yl ester

starting from (3S,4S,5R)-4-[4-(4-ethyl-phenoxy)-phenyl]-5-[4-(3-methoxy-propyl)-3,4-dihydro-2H-benzo[1,4]oxazin-6-ylmethoxy]-piperidin-3-ol.

The starting material is prepared as follows:
a) (3S,4S,5R)-4-[4-(4-Ethyl-phenoxy)-phenyl]-5-[4-(3-methoxy-propyl)-3,4-dihydro-2H-benzo[1,4]oxazin-6-ylmethoxy]-piperidin-3-ol
   According to general procedure E, (3S,4S,5R)-4-[4-(4-ethyl-phenoxy)-phenyl]-3-hydroxy-5-[4-(3-methoxy-propyl)-3,4-dihydro-2H-benzo[1,4]oxazin-6-ylmethoxy]-piperidine-1-carboxylic acid benzyl ester (example 46a) is used to afford the title compound, which is identified based on its Rf value.

### 57 (S)-2-(2-Amino-acetylamino)-3-methyl-butyric acid (3S,4R,5R)-5-{2-[2-(2-acetylaminoethyl)-phenoxy]-ethoxy}-1-((S)-2-amino-3-methyl-butyryl)-4-{4-[3-(2-methoxy-benzyloxy)-propoxy]-phenyl}-piperidin-3-yl ester

starting from (N-(2-{2-[2-((3R,4S,5S)-5-hydroxy-4-{4-[3-(2-methoxy-benzyloxy)-propoxy]-phenyl}-piperidin-3-yloxy)-ethoxy]-phenyl}-ethyl)-acetamide.

The starting material is prepared as follows:
a) (N-(2-{2-[2-((3R,4S,5S)-5-Hydroxy-4-{4-[3-(2-methoxy-benzyloxy)-propoxy]-phenyl}-piperidin-3-yloxy)-ethoxy]-phenyl}-ethyl)-acetamide
   According to general procedure E, (3R,4S,5S)-3-{2-[2-(2-acetylamino-ethyl)-phenoxy]-ethoxy}-5-hydroxy-4-{4-[3-(2-methoxy-benzyloxy)-propoxy]-phenyl}-piperidine-1-carboxylic acid benzyl ester (example 55a) is used to afford the title compound, which is identified based on its Rf value.

### 66 (S)-2-(2-Amino-acetylamino)-3-methyl-butyric acid (3S,4R,5R)-1-((S)-2-amino-3-methyl-butyryl)-4-{4-[(S)-1-(3-fluoro-phenyl)-pyrrolidin-3-yloxy]-phenyl}-5-[4-(3-methoxy-propyl)-3,4-dihydro-2H-benzo[1,4]oxazin-6-ylmethoxy]-piperidin-3-yl ester

starting from (3S,4S,5R)-4-{4-[(S)-1-(3-fluoro-phenyl)-pyrrolidin-3-yloxy]-phenyl}-5-[4-(3-methoxy-propyl)-3,4-dihydro-2H-benzo[1,4]oxazin-6-ylmethoxy]-piperidin-3-ol [873945-18-7].

### 75 (S)-2-(2-Amino-acetylamino)-3-methyl-butyric acid (R)-2-{(3S,4R,5R)-1-((S)-2-amino-3-methyl-butyryl)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-3-yloxy}-1-methyl-ethyl ester

starting from (R)-1-{(3S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-3-yloxy}-propan-2-ol (example 73a).

### 84 (S)-2-(2-Amino-acetylamino)-3-methyl-butyric acid (R)-1-methoxymethyl-2-{(3S,4R,5R)-1-((S)-2-amino-3-methyl-butyryl)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-yloxy}-ethyl ester

starting from (R)-1-methoxy-3-{(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-yloxy}-propan-2-ol (example 82a).

### 93 (S)-2-(2-Amino-acetylamino)-3-methyl-butyric acid (R)-2-{(3S,4R,5R)-1-((S)-2-amino-3-methyl-butyryl)-4-[4-(4-methoxy-butoxy)-phenyl]-5-[4-(3-methoxy-propyl)-3,4-dihydro-2H-benzo[1,4]oxazin-6-ylmethoxy]-piperidin-3-yloxy}-1-methyl-ethyl ester

starting from (R)-1-{(3S,4R,5R)-4-[4-(4-methoxy-butoxy)-phenyl]-5-[4-(3-methoxy-propyl)-3,4-dihydro-2H-benzo[1,4]oxazin-6-ylmethoxy]-piperidin-3-yloxy}-propan-2-ol (example 91 a).

### 102 (S)-2-(2-Amino-acetylamino)-3-methyl-butyric acid (3S,4R,5R)-1-((S)-2-amino-3-methylbutyryl)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-ylmethyl ester

starting from {(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-yl}-methanol.

The starting material is prepared as follows:
a) {(3S,4R,5R)-4-[4-((S)-3-Methoxy-2-methyl-propoxymethyl)-phenyl]-5-[4-(3-methoxy-propyl)-3,4-dihydro-2H-benzo[1,4]oxazin-6-ylmethoxy]-piperidin-3-yl}-methanol
   According to general procedure E, (3S,4R,5R)-3-hydroxymethyl-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidine-1-carboxylic acid benzyl ester (example 100a) is used to afford the title compound, which is identified based on its Rf value.

### 111 (S)-2-(2-Amino-acetylamino)-3-methyl-butyric acid 2-{(2R,4R,5R)-1-((S)-2-amino-3-methyl-butyryl)-4-(4-methoxy-phenyl)-5-[4-(3-methoxy-propyl)-3,4-dihydro-2H-benzo[1,4]oxazin-6-ylmethoxy]-piperidin-2-yl}-1-methoxymethyl-1-methyl-ethyl ester (diastereomeric mixture)

starting from (R,S)-1-methoxy-3-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2-methyl-propan-2-ol (diastereomeric mixture) (example 109a).

### Example 4

### (S)-2-Amino-3-methyl-butyric acid (3S,4R,5R)-1-((S)-2-amino-3-methyl-butyryl)-4-(4-methoxy-phenyl)-5-[4-(3-methoxy-propyl)-3,4-dihydro-2H-benzo[1,4]oxazin-6-ylmethoxy]-piperidin-3-yl ester

According to the process described in example 3, 3a and 3b, using (S)-2-benzyloxycarbonylamino-3-methyl-butyric acid [1149-26-4] in step a, the title compound is obtained starting from (3S,4S,5R)-4-(4-methoxy-phenyl)-5-[4-(3-methoxy-propyl)-3,4-dihydro-2H-benzo[1,4]oxazin-6-ylmethoxy]-piperidin-3-ol [873945-55-2], and is identified based on its Rf value.

According to the procedure described in example 4, the following compounds are prepared in analogous manner:
13 (S)-2-Amino-3-methyl-butyric acid (3S,4R,5R)-1-((S)-2-amino-3-methyl-butyryl)-4-{4-[3-(2-methoxy-benzyloxy)-propoxy]-phenyl}-5-[1-(3-methoxy-propyl)-3,3-dimethyl-2,3-dihydro-1H-indol-6-ylmethoxy]-piperidin-3-yl ester
   starting from (3S,4S,5R)-4-{4-[3-(2-methoxy-benzyloxy)-propoxy]-phenyl}-5-[1-(3-methoxy-propyl)-3,3-dimethyl-2,3-dihydro-1 H-indol-6-ylmethoxy]-piperidin-3-ol (example 12a).
22 (S)-2-Amino-3-methyl-butyric acid (3S,4R,5R)-1-((S)-2-amino-3-methyl-butyryl)-4-{4-[3-(2-methoxy-benzyloxy)-propoxy]-phenyl}-5-[4-methoxy-3-(3-methoxy-propoxy)-benzyloxy]-piperidin-3-yl ester
   starting from (3S,4S,5R)-4-{4-[3-(2-methoxy-benzyloxy)-propoxy]-phenyl}-5-[4-methoxy-3-(3-methoxy-propoxy)-benzyloxy]-piperidin-3-ol (example 21 a).
31 (S)-2-Amino-3-methyl-butyric acid (3S,4R,5R)-1-((S)-2-amino-3-methyl-butyryl)-4-{4-[3-(2-chloro-phenoxy)-propoxy]-phenyl}-5-[4-(3-methoxy-propyl)-3,4-dihydro-2H-benzo[1,4]oxazin-6-ylmethoxy]-piperidin-3-yl ester
   starting from (3S,4S,5R)-4-{4-[3-(2-chloro-phenoxy)-propoxy]-phenyl}-5-[4-(3-methoxy-propyl)-3,4-dihydro-2H-benzo[1,4]oxazin-6-ylmethoxy]-piperidin-3-ol [873945-66-5].
40 (S)-2-Amino-3-methyl-butyric acid (3S,4R,5R)-1-((S)-2-amino-3-methyl-butyryl)-4-{4-[3-(2-methoxy-benzyloxy)-propoxy]-phenyl}-5-[4-(3-methoxy-propyl)-3,4-dihydro-2H-benzo[1,4]oxazin-6-ylmethoxy]-piperidin-3-yl ester
   starting from (3S,4S,5R)-4-{4-[3-(2-methoxy-benzyloxy)-propoxy]-phenyl}-5-[4-(3-methoxy-propyl)-3,4-dihydro-2H-benzo[1,4]oxazin-6-ylmethoxy]-piperidin-3-ol [873945-50-7].
49 (S)-2-Amino-3-methyl-butyric acid (3S,4R,5R)-1-((S)-2-amino-3-methyl-butyryl)-4-[4-(4-ethyl-phenoxy)-phenyl]-5-[4-(3-methoxy-propyl)-3,4-dihydro-2H-benzo[1,4]oxazin-6-ylmethoxy]-piperidin-3-yl ester
   starting from (3S,4S,5R)-4-[4-(4-ethyl-phenoxy)-phenyl]-5-[4-(3-methoxy-propyl)-3,4-dihydro-2H-benzo[1,4]oxazin-6-ylmethoxy]-piperidin-3-ol (example 48a).
58 (S)-2-Amino-3-methyl-butyric acid (3S,4R,5R)-5-{2-[2-(2-acetylamino-ethyl)-phenoxy]-ethoxy}-1-((S)-2-amino-3-methyl-butyryl)-4-{4-[3-(2-methoxy-benzyloxy)-propoxyl-phenyl}-piperidin-3-yl ester
   starting from (N-(2-{2-[2-((3R,4S,5S)-5-hydroxy-4-{4-[3-(2-methoxy-benzyloxy)-propoxy]-phenyl}-piperidin-3-yloxy)-ethoxy]-phenyl}-ethyl)-acetamide (example 57a).
67 (S)-2-Amino-3-methyl-butyric acid (3S,4R,5R)-1-((S)-2-amino-3-methyl-butyryl)-4-{4-[(S)-1-(3-fluoro-phenyl)-pyrrolidin-3-yloxy]-phenyl}-5-[4-(3-methoxy-propyl)-3,4-dihydro-2H-benzo[1,4]oxazin-6-ylmethoxy]-piperidin-3-yl ester
   starting from (3S,4S,5R)-4-{4-[(S)-1-(3-fluoro-phenyl)-pyrrolidin-3-yloxy]-phenyl}-5-[4-(3-methoxy-propyl)-3,4-dihydro-2H-benzo[1,4]oxazin-6-ylmethoxy]-piperidin-3-ol [873945-18-7].
76 (S)-2-Amino-3-methyl-butyric acid (R)-2-{(3S,4R,5R)-1-((S)-2-amino-3-methylbutyryl)-4-[4-(2-methoxy-ethoxymethyl)-phenyl]-5-[4-(3-methoxy-propyl)-3,4-dihydro-2H-benzo[1,4]oxazin-6-ylmethoxyl-piperidin-3-yloxy}-1-methyl-ethyl ester
   starting from (R)-1-{(3S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-3-yloxy}-propan-2-ol (example 73a).
85 (S)-2-Amino-3-methyl-butyric acid (R)-1-methoxymethyl-2-{(3S,4R,5R)-1-((S)-2-amino-3-methyl-butyryl)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-yloxy}-ethyl ester
   starting from (R)-1-methoxy-3-{(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-yloxyl-propan-2-ol (example 82a).
94 S)-2-Amino-3-methyl-butyric acid (R)-2-{(3S,4R,5R)-1-((S)-2-amino-3-methyl-butyryl)-4-[4-(4-methoxy-butoxy)-phenyl]-5-[4-(3-methoxy-propyl)-3,4-dihydro-2H-benzo[1,4]oxazin-6-ylmethoxy]-piperidin-3-yloxy}-1-methyl-ethyl ester
   starting from (R)-1-{(3S,4R,5R)-4-[4-(4-methoxy-butoxy)-phenyl]-5-[4-(3-methoxy-propyl)-3,4-dihydro-2H-benzo[1,4]oxazin-6-ylmethoxy]-piperidin-3-yloxy}-propan-2-ol (example 91 a).
103 (S)-2-Amino-3-methyl-butyric acid (3S,4R,5R)-1-((S)-2-amino-3-methyl-butyryl)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-ylmethyl ester
   starting from {(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-yl}-methanol (example 102a).
112 (S)-2-Amino-3-methyl-butyric acid 2-{(2R,4R,5R)-1-((S)-2-amino-3-methyl-butyryl)-4-(4-methoxy-phenyl)-5-[4-(3-methoxy-propyl)-3,4-dihydro-2H-benzo[1,4]oxazin-6-ylmethoxyl-piperidin-2-yl}-1-methoxymethyl-1-methyl-ethyl ester (diastereomeric mixture)
   starting from (R,S)-1-methoxy-3-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2-methyl-propan-2-ol (diastereomeric mixture) (example 109a).

### Example 5

### (S)-2-Amino-1-{(3S,4S,5R)-3-hydroxy-4-(4-methoxy-phenyl)-5-[4-(3-methoxy-propyl)-3,4-dihydro-2H-benzo[1,4]oxazin-6-ylmethoxy]-piperidin-1-yl}-3-methyl-butan-1-one

According to general procedure E, ((S)-1-{(3S,4S,5R)-3-hydroxy-4-(4-methoxy-phenyl)-5-[4-(3-methoxy-propyl)-3,4-dihydro-2H-benzo[1,4]oxazin-6-ylmethoxy]-piperidine-1-carbonyl}-2-methyl-propyl)-carbamic acid benzyl ester is used to afford the title compound, which is identified based on its Rf value.

The starting material is prepared as follows:
a) ((S)-1-{(3S,4S,5R)-3-Hydroxy-4-(4-methoxy-phenyl)-5-[4-(3-methoxy-propyl)-3,4-dihydro-2H-benzo[1,4]oxazin-6-ylmethoxyl-piperidine-1-carbonyl}-2-methyl-propyl)-carbamic acid benzyl ester
   According to general procedure D, (3S,4S,5R)-4-(4-methoxy-phenyl)-5-[4-(3-methoxy-propyl)-3,4-dihydro-2H-benzo[1,4]oxazin-6-ylmethoxy]-piperidin-3-ol [873945-55-2] and (S)-2-benzyloxycarbonylamino-3-methyl-butyric acid [1149-26-4] are used to afford the title compound, which is identified based on its Rf value.

According to the procedure described in example 5, the following compounds are prepared in analogous manner:
14 (S)-2-Amino-1-{(3S,4S,5R)-3-hydroxy-4-{4-[3-(2-methoxy-benzyloxy)-propoxy]-phenyl}-5-[1-(3-methoxy-propyl)-3,3-dimethyl-2,3-dihydro-1H-indol-6-ylmethoxyl-piperidin-1-yl}-3-methyl-butan-1-one
   starting from (3S,4S,5R)-4-{4-[3-(2-methoxy-benzyloxy)-propoxy]-phenyl}-5-[1-(3-methoxy-propyl)-3,3-dimethyl-2,3-dihydro-1H-indol-6-ylmethoxy]-piperidin-3-ol (example 12a).
23 (S)-2-Amino-1-{(3S,4S,5R)-3-hydroxy-4-{4-[3-(2-methoxy-benzyloxy)-propoxy]-phenyl}-5-[4-methoxy-3-(3-methoxy-propoxy)-benzyloxyl-piperidin-1-yl}-3-methyl-butan-1-one
   starting from (3S,4S,5R)-4-{4-[3-(2-methoxy-benzyloxy)-propoxy]-phenyl}-5-[4-methoxy-3-(3-methoxy-propoxy)-benzyloxy]-piperidin-3-ol (example 21 a).
32 (S)-2-Amino-1-{(3S,4S,5R)-4-{4-[3-(2-chloro-phenoxy)-propoxy]-phenyl}-3-hydroxy-5-[4-(3-methoxy-propyl)-3,4-dihydro-2H-benzo[1,4]oxazin-6-ylmethoxy]-piperidin-1-yl}-3-methyl-butan-1-one
   starting from (3S,4S,5R)-4-{4-[3-(2-chloro-phenoxy)-propoxy]-phenyl}-5-[4-(3-methoxy-propyl)-3,4-dihydro-2H-benzo[1,4]oxazin-6-ylmethoxy]-piperidin-3-ol [873945-66-5].
41 (S)-2-Amino-1-{(3S,4S,5R)-3-hydroxy-4-{4-[3-(2-methoxy-benzyloxy)-propoxy]-Phenyl}-5-[4-(3-methoxy-propyl)-3,4-dihidro-2H-benzo[1,4]oxazin-6-ylmethoxy]-piperidin-1-yl}-3-methyl-butan-1-one
   starting from (3S,4S,5R)-4-{4-[3-(2-methoxy-benzyloxy)-propoxy]-phenyl}-5-[4-(3-methoxy-propyl)-3,4-dihydro-2H-benzo[1,4]oxazin-6-ylmethoxy]-piperidin-3-ol [873945-50-7].
50 (S)-2-Amino-1-{(3S,4S,5R)-4-[4-(4-ethyl-phenoxy)-phenyl]-3-hydroxy-5-[4-(3-methoxy-propyl)-3,4-dihydro-2H-benzo[1,4]oxazin-6-ylmethoxy]-piperidin-1-yl}-3-methyl-butan-1-one
   starting from (3S,4S,5R)-4-[4-(4-ethyl-phenoxy)-phenyl]-5-[4-(3-methoxy-propyl)-3,4-dihydro-2H-benzo[1,4]oxazin-6-ylmethoxy]-piperidin-3-ol (example 48a).
59 N-(2-{2-[2-((3R,4S,5S)-1-((S)-2-Amino-3-methyl-butyryl)-5-hydroxy-4-{4-[3-(2-methoxy-benzyloxy)-propoxy]-phenyl}-piperidin-3-yloxy)-ethoxy]-phenyl}-ethyl)-acetamide
   starting from (N-(2-{2-[2-((3R,4S,5S)-5-hydroxy-4-{4-[3-(2-methoxy-benzyloxy)-propoxy]-phenyl}-piperidin-3-yloxy)-ethoxy]-phenyl}-ethyl)-acetamide (example 57a).
68 (S)-2-Amino-1-{(3S,4S,5R)-4-{4-[(S)-1-(3-fluoro-phenyl)-pyrrolidin-3-yloxy]-phenyl}-3-hydroxy-5-[4-(3-methoxy-propyl)-3,4-dihydro-2H-benzo[1,4]oxazin-6-ylmethoxyl-piperidin-1-yl}-3-methyl-butan-1-one
   starting from (3S,4S,5R)-4-{4-[(S)-1-(3-fluoro-phenyl)-pyrrolidin-3-yloxy]-phenyl}-5-[4-(3-methoxy-propyl)-3,4-dihydro-2H-benzo[1,4]oxazin-6-ylmethoxy]-piperidin-3-ol [873945-18-7].
77 (S)-2-Amino-1-{(3S,4R,5R)-3-((R)-2-hydroxy-propoxy)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-1-yl}-3-methyl-butan-1-one
   starting from (R)-1-{(3S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-3-yloxy}-propan-2-ol (example 73a).
86 S)-2-Amino-1-{(3S,4R,5R)-3-((R)-2-hydroxy-3-methoxy-propoxy)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-1-yl}-3-methyl-butan-1-one
   starting from (R)-1-methoxy-3-{(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-yloxy}-propan-2-ol (example 82a).
95 (S)-2-Amino-1-{(3S,4R,5R)-3-((R)-2-hydroxy-propoxy)-4-[4-(4-methoxy-butoxy)-phenyl]-5-[4-(3-methoxy-propyl)-3,4-dihydro-2H-benzo[1,4]oxazin-6-ylmethoxy]-piperidin-1-yl}-3-methyl-butan-1-one
   starting from (R)-1-{(3S,4R,5R)-4-[4-(4-methoxy-butoxy)-phenyl]-5-[4-(3-methoxy-propyl)-3,4-dihydro-2H-benzo[1,4]oxazin-6-ylmethoxy]-piperidin-3-yloxy}-propan-2-ol (example 91 a).
104 (S)-2-Amino-1-{(3S,4R,5R)-3-hydroxymethyl-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-1-yl}-3-methyl-butan-1-one
   starting from {(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-yl}-methanol (example 102a).
113 (S)-2-Amino-1-{(2R,4R,5R)-2-(2-hydroxy-3-methoxy-2-methyl-propyl)-4-(4-methoxyPhenyl)-5-[4-(3-methoxy-propyl)-3,4-dihydro-2H-benzo[1,4]oxazin-6-ylmethoxyl-piperidin-1-yl}-3-methyl-butan-1-one (diastereomeric mixture)
   starting from (R,S)-1-methoxy-3-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2-methyl-propan-2-ol (diastereomeric mixture) (example 109a).
118 3-{(2S,4R,5R)-1-((S)-2-Amino-3-methyl-butyryl)-4-(4-methoxy-phenyl)-5-[4-(3-methoxy-propyl)-3,4-dihydro-2H-benzo[1,4]oxazin-6-ylmethoxyl-piperidin-2-yl}-2,2,N-trimethyl-propionamide
   starting from 3-{(2S,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2,2,N-trimethyl-propionamide

The starting materials are prepared as follows:
a) 3-{(2S,4R,5R)-4-(4-Methoxy-phenyl)-5-[4-(3-methoxy-propyl)-3,4-dihydro-2H-benzo[1,4]oxazin-6-ylmethoxyl-piperidin-2-yl}-2,2,N-trimethyl-propionamide
   According to general procedure A, 3-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2,N-trimethyl-propionamide is used to afford the title compound.
b) 3-[(2S,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2,N-trimethyl-propionamide
   According to general procedure D, 3-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid and methylamine are used to afford the title compound as a brown oil. Rf = 0.18 (EtOAc-heptane 5:1); Rt = 4.97 (gradient I).
c) 3-[(2S,4R,5R)-4-(4-Methoxy-phenyl)-5-[4-(3-methoxy-propyl)-3,4-dihydro-2H-benzo[1,4]oxazin-6-ylmethoxyl-1-(toluene-4-sulfonyl)-piperidin-2-yl]-2,2-dimethyl-propionic acid
   To a solution of 8.62 g of 3-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid methyl ester in 20 ml tetrahydrofuran and 50 ml ethanol are added 60 ml of a 20% sodium hydroxide solution. The reaction mixture is stirred for 4 hours at 65°C. The organic solvents are evaporated under reduced pressure and the remaining solution is acidified with 6M aqueous hydrochloric acid until pH 2. This mixture is extracted with 200 ml ethyl acetate (3x). The combined organic layers are washed with brine and dried with sodium sulfate. The organic layer is filtered and evaporated under reduced pressure. The residue is purified by flash chromatography (SiO₂ 60F) to afford the title compound as a colourless foam. Rf = 0.27 (EtOAc-heptane 3:1); Rt = 5.10 (gradient I).
d) 3-[(2S,4R,5R)-4-(4-Methoxy-phenyl)-5-[4-(3-methoxy-propyl)-3,4-dihydro-2H-benzo[1,4]oxazin-6-ylmethoxyl-1-(toluene-4-sulfonyl)-piperidin-2-yl]-2,2-dimethyl-propionic acid methyl ester
   To a solution of 5.75 ml methyl isobutyrate in 28 ml tetrahydrofuran at -78°C are added 100 ml of a 0.5M lithiumdiisopropyl amide solution and the reaction mixture is stirred for 30 minutes at -78°C. Then 17.74 ml hexamethylphosphoramide is added at -78°C and the mixture is stirred for further 30 minutes. A solution of 8.66 g 6-[(3R,4R,6S)-6-bromomethyl-4-(4-methoxy-phenyl)-1-(toluene-4-sulfonyl)-piperidin-3-yloxymethyl]-4-(3-methoxy-propyl)-3,4-dihydro-2H-benzo[1,4]oxazine in 28 ml tetrahydrofuran is then added dropwise to the "enolate" at -78°C and the solution is stirred for 30 minutes. The reaction mixture is allowed to warm to -12°C and stirred at this temperature for additional 40 minutes. The solution is quenched with 1 M aqueous hydrochloric acid and extracted with 200 ml ethyl acetate (3x). The combined organic layers are washed with brine and dried with sodium sulfate. The organic layer is filtered and evaporated under reduced pressure. The residue is purified by flash chromatography (SiO₂ 60F) to afford the title compound as a colourless foam. Rf = 0.29 (EtOAc-heptane 1:1); Rt = 5.65 (gradient I).
e) 6-[(3R,4R,6S)-6-Bromomethyl-4-(4-methoxy-phenyl)-1-(toluene-4-sulfonyl)-piperidin-3-yloxymethyl]-4-(3-methoxy-propyl)-3,4-dihydro-2H-benzo[1,4]oxazine
   To a solution of 11.18 g of methanesulfonic acid (2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-ylmethyl ester (example 109g) in 40 ml dimethylformamide is added 13.78 g lithium bromide and the reaction mixture is stirred for 14 hours at 65°C. The reaction mixture is allowed to warm to room temperature and 50 ml water is added. This mixture is extracted with 300 ml tert-butyl methyl ether (3x). The combined organic layers are washed with brine and dried with sodium sulfate. The organic layer is filtered and evaporated under reduced pressure. The residue is purified by flash chromatography (SiO₂ 60F) to afford the title compound as a colourless foam. Rf = 0.39 (EtOAc-heptane 1:1); Rt = 5.68 (gradient l).

### 123 3-{(2S,4R,5R)-1-((S)-2-Amino-3-methyl-butyryl)-4-[4-(3-methoxy-propoxy)-phenyl]-5-[4-(3-methoxy-propyl)-3,4-dihydro-2H-benzo[1,4]oxazin-6-ylmethoxy]-piperidin-2-yl}-2,2,N-trimethyl-propionamide

starting from 3-{(2S,4R,5R)-4-[4-(3-methoxy-propoxy)-phenyl]-5-[4-(3-methoxy-propyl)-3,4-dihydro-2H-benzo[1,4]oxazin-6-ylmethoxy]-piperidin-2-yl}-2,2,N-trimethyl-propionamide.

The starting materials are prepared as follows:
a) 3-{(2S,4R,5R)-4-[4-(3-Methoxy-propoxy)-phenyl]-5-[4-(3-methoxy-propyl)-3,4-dihydro-2H-benzo[1,4]oxazin-6-ylmethoxy]-piperidin-2-yl}-2,2,N-trimethyl-propionamide
   According to general procedure A, 3-[(2S,4R,5R)-4-[4-(3-methoxy-propoxy)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-2,2,N-trimethyl-propionamide is used to afford the title compound.
b) 3-[(2S,4R,5R)-4-[4-(3-Methoxy-propoxy)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-2,2,N-trimethyl-propionamide
   According to general procedure D, 3-[(2S,4R,5R)-4-[4-(3-methoxy-propoxy)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid and methylamine are used to afford the title compound as a yellow oil. Rf = 0.05 (EtOAc-heptane 1:1); Rt = 5.10 (gradient I).
c) 3-[(2S,4R,5R)-4-[4-(3-Methoxy-propoxy)-phenyl]-5-[4-(3-methoxy-propyl)-3,4-dihydro-2H-benzo[1,4]oxazin-6-ylmethoxy]-1-(toluene-4-sulfonyl)}-piperidin-2-yl]-2,2-dimethyl-propionic acid
   A solution of 806 mg of 3-[(2S,4R,5R)-4-[4-(3-methoxy-propoxy)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid 3-methoxy-propyl ester in 6 ml methanol-tetrahydrofuran-water 1:1:1 is cooled to 0°C. Lithium hydroxide (0.073 g) is added and the mixture heated to 65°C for 16 hours. The mixture is acidified with 1 M hydrochloric acid and extracted with tert-butyl methyl ether (2x). The combined organic phases are dried over sodium sulfate, filtered and concentrated under reduced pressure. The residue is purified via flash chromatography (SiO₂ 60F) to afford the title compound as a yellow oil. Rf = 0.17(EtOAc-heptane 1:1); Rt = 5.25 (gradient I).
d) 3-[(2S,4R,5R)-4-[4-(3-Methoxy-propoxy)-phenyl]-5-[4-(3-methoxy-propyl)-3,4-dihydro-2H-benzo[1,4]oxazin-6-ylmethoxyl-1-(toluene-4-sulfonyl)-piperidin-2-yl]-2,2-dimethyl-propionic acid 3-methoxy-propyl ester
   To a stirred solution of 702 mg of 3-[(2S,4R,5R)-4-(4-hydroxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid in 5 ml N,N-dimethylformamide is added 1.34 g of caesium carbonate and 890 mg of 3-methoxypropyl chloride. The reaction mixture is heated to 50°C overnight, diluted with 1 N hydrochloric acid and extracted with ethyl acetate. The combined organic phases are dried and concentrated under reduced pressure to afford the crude title compound. The residue is purified by flash chromatography (SiO₂ 60F) to afford the title compound as a yellow oil. Rt = 5.86 (gradient I).
e) 3-[(2S,4R,5R)-4-(4-Hydroxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid
   To a stirred solution of 1.1 g of 3-[(2S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid (example 118c) in 10 ml N,N-dimethylformamide are added 1.35 g of sodium ethanethiolate and the reaction mixture is heated to 95°C for 2 days. The reaction mixture is diluted with 1 N hydrochloric acid solution and extracted with tert-butyl methyl ether (3X). The organic phases are combined, dried over sodium sulfate and concentrated under reduced pressure to afford the crude title compound. The residue is purified by flash chromatography (SiO₂ 60F) to afford the title compound as a colourless oil. Rf = 0.10 (EtOAc-heptane 1:1); Rt = 4.67 (gradient I).

### 128 3-{(2S,4R,5R)-1-((S)-2-Amino-3-methyl-butyryl)-4-[4-(2-methoxy-ethoxymethyl)-phenyl]-5-[4-(3-methoxy-propyl)-3,4-dihydro-2H-benzo[1,4]oxazin-6-ylmethoxyl-piperidin-2-yl}-2,2,N-trimethyl-propionamide

starting from 3-{(2S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-2,2,N-trimethyl-propionamide.

The starting materials are prepared as follows:
a) 3-{(2S,4R,5R)-4-[4-(2-Methoxy-ethoxymethyl)-phenyl]-5-[4-(3-methoxy-propyl)-3,4-dihydro-2H-benzo[1,4]oxazin-6-ylmethoxy]-piperidin-2-yl]-2,2,N-trimethyl-propionamide
   According to general procedure A, 3-[(2S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-2,2,N-trimethyl-propionamide is used to afford the title compound.
b) 3-[(2S,4R,5R)-4-[4-(2-Methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-2,2,N-trimethyl-propionamide
   According to general procedure D, 3-[(2S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid and methylamine are used to afford the title compound as a yellow oil. Rf = 0.13 (EtOAc-heptane 5:1); Rt = 4.85 (gradient I).
c) 3-[(2S,4R,5R)-4-[4-(2-Methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid
   According to the procedure described in example 118c and 118d, 6-[(3R,4R,6S)-6-bromomethyl-4-[4-(2-methoxy-ethoxymethyl)-phenyl]-1-(toluene-4-sulfonyl)-piperidin-3-yloxymethyl]-4-(3-methoxy-propyl)-3,4-dihydro-2H-benzo[1,4]oxazine is used to afford the title compound as a yellow oil. Rf = 0.25 (EtOAc); Rt = 5.01 (gradient I).
d) 6-[(3R,4R,6S)-6-Bromomethyl-4-[4-(2-methoxy-ethoxymethyl)-phenyl]-1-(toluene-4-sulfonyl)-piperidin-3-yloxymethyl]-4-(3-methoxy-propyl)-3,4-dihydro-2H-benzo[1,4]oxazine
   According to the procedure described in example 118e, methanesulfonic acid (2S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-ylmethyl ester is used to afford the title compound as a yellow oil. Rf = 0.34 (EtOAc-heptane 2:1); Rt = 5.55 (gradient I).
e) Methanesulfonic acid (2S,4R,5R)-4-[4-(2-methoxy-ethoxymethyl)-phenyll-5-[4-(3-methoxy-propyl)-3,4-dihydro-2H-benzo[1,4]oxazin-6-ylmethoxyl-1-(toluene-4-sulfonyl)-piperidin-2-ylmethyl ester
   According to general procedure F, [(2S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-methanol is used to afford the title compound as a brown oil. Rf = 0.22 (EtOAc-heptane 4:1); Rt = 5.02 (gradient I).
f) [(2S,4R,5R)-4-[4-(2-Methoxy-ethoxymethyl)-phenyl]-5-[4-(3-methoxy-propyl)-3,4-dihydro-2H-benzo[1,4]oxazin-6-ylmethoxyl-1-(toluene-4-sulfonyl)-piperidin-2-yl]-methanol
   According to general procedure I, 6-[(3R,4R,6S)-4-[4-(2-methoxy-ethoxymethyl)-phenyl]-1-(toluene-4-sulfonyl)-6-triisopropylsilanyloxymethyl-piperidin-3-yloxymethyl]-4-(3-methoxy-propyl)-3,4-dihydro-2H-benzo[1,4]oxazine is used to afford the title compound as a yellow oil. Rf = 0.13 (EtOAc-heptane 3:1); Rt = 4.76 (gradient I).
g) 6-[(3R,4R,6S)-4-[4-(2-Methoxy-ethoxymethyl)-phenyl]-1-(toluene-4-sulfonyl)-6-triisopropylsilanyloxymethyl-piperidin-3-yloxymethyl]-4-(3-methoxy-propyl)-3,4-dihydro-2H-benzor[1,4]oxazine
   According to general procedure K (carried out at 70°C, with addition of 0.1 equivalents of tetrabutyl ammoniumiodide), {4-[(2S,4R,5R)-5-[4-(3-methoxy-propyl)-3,4-dihydro-2H-benzo[1,4]oxazin-6-ylmethoxy]-1-(toluene-4-sulfonyl)-2-trilsopropylsilanyloxymethyl-piperidin-4-yl]-phenyl}-methanol is used to afford the title compound as a yellow oil. Rf = 0.31 (EtOAc-heptane 1:1); Rt = 6.65 (gradient I).
h) {4-[(2S,4R,5R)-5-[4-(3-Methoxy-propyl)-3,4-dihydro-2H-benzo[1,4]oxazin-6-ylmethoxy]-1-(toluene-4-sulfonyl)-2-triisopropylsilanyloxymethyl-piperidin-4-yl]-phenyl}-methanol
   According to general procedure C, 4-[(2S,4R,5R)-5-[4-(3-methoxy-propyl)-3-oxo-3,4-dihydro-2H-benzo[1,4]oxazin-6-ylmethoxy]-1-(toluene-4-sulfonyl)-2-triisopropylsilanyloxymethyl-piperidin-4-yl]-benzoic acid is used to afford the title compound as a yellow oil. Rf = 0.19 (EtOAc-heptane 1:1); Rt = 6.03 (gradient I).
i) 4-[(2S,4R,5R)-5-[4-(3-Methoxy-propyl)-3-oxo-3,4-dihydro-2H-benzo[1,4]oxazin-6-ylmethoxy]-1-(toluene-4-sulfonyl)-2-triisopropylsilanyloxymethyl-piperidin-4-yl]-benzoic acid
   According to the procedure described in example 118c, 4-[(2S,4R,5R)-5-[4-(3-methoxy-propyl)-3-oxo-3,4-dihydro-2H-benzo[1,4]oxazin-6-ylmethoxy]-1-(toluene-4-sulfonyl)-2-triisopropylsilanyloxymethyl-piperidin-4-yl]-benzoic acid methyl ester is used to afford the title compound as a white foam. Rf = 0.20 (EtOAc-heptane 1:1); Rt = 6.30 (gradient 1).
j) 4-[(2S,4R,5R)-5-[4-(3-Methoxy-propyl)-3-oxo-3,4-dihydro-2H-benzo[1,4]oxazin-6-ylmethoxy]-1-(toluene-4-sulfonyl)-2-triisopropylsilanyloxymethyl-piperidin-4-yl]-benzoic acid methyl ester
   According to general procedure K (with addition of 0.1 equivalents of tetrabutylammonium iodide), 4-[(2S,4R,5R)-5-hydroxy-1-(toluene-4-sulfonyl)-2-triisopropylsilanyloxymethyl-piperidin-4-yl]-benzoic acid methyl ester and 6-bromomethyl-4-(3-methoxy-propyl)-4H-benz[1,4]-oxazin-3-one are used to afford the title compound as a yellow oil. Rf = 0.43 (EtOAc-heptane 1:1); Rt = 6.79 (gradient I).
k) 4-[(2S,4R,5R)-5-Hydroxy-1-(toluene-4-sulfonyl)-2-triisopropylsilanyloxymethyl-piperidin-4-yl]-benzoic acid methyl ester
   To a stirred solution of 20.68 g 4-[(2S,4R,5R)-5-hydroxy-2-hydroxymethyl-1-(toluene-4-sulfonyl)-piperidin-4-yl]-benzoic acid methyl ester in 400 ml of N,N-dimethylformamide is added 10.9 g of imidazole and 6.79 g of triisopropylchlorosilane at room temperature. The reaction mixture is stirred overnight, diluted with 1 N hydrochloric acid and extracted with tert-buty methyl ether (3X). The organic phases are combined, dried over sodium sulfate and concentrated under reduced pressure. The crude product is purified by flash chromatography (SiO₂ 60F) to afford the title compound as a yellow oil. Rf = 0.45 (EtOAc-heptane 1:1); Rt = 6.46 (gradient I).
l) 4-[(2S,4R,5R)-5-Hydroxy-2-hydroxymethyl-1-(toluene-4-sulfonyl)-piperidin-4-yl]-benzoic acid methyl ester
   To a solution of 6 g of trifluoro-methanesulfonic acid 4-[(2S,4R,5R)-5-hydroxy-2-hydroxymethyl-1-(toluene-4-sulfonyl)-piperidin-4-yl]-phenyl ester in 42 ml of N,N-dimethylformamide and 32 ml of methanol is added 3.1 ml of triethylamine, 245 mg of palladium(II) acetate and 454 mg of 1,3-bisdiphenylphosphinopropane. The reaction mixture is charged into an autoclave and subjected to a pressure of carbon monoxide of 70 bars and subsequently heated to 70°C. The reaction mixture is kept for 5 hours under those reaction conditions followed by concentrating the mixture under reduced pressure. The residue is purified by flash chromatography (SiO₂ 60F) to afford the title compound as a dark yellow oil. Rf = 0.38 (EtOAc-heptane 1:1); Rt = 3.75 (gradient I).
m) Trifluoro-methanesulfonic acid 4-[(2S,4R,5R)-5-hydroxy-2-hydroxymethyl-1-(toluene-4-sulfonyl)-piperidin-4-yl]-phenyl ester
   To a solution of 29.39 g of (3R,4R,6S)-6-hydroxymethyl-4-(4-hydroxy-phenyl)-1-(toluene-4-sulfonyl)-piperidin-3-ol in 170 ml of dichloromethane are added 29.23 g of N-phenyltrifluoro-methane sulfonimide and 11.55 ml of triethylamine. The reaction mixture is stirred for 2 hours at room temperature, diluted with sodium bicarbonate solution and extracted with dichloromethane. The organic phases are combined, dried and concentrated under reduced pressure. The residue is purified by flash chromatography (SiO₂ 60F) to afford the title compound white solid. Rf = 0.27 (EtOAc-heptane 1:1); Rt = 4.46 (gradient I).
n) (3R,4R,6S)-6-Hydroxymethyl-4-(4-hydroxy-phenyl)-1-(toluene-4-sulfonyl)-piperidin-3-ol
   According to the procedure described in example 123e, (3R,4R,6S)-6-hydroxymethyl-4-(4-methoxy-phenyl)-1-(toluene-4-sulfonyl)-piperidin-3-ol is used to afford the title compound as a white foam. Rf = 0.20 (EtOAc-heptane 2:1); Rt = 3.22 (gradient I).
o) (3R,4R,6S)-6-Hydroxymethyl-4-(4-methoxy-phenyl)-1-(toluene-4-sulfonyl)-piperidin-3-ol
   To a stirred solution of 108.1 g of [(S)-4-(4-methoxy-phenyl)-1-(toluene-4-sulfonyl)-1,2,3,6-tetrahydro-pyridin-2-yl]-methanol in 1000 ml of tetrahydrofuran are added 504 ml of a solution of borane-tetrahydrofuran (1 M) at room temperature. The reaction mixture is heated to 40°C for 2-6 hours, cooled to room temperature, and quenched by the addition of 150 ml of 2N sodium hydroxide solution and 150 ml of 30% hydrogen peroxide solution. The organic phase is separated, dried over sodium sulfate and concentrated under reduced pressure. The residue is purified by flash chromatography (SiO₂ 60F) to afford the title compound as a white solid. Rf = 0.19 (EtOAc-heptane 1:1); Rt = 3.67 (gradient I).
p) [(S)-4-(4-methoxy-phenyl)-1-(toluene-4-sulfonyl)-1,2,3,6-tetrahydro-pyridin-2-yl]-methanol
   To a solution of 72.0 g of [(S)-4-(4-methoxy-phenyl)-1,2,3,6-tetrahydro-pyridin-2-yl]-methanol in 1.5 I of ethyl acetate and 1.5 I of 2N aqueous sodium carbonate are added 57.7 g of p-toluenesulfonyl chloride at 0°C. The reaction mixture is stirred overnight, extracted with ethyl acetate (3X) and the combined organic phases are dried over sodium sulfate and concentrated under reduced pressure. The crude product is used for the next step without any further purification. Rf = 0.1 (EtOAc-heptane 1:1); Rt = 4.20 (gradient I).
q) [(S)-4-(4-Methoxy-phenyl)-1,2,3,6-tetrahydro-pyridin-2-yl]-methanol
   To a stirred suspension of 10.9 g of lithium aluminium hydride in 1l of tetrahydrofuran is added a solution of 43 g of (S)-4-(4-methoxy-phenyl)-6-oxo-1,2,3,6-tetrahydro-pyridine-2-carboxylic acid benzyl ester in 1l of tetrahydrofuran at 70°C. The reaction mixture is stirred for another 3 - 4 hours at this temperature, cooled to 0°C and quenched by the slow addition of 12.4 ml of water, 12.4 ml of 2N sodium hydroxide and 37 ml of water. The mixture is filtered and the filtrate is concentrated under reduced pressure. The residue is purified by flash chromatography (SiO₂ 60F) to afford the title compound as a white solid. Rf = 0.17 (dichloromethane-methanol-25% ammonia conc. 90:10:1); Rt = 2.55 (gradient I).
r) (S)-4-(4-Methoxy-phenyl)-6-oxo-1,2,3,6-tetrahydro-pyridine-2-carboxylic acid benzyl ester
   To a solution of 1.8 g of (S)-4-(4-methoxy-phenyl)-6-oxo-3,6-dihydro-2H-pyridine-1,2-dicarboxylic acid 2-benzyl ester 1-tert-butyl ester in 15 ml of 1,4 dioxane are added 1.2 ml of 4N hydrochloric acid in 1,4 dioxane at room temperature. The reaction mixture is stirred for 1 hour and concentrated under reduced pressure to afford the title compound as an off-white solid. Rf = 0.1 (EtOAc-heptane 1:1); Rt = 3.94 (gradient I).
s) (S)-4-(4-Methoxy-phenyl)-6-oxo-3,6-dihydro-2H-pyridine-1,2-dicarboxylic acid 2-benzyl ester 1-tert-butyl ester
   To a solution of 2.75 g of 6-oxo-4-(toluene-4-sulfonyloxy)-3,6-dihydro-2H-pyridine-1,2-dicarboxylic acid 2-benzyl ester 1-tert-butyl ester in 18 ml of tetrahydrofuran are added 1.4 g of 4-methoxyphenylboronic acid, 3.15 g of potassium phosphate, 0.12 g of dicyclohexyl[2',4',6'-tris(1-methylethyl)[1,1'-biphenyl]-2-yl]-phosphine (X-PHOS) and 22 mg of palladium acetate under argon. The reaction mixture is heated to 80°C for 2 hours, cooled to room temperature, diluted with ethyl acetate and the organic phase is washed with water and dried over sodium sulfate. The residue is purified by flash chromatography (SiO₂ 60F) to afford the title compound as a brown solid. Rf = 0.35 (EtOAc-heptane 1:2); Rt = 4.95 (gradient I).
t) (S)-6-Oxo-4-(toluene-4-sulfonyloxy)-3,6-dihydro-2H-pyridine-1,2-dicarboxylic acid 2-benzyl ester 1-tert-butyl ester
   According to general procedure M, (2S)-4,6-dioxo-piperidine-1,2-dicarboxylic acid 2-benzyl ester 1-tert-butyl ester [176436-10-5] and p-toluene sulfonyl chloride are used to afford the title compound as a yellow oil. Rf = 0.64 (EtOAc-heptane 1:1); Rt = 5.20 (gradient I).
u) 6-Bromomethyl-4-(3-methoxy-propyl)-4H-benzo[1,4]oxazin-3-one
   To a solution of 74.0 g of 6-hydroxymethyl-4-(3-methoxy-propyl)-4H-benzo[1,4]oxazin-3-one [857272-03-8] in 600 ml of chloroform are added 58 ml of bromotrimethylsilane at room temperature. The reaction mixture is stirred for 30 minutes and concentrated under reduced pressure. The residue is purified by flash chromatography (SiO₂ 60F) to afford the title compound as an off white solid. Rf = 0.46 (EtOAc-heptane 1:1); Rt = 4.03 (gradient I).

### 133 3-{(2R,4R,5R)-1-((S)-2-Amino-3-methyl-butyryl)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-2,2,N-trimethyl-propionamide

starting from 3-{(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-2,2,N-trimethyl-propionamide

The starting materials are prepared as follows:
a) 3-{(2R,4R,5R)-4-[4-(2-Methoxy-ethoxymethyl)-phenyl]-5-[4-(3-methoxy-propyl)-3,4-dihydro-2H-benzo[1,4]oxazin-6-ylmethoxy]-piperidin-2-yl}-2,2,N-trimethyl-propionamide
   According to general procedure A, 3-[(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-2,2,N-trimethyl-propionamide is used to afford the title compound as a yellowish oil. Rf = 0.20 (dichloromethane-methanol 25% ammonia conc. 200:20:1); Rt = 3.77 (gradient I).
b) 3-[(2R,4R,5R)-4-[4-(2-Methoxy-ethoxymethyl)-phenyl]-5-[4-(3-methoxy-propyl)-3,4-dihydro-2H-benzo[1,4]oxazin-6-ylmethoxyl-1-(toluene-4-sulfonyl)-piperidin-2-yl]-2,2,N-trimethyl-propionamide
   According to general procedure D, 3-[(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid and methylamine are used to afford the title compound as a yellow oil. Rf = 0.25 (EtOAc); Rt = 4.75 (gradient I).
c) 3-[(2R,4R,5R)-4-[4-(2-Methoxy-ethoxymethyl)-phenyl]-5-[4-(3-methoxy-propyl)-3,4-dihydro-2H-benzo[1,4]oxazin-6-ylmethoxyl-1-(toluene-4-sulfonyl)-piperidin-2-yl]-2,2-dimethyl-propionic acid
   According to example 65b, 3-[(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid methyl ester is used to afford the crude title compound.
d) 3-[(2R,4R,5R)-4-[4-(2-Methoxy-ethoxymethyl)-phenyl]-5-[4-(3-methoxv-propyl)-3,4-dihydro-2H-benzo[1,4]oxazin-6-ylmethoxyl-1-(toluene-4-sulfonyl)-piperidin-2-yl]-2,2-dimethyl-propionic acid methyl ester
   To a stirred solution of 0.157 mmol of lithium diisopropylamide (0.5 M in tetrahydrofuran) is added at -78°C 0.150 mmol of 3-[(2S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-propionic acid methyl ester (dissolved in 0.3 ml of tetrahydrofuran). After stirring for 30 minutes 0.11 mmol of methyl iodide is added at -78°C and the reaction mixture is allowed to warm to room temperature. To this solution is then added again to a solution of 0.157 mmol lithium diisopropylamide (0.5 M in THF) at -78°C and the solution is stirred for additional 30 minutes. 0.11 mmol of methyl iodide is added at -78°C and the reaction mixture is allowed to warm to room temperature. The reaction mixture is hydrolyzed with 0.5M HCl and extracted three times with tert-butyl methyl ether. The combined organic layers are washed with brine, dried over sodium sulfate and the solvent is concentrated under reduced pressure. The residue is purified by flash chromatography (SiO₂ 60F) to afford the title compound as a yellow oil. Rf = 0.31 (EtOAc-heptane 3:1); Rt = 5.41 (gradient I).
e) 3-[(2S,4R,5R)-4-[4-(2-Methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-propionic acid methyl ester
   4 ml of a solution of diazomethane in ether (0.2M) are added dropwise to a solution of 0.13 g of 3-[(2S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-propionic acid in 2 ml of methanol at 0°C. The reaction mixture is worked up after 3.5 hours by quenching with solid magnesium sulfate to decompose any excess diazomethane, filtering and concentrating under reduced pressure to afford the crude title compound as a yellow oil. Rf = 0.50 (EtOAc-heptane 4:1); Rt = 5.12 (gradient I).
f) 3-[(2S,4R,5R)-4-[4-(2-Methoxy-ethoxymethyl)-phenyl]-5-[4-(3-methoxy-propyl)-3,4-dihydro-2H-benzo[1,4]oxazin-6-ylmethoxyl-1-(toluene-4-sulfonyl)-piperidin-2-yl]-propionic acid
   According to the procedure described in example 109f and 109e, methanesulfonic acid 2-[(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-ethyl ester is used to afford the title compound as a dark yellow oil. Rf = 0.10 (dichlorometane-methanol-25% ammonia conc. 200:15:1); Rt = 4.66 (gradient I).
g) Methanesulfonic acid 2-[(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-Piperidin-2-yl]-ethyl ester
   According to general procedure F, 2-[(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-ethanol is used to afford the title compound as a dark yellow oil. Rf = 0.35 (EtOAc-heptane 3:1); Rt = 4.96 (gradient I).
h) 2-[(2R,4R,5R)-4-[4-(2-Methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-ethanol
   According to general procedure C, [(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-acetic acid is used to afford the title compound as a dark yellow oil. Rf = 0.14 (EtOAc-heptane 2:1); Rt = 4.66 (gradient I).
i) [(2R,4R,5R)-4-[4-(2-Methoxy-ethoxymethyl)-phenyl]-5-[4-(3-methoxy-propyl)-3,4-dihydro-2H-benzo[1,4]oxazin-6-ylmethoxyl-1-(toluene-4-sulfonyl)-piperidin-2-yl]-acetic acid
   According to the procedure described in example 109f and 109e, methanesulfonic acid (2S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-ylmethyl ester (example 128e) is used to afford the title compound as a brown oil. Rf = 0.42 (EtOAc-heptane 1:1);
   Rt = 4.59 (gradient I).

### 138 3-{(2R,4R,5R)-1-((S)-2-Amino-3-methyl-butyryl)-4-(4-methoxy-phenyl)-5-[4-(3-methoxy-propyl)-3,4-dihydro-2H-benzo[1,4]oxazin-6-ylmethoxy]-piperidin-2-yl}-2,2,N-trimethyl-propionamide

starting from 3-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2,2,N-trimethyl-propionamide

The starting materials are prepared as follows:
a) 3-{(2R,4R,5R)-4-(4-Methoxy-phenyl)-5-[4-(3-methoxy-propyl)-3,4-dihydro-2H-benzo[1,4]oxazin-6-ylmethoxy]-piperidin-2-yl}-2,2,N-trimethyl-propionamide
   According to the procedure described in example 133a-133c, 3-[(2R,4R,5R)-4-(4-methoxyphenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid methyl ester is used to afford the title compound, which is identified based on its Rf value.
b) 3-[(2R,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid methyl ester
   According to the procedure described in example 133d-133h, [(2R,4R,5R)-4-(4-methoxyphenyl)-5-[4-(3-methoxy-propyl)-3,4-dihydro-2H-benzo[1,4]oxazin-6-ylmethoxy]-1-(toluene-4-sulfonyl)-piperidin-2-yl]-acetic acid (example 109e) is used to afford the title compound, which is identified based on its Rf value.

### 143 N-(2-{(2R,4R,5R)-1-((S)-2-Amino-3-methyl-butyryl)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-1,1-dimethyl-ethyl)-acetamide

starting from N-(2-{(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-1,1-dimethyl-ethyl)-acetamide.

The starting materials are prepared as follows:
a) N-(2-{(2R,4R,5R)-4-[4-(2-Methoxy-ethoxymethyl)-phenyl]-5-[4-(3-methoxy-propyl)-3,4-dihydro-2H-benzo[1,4]oxazin-6-ylmethoxy]-piperidin-2-yl}-1,1-dimethyl-ethyl)-acetamide
   According to general procedure A, N-{2-[(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-1,1-dimethyl-ethyl}-acetamide is used to afford the title compound as a yellowish oil. Rf = 0.28 (dichloromethane-methanol-25% ammonia conc. 200:20:1); Rt = 3.66 (gradient I).
b) N-{2-[(2R,4R,5R)-4-[4-(2-Methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-1,1-dimethyl-ethyl}-acetamide
   A solution of 0.394 mmol of 2-[(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-1,1-dimethyl-ethylamine in 4 ml of dichloromethane at 0°C is treated with 3.15 mmol of triethylamine and 1.732 mmol of acetyl chloride. The mixture is stirred for 4.5 hours at 0°C, then poured on water. The phases are separated and the aqueous phase is extracted with dichloromethane (2X). The combined organic phases are washed with brine, dried over sodium sulfate and concentrated under reduced pressure. The residue is purified by flash chromatography (SiO₂ 60F) to afford the title compound as a yellowish oil. Rf = 0.21 (EtOAc); Rt = 4.80 (gradient I).
c) 2-[(2R,4R,5R)-4-[4-(2-Methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-1,1-dimethyl-ethylamine
   According to general procedure E (using methanol-tetrahydrofuran 10:1 as solvent), {2-[(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-1,1-dimethyl-ethyl}-carbamic acid benzyl ester is used to afford the crude title compound as a yellowish oil. Rf = 0.41 (dichloromethane-methanol-25% ammonia conc. 200:20:1); Rt = 4.32 (gradient I).
d) {2-[(2R,4R,5R)-4-[4-(2-Methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-1,1-dimethyl-ethyl}-carbamic acid benzyl ester
   A solution of 0.57 mmol of 6-[(3R,4R,6R)-6-(2-isocyanato-2-methyl-propyl)-4-[4-(2-methoxyethoxymethyl)-phenyl]-1-(toluene-4-sulfonyl)-piperidin-3-yloxymethyl]-4-(3-methoxy-propyl)-3,4-dihydro-2H-benzo[1,4]oxazine in 28.8 mmol of benzyl alcohol is stirred for 15 hours at 115°C. The reaction mixture is concentrated under reduced pressure and the residue is purified by flash chromatography (SiO₂ 60F) to afford the title compound as a yellow oil.
   Rf = 0.32 (EtOAc-heptane 2:1); Rt = 5.59 (gradient I).
e) 6-[(3R,4R,6R)-6-(2-Isocyanato-2-methyl-propyl)-4-f4-(2-methoxy-ethoxymethyl)-phenyl]-1-(toluene-4-sulfonyl)-piperidin-3-yloxymethyl]-4-(3-methoxy-propyl)-3,4-dihydro-2H-benzo[1,4]oxazine
   A solution of 0.668 mmol of 3-[(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionyl azide in 6 ml of toluene is stirred for 1.5 hours at 115°C. The reaction mixture is concentrated under reduced pressure to afford the crude title compound as a brown oil. Rf = 0.29 (EtOAc-heptane 2:1); Rt = 5.49 (gradient I).
f) 3-[(2R,4R,5R)-4-[4-(2-Methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionyl azide
   A solution of 0.623 mmol of 3-[(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid (example 133c) in 6 ml of tetrahydrofuran is treated with 1.558 mmol of triethylamine, cooled to 0°C and treated with 1.246 mmol of ethyl chloroformate. The reaction mixture is stirred for 1 hour at 0°C and then treated with a solution of 12.460 mmol of sodium azide in 3 ml of water. The reaction mixture is stirred for 1 hour at 0°C and is then diluted with ethyl acetate and quenched with small amounts of water. The organic phase is separated, washed with water (2X), dried over sodium sulfate and concentrated under reduced pressure to afford the crude title compound as a brownish oil. Rf = 0.27 (EtOAc-heptane 2:1); Rt = 5.53 (gradient I).

### 148 N-(2-{(2R,4R,5R)-1-((S)-2-Amino-3-methyl-butyryl)-4-(4-methoxy-phenyl)-5-[4-(3-methoxy-propyl)-3,4-dihydro-2H-benzo[1,4]oxazin-6-ylmethoxy]-piperidin-2-yl}-1,1-dimethyl-ethyl)-acetamide

starting from N-(2-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-1,1-dimethyl-ethyl)-acetamide

The starting material is prepared as follows:
a) N-(2-{(2R,4R,5R)-4-(4-Methoxy-phenyl)-5-[4-(3-methoxy-propyl)-3,4-dihydro-2H-benzo[1,4]oxazin-6-ylmethoxy]-piperidin-2-yl}-1,1-dimethyl-ethyl)-acetamide
   According to the procedure described in example 143a-143f, 3-[(2R,4R,5R)-4-(4-methoxyphenyl)-5-[4-(3-methoxy-propyl)-3,4-dihydro-2H-benzo[1,4]oxazin-6-ylmethoxy]-1-(toluene-4-sulfonyl)-piperidin-2-yl]-2,2-dimethyl-propionic acid methyl ester (example 138b) is used to afford the title compound, which is identified based on its Rf value.

### Example 6

### (S)-2-Amino-1-{(3S,4S,5R)-3-hydroxy-4-(4-methoxy-phenyl)-5-[4-(3-methoxy-propyl)-3,4-dihydro-2H-benzo[1,4]oxazin-6-ylmethoxy]-piperidin-1-yl}-4-methyl-pentan-1-one

According to the process described in example 5 and 5a, using (S)-2-benzyloxycarbonylamino-4-methyl-pentanoic acid [2018-66-8] in step a, the title compound is obtained starting from (3S,4S,5R)-4-(4-methoxy-phenyl)-5-[4-(3-methoxy-propyl)-3,4-dihydro-2H-benzo[1,4]oxazin-6-ylmethoxy]-piperidin-3-ol [873945-55-2], and is identified based on its Rf value.

According to the procedure described in example 6, the following compounds are prepared in analogous manner:
15 (S)-2-Amino-1-{(3S,4S,5R)-3-hydroxy-4-{4-[3-(2-methoxy-benzyloxy)-propoxy]-phenyl}-5-[1-(3-methoxy-propyl)-3,3-dimethyl-2,3-dihydro-1H-indol-6-ylmethoxy]-piperidin-1-yl}-4-methyl-pentan-1-one
   starting from (3S,4S,5R)-4-{4-[3-(2-methoxy-benzyloxy)-propoxy]-phenyl}-5-[1-(3-methoxy-propyl)-3,3-dimethyl-2,3-dihydro-1H-indol-6-ylmethoxy]-piperidin-3-ol (example 12a).
24 (S)-2-Amino-1-{(3S,4S,5R)-3-hydroxy-4-{4-[3-(2-methoxy-benzyloxy)-propoxy]-phenyl}-5-[4-methoxy-3-(3-methoxy-propoxy)-benzyloxy]-piperidin-1-yl}-4-methyl-pentan-1-one
   starting from (3S,4S,5R)-4-{4-[3-(2-methoxy-benzyloxy)-propoxy]-phenyl}-5-[4-methoxy-3-(3-methoxy-propoxy)-benzyloxy]-piperidin-3-ol (example 21 a).
33 (S)-2-Amino-1-{(3S,4S,5R)-4-{4-[3-(2-chloro-phenoxy)-propoxy]-phenyl}-3-hydroxy-5-[4-(3-methoxy-propyl)-3,4-dihydro-2H-benzo[1,4]oxazin-6-ylmethoxy]-piperidin-1-yl}-4-methyl-pentan-1-one
   starting from (3S,4S,5R)-4-{4-[3-(2-chloro-phenoxy)-propoxy]-phenyl}-5-[4-(3-methoxy-propyl)-3,4-dihydro-2H-benzo[1,4]oxazin-6-ylmethoxy]-piperidin-3-ol [873945-66-5].
42 (S)-2-Amino-1-{(3S,4S,5R)-3-hydroxy-4-{4-[3-(2-methoxy-benzyloxy)-propoxy]-phenyl}-5-[4-(3-methoxy-propyl)-3,4-dihydro-2H-benzo[1,4]oxazin-6-ylmethoxy]-piperidin-1-yl}-4-methyl-pentan-1-one
   starting from (3S,4S,5R)-4-{4-[3-(2-methoxy-benzyloxy)-propoxy]-phenyl}-5-[4-(3-methoxy-propyl)-3,4-dihydro-2H-benzo[1,4]oxazin-6-ylmethoxy]-piperidin-3-ol [873945-50-7].
51 (S)-2-Amino-1-{(3S,4S,5R)-4-[4-(4-ethyl-phenoxy)-phenyl]-3-hydroxy-5-[4-(3-methoxy-propyl)-3,4-dihydro-2H-benzo[1,4]oxazin-6-ylmethoxy]-piperidin-1-yl}-4-methyl-pentan-1-one
   starting from (3S,4S,5R)-4-[4-(4-ethyl-phenoxy)-phenyl]-5-[4-(3-methoxy-propyl)-3,4-dihydro-2H-benzo[1,4]oxazin-6-ylmethoxy]-piperidin-3-ol (example 48a).
60 N-(2-{2-[2-((3R,4S,5S)-1-((S)-2-Amino-4-methyl-pentanoyl)-5-hydroxy-4-{4-[3-(2-methoxy-benzyloxy)-propoxyl-phenyl}-piperidin-3-yloxy)-ethoxyl-phenyl}-ethyl)-acetamide
   starting from (N-(2-{2-[2-((3R,4S,5S)-5-hydroxy-4-{4-[3-(2-methoxy-benzyloxy)-propoxy]-phenyl}-piperidin-3-yloxy)-ethoxy]-phenyl}-ethyl)-acetamide (example 57a).
69 (S)-2-Amino-1-{(3S,4S,5R)-4-{4-[(S)-1-(3-fluoro-phenyl)-pyrrolidin-3-yloxy]-phenyl}-3-hydroxy-5-[4-(3-methoxy-propyl)-3,4-dihydro-2H-benzo[1,4]oxazin-6-ylmethoxyl-piperidin-1-yl}-4-methyl-pentan-1-one
   starting from (3S,4S,5R)-4-{4-[(S)-1-(3-fluoro-phenyl)-pyrrolidin-3-yloxy]-phenyl}-5-[4-(3-methoxy-propyl)-3,4-dihydro-2H-benzo[1,4]oxazin-6-ylmethoxy]-piperidin-3-ol [873945-18-7].
78 (S)-2-Amino-1-{(3S,4R,5R)-3-((R)-2-hydroxy-propoxy)-4-[4-(2-methoxyethoxymethyl)-phenyl]-5-[4-(3-methoxy-propyl)-3,4-dihydro-2H-benzo[1,4]oxazin-6-ylmethoxyl-piperidin-1-yl}-4-methyl-pentan-1-one
   starting from (R)-1-{(3S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-3-yloxy}-propan-2-ol (example 73a).
87 (S)-2-Amino-1-{(3S,4R,5R)-3-((R)-2-hydroxy-3-methoxy-propoxy)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-1-yl}-4-methyl-petan-1-one
   starting from (R)-1-methoxy-3-{(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-yloxy}-propan-2-ol (example 82a).
96 (S)-2-Amino-1-{(3S,4R,5R)-3-((R)-2-hydroxy-propoxy)-4-[4-(4-methoxy-butoxy)-phenyl]-5-[4-(3-methoxy-propyl)-3,4-dihydro-2H-benzo[1,4]oxazin-6-ylmethoxyl-piperidin-1-yl}-4-methyl-pentan-1-one
   starting from (R)-1-{(3S,4R,5R)-4-[4-(4-methoxy-butoxy)-phenyl]-5-[4-(3-methoxy-propyl)-3,4-dihydro-2H-benzo[1,4]oxazin-6-ylmethoxy]-piperidin-3-yloxy}-propan-2-ol (example 91 a).
105 (S)-2-Amino-1-{(3S,4R,5R)-3-hydroxymethyl-4-[4-((S)-3-methoxy-2-methyl-propoxymethyl)-phenyl]-5-[4-(3-methoxy-propyl)-3,4-dihydro-2H-benzo[1,4]oxazin-6-ylmethoxyl-piperidin-1-yl}-4-methyl-pentan-1-one
   starting from {(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-yl}-methanol (example 102a).
114 (S)-2-Amino-1-{(2R,4R,5R)-2-(2-hydroxy-3-methoxy-2-methyl-propyl)-4-(4-methoxyphenyl)-5-[4-(3-methoxy-propyl)-3,4-dihydro-2H-benzo[1,4]oxazin-6-ylmethoxyl-piperidin-1-yl}-4-methyl-pentan-1-one (diastereomeric mixture)
   starting from (R,S)-1-methoxy-3-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2-methyl-propan-2-ol (diastereomeric mixture) (example 109a).
119 3-{(2S,4R,5R)-1-((S)-2-Amino-4-methyl-pentanoyl)-4-(4-methoxy-phenyl)-5-[4-(3-methoxy-propyl)-3,4-dihydro-2H-benzo[1,4]oxazin-6-ylmethoxyl-piperidin-2-yl}-2,2,N-trimethyl-propionamide
   starting from 3-{(2S,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2,2,N-trimethyl-propionamide (example 118a).
124 3-{(2S,4R,5R)-1-((S)-2-Amino-4-methyl-pentanoyl)-4-[4-(3-methoxy-propoxy)-phenyl]-5-[4-(3-methoxy-propyl)-3,4-dihydro-2H-benzo[1,4]oxazin-6-ylmethoxyl-piperidin-2-yl}-2,2,N-trimethyl-propionamide
   starting from 3-{(2S,4R,5R)-4-[4-(3-methoxy-propoxy)-phenyl]-5-[4-(3-methoxy-propyl)-3,4-dihydro-2H-benzo[1,4]oxazin-6-ylmethoxy]-piperidin-2-yl}-2,2,N-trimethyl-propionamide (example 123a).
129 3-{(2S,4R,5R)-1-((S)-2-Amino-4-methyl-pentanoyl)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-2,2,N-trimethyl-propionamide
   starting from 3-{(2S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-2,2,N-trimethyl-propionamide (example 128a).
134 3-{(2R,4R,5R)-1-((S)-2-Amino-4-methyl-pentanoyl)-4-[4-(2-methoxy-ethoxymethyl)-Phenyl]-5-[4-(3-methoxy-propyl)-3,4-dihydro-2H-benzo[1,4]oxazin-6-ylmethoxy]-piperidin-2-yl}-2,2,N-trimethyl-propionamide
   starting from 3-{(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-2,2,N-trimethyl-propionamide (example 133a).
139 3-{(2R,4R,5R)-1-((S)-2-Amino-4-methyl-pentanoyl)-4-(4-methoxy-phenyl)-5-[4-(3-methoxy-propyl)-3,4-dihydro-2H-benzo[1,4]oxazin-6-ylmethoxyl-piperidin-2-yl}-2,2,N-trimethyl-propionamide
   starting from 3-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2,2,N-trimethyl-propionamide (example 138a).
144 N-(2-{(2R,4R,5R)-1-((S)-2-Amino-4-methyl-pentanoyl)-4-[4-(2-methoxyethoxymethyl)-phenyl]-5-[4-(3-methoxy-propyl)-3,4-dihydro-2H-benzo[1,4]oxazin-6-ylmethoxyl-piperidin-2-yl}-1,1-dimethyl-ethyl)-acetamide
   starting from N-(2-{(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-1,1-dimethyl-ethyl)-acetamide (example 143a).
149 N-(2-{(2R,4R,5R)-1-((S)-2-Amino-4-methyl-pentanoyl)-4-(4-methoxy-phenyl)-5-[4-(3-methoxy-propyl)-3,4-dihydro-2H-benzo[1,4]oxazin-6-ylmethoxyl-piperidin-2-yl}-1,1-dimethyl-ethyl)-acetamide
   starting from N-(2-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-1,1-dimethyl-ethyl)-acetamide (example 148a).

### Example 7

### (S)-2-Amino-1-{(3S,4S,5R)-3-hydroxy-4-(4-methoxy-phenyl)-5-[4-(3-methoxy-propyl)-3,4-dihydro-2H-benzo[1,4]oxazin-6-ylmethoxy]-piperidin-1-yl}-propan-1-one

According to the process described in example 5 and 5a, using (S)-2-benzyloxycarbonylamino-propionic acid [1142-20-7] in step a, the title compound is obtained starting from (3S,4S,5R)-4-(4-methoxy-phenyl)-5-[4-(3-methoxy-propyl)-3,4-dihydro-2H-benzo[1,4]oxazin-6-ylmethoxy]-piperidin-3-ol [873945-55-2], and is identified based on its Rf value.

According to the procedure described in example 7, the following compounds are prepared in analogous manner:
16 (S)-2-Amino-1-{(3S,4S,5R)-3-hydroxy-4-{4-[3-(2-methoxy-benzyloxy)-propoxy]-phenyl}-5-[1-(3-methoxy-propyl)-3,3-dimethyl-2,3-dihydro-1H-indol-6-ylmethoxyl-piperidin-1-yl}-propan-1-one
   starting from (3S,4S,5R)-4-{4-[3-(2-methoxy-benzyloxy)-propoxy]-phenyl}-5-[1-(3-methoxy-propyl)-3,3-dimethyl-2,3-dihydro-1H-indol-6-ylmethoxy]-piperidin-3-ol (example 12a).
25 (S)-2-Amino-1-{(3S,4S,5R)-3-hydroxy-4-{4-[3-(2-methoxy-benzyloxy)-propoxy]-phenyl}-5-[4-methoxy-3-(3-methoxy-propoxy)-benzyloxyl-piperidin-1-yl}-propan-1-one
   starting from (3S,4S,5R)-4-{4-[3-(2-methoxy-benzyloxy)-propoxy]-phenyl}-5-[4-methoxy-3-(3-methoxy-propoxy)-benzyloxy]-piperidin-3-ol (example 21 a).
34 (S)-2-Amino-1-{(3S,4S,5R)-4-{4-[3-(2-chloro-phenoxy)-propoxyl-phenyl}-3-hydroxy-5-[4-(3-methoxy-propyl)-3,4-dihydro-2H-benzo[1,4]oxazin-6-ylmethoxyl-piperidin-1-yl}-propan-1-one
   starting from (3S,4S,5R)-4-{4-[3-(2-chloro-phenoxy)-propoxy]-phenyl}-5-[4-(3-methoxy-propyl)-3,4-dihydro-2H-benzo[1,4]oxazin-6-ylmethoxy]-piperidin-3-ol [873945-66-5].
43 (S)-2-Amino-1-{(3S,4S,5R)-3-hydroxy-4-{4-[3-(2-methoxy-benzyloxy)-propoxy]-phenyl}-5-[4-(3-methoxy-propyl)-3,4-dihydro-2H-benzo[1,4]oxazin-6-ylmethoxyl-piperidin-1-yl}-propan-1-one
   starting from (3S,4S,5R)-4-{4-[3-(2-methoxy-benzyloxy)-propoxy]-phenyl}-5-[4-(3-methoxy-propyl)-3,4-dihydro-2H-benzo[1,4]oxazin-6-ylmethoxy]-piperidin-3-ol [873945-50-7].
52 (S)-2-Amino-1-{(3S,4S,5R)-4-[4-(4-ethyl-phenoxy)-phenyl]-3-hydroxy-5-[4-(3-methoxy-propyl)-3,4-dihydro-2H-benzo[1,4]oxazin-6-ylmethoxy]-piperidin-1-yl}-propan-1-one
   starting from (3S,4S,5R)-4-[4-(4-ethyl-phenoxy)-phenyl]-5-[4-(3-methoxy-propyl)-3,4-dihydro-2H-benzo[1,4]oxazin-6-ylmethoxy]-piperidin-3-ol (example 48a).
61 N-(2-{2-[2-((3R,4S,5S)-1-((S)-2-Amino-propionyl)-5-hydroxy-4-{4-[3-(2-methoxy-benzyloxy)-propoxy]-phenyl}-piperidin-3-yloxy)-ethoxy]-phenyl}-ethyl)-acetamide
   starting from (N-(2-{2-[2-((3R,4S,5S)-5-hydroxy-4-{4-[3-(2-methoxy-benzyloxy)-propoxy]-phenyl}-piperidin-3-yloxy)-ethoxy]-phenyl}-ethyl)-acetamide (example 57a).
70 (S)-2-Amino-1-{(3S,4S,5R)-4-{4-[(S)-1-(3-fluoro-phenyl)-pyrrolidin-3-yloxy]-phenyl}-3-hydroxy-5-[4-(3-methoxy-propyl)-3,4-dihydro-2H-benzo[1,4]oxazin-6-ylmethoxy]-piperidin-1-yl}-propan-1-one
   starting from (3S,4S,5R)-4-{4-[(S)-1-(3-fluoro-phenyl)-pyrrolidin-3-yloxy]-phenyl}-5-[4-(3-methoxy-propyl)-3,4-dihydro-2H-benzo[1,4]oxazin-6-ylmethoxy]-piperidin-3-ol [873945-18-7].
79 (S)-2-Amino-1-{(3S,4R,5R)-3-((R)-2-hydroxy-propoxy)-4-[4-(2-methoxyethoxymethyl)-phenyl]-5-[4-(3-methoxy-propyl)-3,4-dihydro-2H-benzo[1,4]oxazin-6-ylmethoxy]-piperidin-1-yl}-propan-1-one
   starting from (R)-1-{(3S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-3-yloxy}-propan-2-ol (example 73a).
88 (S)-2-Amino-1-{(3S,4R,5R)-3-((R)-2-hydroxy-3-methoxy-propoxy)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxyl-piperidin-1-yl}-propan-1-one
   starting from (R)-1-methoxy-3-{(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-yloxy}-propan-2-ol (example 82a).
97 (S)-2-Amino-1-{(3S,4R,5R)-3-((R)-2-hydroxy-propoxy)-4-[4-(4-methoxy-butoxy)-phenyl]-5-[4-(3-methoxy-propyl)-3,4-dihydro-2H-benzo[1,4]oxazin-6-ylmethoxy]-piperidin-1-yl}-propan-1-one
   starting from (R)-1-{(3S,4R,5R)-4-[4-(4-methoxy-butoxy)-phenyl]-5-[4-(3-methoxy-propyl)-3,4-dihydro-2H-benzo[1,4]oxazin-6-ylmethoxy]-piperidin-3-yloxy}-propan-2-ol (example 91 a).
106 (S)-2-Amino-1-{(3S,4R,5R)-3-hydroxymethyl-4-[4-((S)-3-methoxy-2-methyl-propoxymethyl)-phenyl]-5-[4-(3-methoxy-propyl)-3,4-dihydro-2H-benzo[1,4]oxazin-6-ylmethoxyl-piperidin-1-yl}-propan-1-one
   starting from {(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-yl}-methanol (example 102a).
115 (S)-2-Amino-1-{(2R,4R,5R)-2-(2-hydroxy-3-methoxy-2-methyl-propyl)-4-(4-methoxyPhenyl)-5-[4-(3-methoxy-propyl)-3,4-dihydro-2H-benzo[1,4]oxazin-6-ylmethoxyl-piperidin-1-yl}-propan-1-one (diastereomeric mixture)
   starting from (R,S)-1-methoxy-3-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2-methyl-propan-2-ol (diastereomeric mixture) (example 109a).
120 3-{(2S,4R,5R)-1-((S)-2-Amino-propionyl)-4-(4-methoxy-phenyl)-5-[4-(3-methoxy-propyl)-3,4-dihydro-2H-benzo[1,4]oxazin-6-ylmethoxy]-piperidin-2-yl}-2,2,N-trimethyl-propionamide
   starting from 3-{(2S,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2,2,N-trimethyl-propionamide (example 118a).
125 3-{(2S,4R,5R)-1-((S)-2-Amino-propionyl)-4-[4-(3-methoxy-propoxy)-phenyl]-5-[4-(3-methoxy-propyl)-3,4-dihydro-2H-benzo[1,4]oxazin-6-ylmethoxy]-piperidin-2-yl}-2,2,N-trimethyl-propionamide
   starting from 3-{(2S,4R,5R)-4-[4-(3-methoxy-propoxy)-phenyl]-5-[4-(3-methoxy-propyl)-3,4-dihydro-2H-benzo[1,4]oxazin-6-ylmethoxy]-piperidin-2-yl}-2,2,N-trimethyl-propionamide (example 123a).
130 3-{(2S,4R,5R)-1-((S)-2-Amino-propionyl)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-2,2,N-trimethyl-propionamide
   starting from 3-{(2S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-2,2,N-trimethyl-propionamide (example 128a).
135 3-{(2R,4R,5R)-1-((S)-2-Amino-propionyl)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-2,2,N-trimethyl-propionamide
   starting from 3-{(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-2,2,N-trimethyl-propionamide (example 133a).
140 3-{(2R,4R,5R)-1-((S)-2-Amino-propionyl)-4-(4-methoxy-phenyl)-5-[4-(3-methoxy-propyl)-3,4-dihydro-2H-benzo[1,4]oxazin-6-ylmethoxy]-piperidin-2-yl}-2,2,N-trimethyl-propionamide
   starting from 3-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2,2,N-trimethyl-propionamide (example 138a).
145 N-(2-{(2R,4R,5R)-1-((S)-2-Amino-propionyl)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-1,1-dimethyl-ethyl)-acetamide
   starting from N-(2-{(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-1,1-dimethyl-ethyl)-acetamide (example 143a).
150 N-(2-{(2R,4R,5R)-1-((S)-2-Amino-propionyl)-4-(4-methoxy-phenyl)-5-[4-(3-methoxy-propyl)-3,4-dihydro-2H-benzo[1,4]oxazin-6-ylmethoxy]-piperidin-2-yl}-1,1-dimethylethyl)-acetamide
   starting from N-(2-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-1,1-dimethyl-ethyl)-acetamide (example 148a).

### Example 8

### (2S,3S)-2-Amino-1-{(3S,4S,5R)-3-hydroxy-4-(4-methoxy-phenyl)-5-[4-(3-methoxy-propyl)-3,4-dihydro-2H-benzo[1,4]oxazin-6-ylmethoxy]-piperidin-1-yl}-3-methyl-pentan-1-one

According to the process described in example 5 and 5a, using (2S,3S)-2-benzyloxycarbonylamino-3-methyl-pentanoic acid [3160-59-6] in step a, the title compound is obtained starting from (3S,4S,5R)-4-(4-methoxy-phenyl)-5-[4-(3-methoxy-propyl)-3,4-dihydro-2H-benzo[1,4]oxazin-6-ylmethoxy]-piperidin-3-ol [873945-55-2], and is identified based on its Rf value.

According to the procedure described in example 8, the following compounds are prepared in analogous manner:
17 (2S,3S)-2-Amino-1-{(3S,4S,5R)-3-hydroxy-4-{4-[3-(2-methoxy-benzyloxy)-propoxy]-phenyl}-5-[1-(3-methoxy-propyl)-3,3-dimethyl-2,3-dihydro-1H-indol-6-ylmethoxy]-piperidin-1-yl}-3-methyl-pentan-1-one
   starting from (3S,4S,5R)-4-{4-[3-(2-methoxy-benzyloxy)-propoxy]-phenyl}-5-[1-(3-methoxy-propyl)-3,3-dimethyl-2,3-dihydro-1H-indol-6-ylmethoxy]-piperidin-3-ol (example 12a).
26 (2S,3S)-2-Amino-1-{(3S,4S,5R)-3-hydroxy-4-{4-[3-(2-methoxy-benzyloxy)-propoxy]-phenyl)-5-[4-methoxy-3-(3-methoxy-propoxy)-benzyloxy]-piperidin-1-yl}-3-methyl-pentan-1-one
   starting from (3S,4S,5R)-4-{4-[3-(2-methoxy-benzyloxy)-propoxy]-phenyl}-5-[4-methoxy-3-(3-methoxy-propoxy)-benzyloxy]-piperidin-3-ol (example 21 a).
35 (2S,3S)-2-Amino-1-{(3S,4S,5R)-4-{4-[3-(2-chloro-phenoxy)-propoxy]-phenyl}-3-hydroxy-5-[4-(3-methoxy-propyl)-3,4-dihydro-2H-benzo[1,4]oxazin-6-ylmethoxy]-piperidin-1-yl}-3-methyl-pentan-1-one
   starting from (3S,4S,5R)-4-{4-[3-(2-chloro-phenoxy)-propoxy]-phenyl}-5-[4-(3-methoxy-propyl)-3,4-dihydro-2H-benzo[1,4]oxazin-6-ylmethoxy]-piperidin-3-ol [873945-66-5].
44 (2S,3S)-2-Amino-1-{(3S,4S,5R)-3-hydroxy-4-{4-[3-(2-methoxy-benzyloxy)-propoxy]-phenyl}-5-[4-(3-methoxy-propyl)-3,4-dihydro-2H-benzo[1,4]oxazin-6-ylmethoxy]-piperid in-1-yl}-3-methyl-pentan-1-one
   starting from (3S,4S,5R)-4-{4-[3-(2-methoxy-benzyloxy)-propoxy]-phenyl}-5-[4-(3-methoxy-propyl)-3,4-dihydro-2H-benzo[1,4]oxazin-6-ylmethoxy]-piperidin-3-ol [873945-50-7].
53 (2S,3S)-2-Amino-1-{(3S,4S,5R)-4-[4-(4-ethyl-phenoxy)-phenyl]-3-hydroxy-5-[4-(3-methoxy-propyl)-3,4-dihydro-2H-benzo[1,4]oxazin-6-ylmethoxy]-piperidin-1-yl}-3-methyl-pentan-1-one
   starting from (3S,4S,5R)-4-[4-(4-ethyl-phenoxy)-phenyl]-5-[4-(3-methoxy-propyl)-3,4-dihydro-2H-benzo[1,4]oxazin-6-ylmethoxy]-piperidin-3-ol (example 48a).
62 N-(2-{2-[2-((3R,4S,5S)-1-((2S,3S)-2-Amino-3-methyl-pentanoyl)-5-hydroxy-4-{4-[3-(2-methoxy-benzyloxy)-propoxy]-phenyl}-piperidin-3-yloxy)-ethoxy]-phenyl}-ethyl)-acetamide
   starting from (N-(2-{2-[2-((3R,4S,5S)-5-hydroxy-4-{4-[3-(2-methoxy-benzyloxy)-propoxy]-phenyl}-piperidin-3-yloxy)-ethoxy]-phenyl}-ethyl)-acetamide (example 57a).
71 (2S,3S)-2-Amino-1-{(3S,4S,5R)-4-{4-[(S)-1-(3-fluoro-phenyl)-pyrrolidin-3-yloxy]-phenyl}-3-hydroxy-5-[4-(3-methoxy-propyl)-3,4-dihydro-2H-benzo[1,4]oxazin-6-ylmethoxy]-piperidin-1-yl}-3-methyl-pentan-1-one
   starting from (3S,4S,5R)-4-{4-[(S)-1-(3-fluoro-phenyl)-pyrrolidin-3-yloxy]-phenyl}-5-[4-(3-methoxy-propyl)-3,4-dihydro-2H-benzo[1,4]oxazin-6-ylmethoxy]-piperidin-3-ol [873945-18-7].
80 (2S,3S)-2-Amino-1-{(3S,4R,5R)-3-((R)-2-hydroxy-propoxy)-4-[4-(2-methoxyethoxymethyl)-phenyl]-5-[4-(3-methoxy-propyl)-3,4-dihydro-2H-benzo[1,4]oxazin-6-ylmethoxy]-piperidin-1-yl}-3-methyl-pentan-1-one
   starting from (R)-1-{(3S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-3-yloxy}-propan-2-ol (example 73a).
89 (2S,3S)-2-Amino-1-{(3S,4R,5R)-3-((R)-2-hydroxy-3-methoxy-propoxy)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-1-yl}-3-methyl-pentan-1-one
   starting from (R)-1-methoxy-3-{(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-yloxy}-propan-2-ol (example 82a).
98 (2S,3S)-2-Amino-1-{(3S,4R,5R)-3-((R)-2-hydroxy-propoxy)-4-[4-(4-methoxy-butoxy)-phenyl]-5-[4-(3-methoxy-propyl)-3,4-dihydro-2H-benzo[1,4]oxazin-6-ylmethoxy]-piperidin-1-yl}-3-methyl-pentan-1-one
   starting from (R)-1-{(3S,4R,5R)-4-[4-(4-methoxy-butoxy)-phenyl]-5-[4-(3-methoxy-propyl)-3,4-dihydro-2H-benzo[1,4]oxazin-6-ylmethoxy]-piperidin-3-yloxy}-propan-2-ol (example 91 a).
107 (2S,3S)-2-Amino-1-{(3S,4R,5R)-3-hydroxymethyl-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-1-yl}-3-methyl-pentan-1-one
   starting from {(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-yl}-methanol (example 102a).
116 (2S,3S)-2-Amino-1-{(2R,4R,5R)-2-(2-hydroxy-3-methoxy-2-methyl-propyl)-4-(4-methoxy-phenyl)-5-[4-(3-methoxy-propyl)-3,4-dihydro-2H-benzo[1,4]oxazin-6-ylmethoxy]-piperidin-1-yl}-3-methyl-pentan-1-one (diastereomeric mixture)
   starting from (R,S)-1-methoxy-3-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2-methyl-propan-2-ol (diastereomeric mixture) (example 109a).
121 3-{(2S,4R,5R)-1-((2S,3S)-2-Amino-3-methyl-pentanoyl)-4-(4-methoxy-phenyl)-5-[4-(3-methoxy-propyl)-3,4-dihydro-2H-benzo[1,4]oxazin-6-ylmethoxy]-piperidin-2-yl}-2,2,N-trimethyl-propionamide
   starting from 3-{(2S,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2,2,N-trimethyl-propionamide (example 118a).
126 3-{(2S,4R,5R)-1-((2S,3S)-2-Amino-3-methyl-pentanoyl)-4-[4-(3-methoxy-propoxy)-phenyl]-5-[4-(3-methoxy-propyl)-3,4-dihydro-2H-benzo[1,4]oxazin-6-ylmethoxy]-piperidin-2-yl}-2,2,N-trimethyl-propionamide
   starting from 3-{(2S,4R,5R)-4-[4-(3-methoxy-propoxy)-phenyl]-5-[4-(3-methoxy-propyl)-3,4-dihydro-2H-benzo[1,4]oxazin-6-ylmethoxy]-piperidin-2-yl}-2,2,N-trimethyl-propionamide (example 123a).
131 3-{(2S,4R,5R)-1-((2S,3S)-2-Amino-3-methyl-pentanoyl)-4-[4-(2-methoxyethoxymethyl)-phenyl]-5-[4-(3-methoxy-propyl)-3,4-dihydro-2H-benzo[1,4]oxazin-6-ylmethoxy]-piperidin-2-yl}-2,2,N-trimethyl-propionamide
   starting from 3-{(2S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-2,2,N-trimethyl-propionamide (example 128a).
136 3-{(2R,4R,5R)-1-((2S,3S)-2-Amino-3-methyl-pentanoyl)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-2,2,N-trimethyl-propionamide
   starting from 3-{(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-2,2,N-trimethyl-propionamide (example 133a).
141 3-{(2R,4R,5R)-1-((2S,3S)-2-Amino-3-methyl-pentanoyl)-4-(4-methoxy-phenyl)-5-[4-(3-methoxy-propyl)-3,4-dihydro-2H-benzo[1,4]oxazin-6-ylmethoxy]-piperidin-2-yl]-2,2,N-trimethyl-propionamide
   starting from 3-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2,2,N-trimethyl-propionamide (example 138a).
146 N-(2-{(2R,4R,5R)-1-((2S,3S)-2-Amino-3-methyl-pentanoyl)-4-[4-(2-methoxyethoxymethyl)-phenyll-5-[4-(3-methoxy-propyl)-3,4-dihydro-2H-benzo[1,4]oxazin-6-ylmethoxy]-piperidin-2-yl}-1,1-dimethyl-ethyl)-acetamide
   starting from N-(2-{(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-1,1-dimethyl-ethyl)-acetamide (example 143a).
151 N-(2-{(2R,4R,5R)-1-((2S,3S)-2-Amino-3-methyl-pentanoyl)-4-(4-methoxy-phenyl)-5-[4-(3-methoxy-propyl)-3,4-dihydro-2H-benzo[1,4]oxazin-6-ylmethoxy]-piperidin-2-yl}-1,1-dimethyl-ethyl)-acetamide
   starting from N-(2-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-1,1-dimethyl-ethyl)-acetamide (example 148a).

### Example 9

### (3S,4S,5R)-3-Hydroxy-4-(4-methoxy-phenyl)-5-[4-(3-methoxy-propyl)-3,4-dihydro-2H-benzo[1,4]oxazin-6-ylmethoxy]-piperidine-1-carboxylic acid 1-isobutyryloxy-ethyl ester

To a stirred solution of 1 mmol of (3S,4S,5R)-3-hydroxy-4-(4-methoxy-phenyl)-5-[4-(3-methoxy-propyl)-3,4-dihydro-2H-benzo[1,4]oxazin-6-ylmethoxy]-piperidine-1-carboxylic acid 1-chloro-ethyl ester in 30 ml of N,N-dimethylformamide is added 5 mmol caesium carbonate, followed by 2 mmol of isobutyric acid. The reaction mixture is stirred for 12 hours at room temperature. The reaction mixture is diluted with water and extracted with tert-butyl methyl ether (4X). The combined organic phases are washed with brine, dried over sodium sulfate and the solvent is concentrated under reduced pressure. The residue is purified by flash chromatography (SiO₂ 60F) to afford the title compound, which is identified based on its
Rf value.

The starting material is prepared as follows:
a) (3S,4S,5R)-3-Hydroxy-4-(4-methoxy-phenyl)-5-[4-(3-methoxy-propyl)-3,4-dihydro-2H-benzo[1,4]oxazin-6-ylmethoxy]-piperidine-1-carboxylic acid 1-chloro-ethyl ester
   To a stirred solution of 1 mmol of (3S,4S,5R)-4-(4-methoxy-phenyl)-5-[4-(3-methoxy-propyl)-3,4-dihydro-2H-benzo[1,4]oxazin-6-ylmethoxy]-piperidin-3-ol [873945-55-2] in 20 ml of dichloromethane is added 3 mmol triethylamine. The solution is cooled to 0°C and 1.1 mmol of 1-chloroethyl chloroformate is added. The reaction mixture is stirred for 5 minutes at 0°C, then for 3 hours at room temperature. The reaction mixture is diluted with dichloromethane and washed with 1 M hydrochloric acid. The aqueous phase is re-extracted with dichloromethane (1 X). The combined organic phases are washed with 1 M aqueous sodium bicarbonate solution, dried over sodium sulfate and the solvent is concentrated under reduced pressure. The residue is purified by flash chromatography (SiO₂ 60F) to afford the title compound, which is identified based on its Rf value.

According to the procedure described in example 9, the following compounds are prepared in analogous manner:
18 (3S,4S,5R)-3-Hydroxy-4-{4-[3-(2-methoxy-benzyloxy)-propoxy]-phenyl}-5-[1-(3-methoxy-propyl)-3,3-dimethyl-2,3-dihydro-1H-indol-6-ylmethoxy]-piperidine-1-carboxylic acid 1-isobutyryloxy-ethyl ester
   starting from (3S,4S,5R)-4-{4-[3-(2-methoxy-benzyloxy)-propoxy]-phenyl}-5-[1-(3-methoxy-propyl)-3,3-dimethyl-2,3-dihydro-1H-indol-6-ylmethoxy]-piperidin-3-ol (example 12a).
27 (3S,4S,5R)-3-Hydroxy-4-{4-[3-(2-methoxy-benzyloxy)-propoxy]-phenyl}-5-[4-methoxy-3-(3-methoxy-propoxy)-benzyloxy]-piperidine-1-carboxylic acid 1-isobutyryloxy-ethyl ester
   starting from (3S,4S,5R)-4-{4-[3-(2-methoxy-benzyloxy)-propoxy]-phenyl}-5-[4-methoxy-3-(3-methoxy-propoxy)-benzyloxy]-piperidin-3-ol (example 21 a).
36 (3S,4S,5R)-4-{4-[3-(2-Chloro-phenoxy)-propoxyl-phenyl}-3-hydroxy-5-[4-(3-methoxy-propyl)-3,4-dihydro-2H-benzo[1,4]oxazin-6-ylmethoxy]-piperidine-1-carboxylic acid 1-isobutyryloxy-ethyl ester
   starting from (3S,4S,5R)-4-{4-[3-(2-chloro-phenoxy)-propoxy]-phenyl}-5-[4-(3-methoxy-propyl)-3,4-dihydro-2H-benzo[1,4]oxazin-6-ylmethoxy]-piperidin-3-ol [873945-66-5].
45 (3S,4S,5R)-3-Hydroxy-4-{4-[3-(2-methoxy-benzyloxy)-propoxy]-phenyl}-5-[4-(3-methoxy-propyl)-3,4-dihydro-2H-benzo[1,4]oxazin-6-ylmethoxy]-piperidine-1-carboxylic acid 1-isobutyryloxy-ethyl ester
   starting from (3S,4S,5R)-4-{4-[3-(2-methoxy-benzyloxy)-propoxy]-phenyl}-5-[4-(3-methoxy-propyl)-3,4-dihydro-2H-benzo[1,4]oxazin-6-ylmethoxy]-piperidin-3-ol [873945-50-7].
54 (3S,4S,5R)-4-[4-(4-Ethyl-phenoxy)-phenyl]-3-hydroxy-5-[4-(3-methoxy-propyl)-3,4-dihydro-2H-benzo[1,4]oxazin-6-ylmethoxy]-piperidine-1-carboxylic acid 1-isobutyryloxy-ethyl ester
   starting from (3S,4S,5R)-4-[4-(4-ethyl-phenoxy)-phenyl]-5-[4-(3-methoxy-propyl)-3,4-dihydro-2H-benzo[1,4]oxazin-6-ylmethoxy]-piperidin-3-ol (example 48a).
63 (3R,4S,5S)-3-{2-[2-(2-Acetylamino-ethyl)-phenoxy]-ethoxy}-5-hydroxy-4-{4-[3-(2-methoxy-benzyloxy)-propoxy]-phenyl}-piperidine-1-carboxylic acid 1-isobutyryloxy-ethyl ester
   starting from (N-(2-{2-[2-((3R,4S,5S)-5-hydroxy-4-{4-[3-(2-methoxy-benzyloxy)-propoxy]-phenyl}-piperidin-3-yloxy)-ethoxy]-phenyl}-ethyl)-acetamide (example 57a).
72 (3S,4S,5R)-4-{4-[(S)-1-(3-Fluoro-phenyl)-pyrrolidin-3-yloxyl-phenyl}-3-hydroxy-5-[4-(3-methoxy-propyl)-3,4-dihydro-2H-benzo[1,4]oxazin-6-ylmethoxyl-piperidine-1-carboxylic acid 1-isobutyryloxy-ethyl ester
   starting from (3S,4S,5R)-4-{4-[(S)-1-(3-fluoro-phenyl)-pyrrolidin-3-yloxy]-phenyl}-5-[4-(3-methoxy-propyl)-3,4-dihydro-2H-benzo[1,4]oxazin-6-ylmethoxy]-piperidin-3-ol [873945-18-7].
81 (3S,4R,5R)-3-((R)-2-Hydroxy-propoxy)-4-[4-(2-methoxy-ethoxymethyl)-phenyl]-5-[4-(3-methoxy-propyl)-3,4-dihydro-2H-benzo[1,4]oxazin-6-ylmethoxy]-piperidine-1-carboxylic acid 1-isobutyryloxy-ethyl ester
   starting from (R)-1-{(3S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-3-yloxy}-propan-2-ol (example 73a).
90 (3S,4R,5R)-3-((R)-2-Hydroxy-3-methoxy-propoxy)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidine-1-carboxylic acid 1-isobutyryloxy-ethyl ester
   starting from (R)-1-methoxy-3-{(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-yloxy}-propan-2-ol (example 82a).
99 (3S,4R,5R)-3-((R)-2-Hydroxy-propoxy)-4-[4-(4-methoxy-butoxy)-phenyl]-5-[4-(3-methoxy-propyl)-3,4-dihydro-2H-benzo[1,4]oxazin-6-ylmethoxy]-piperidine-1-carboxylic acid 1-isobutyryloxy-ethyl ester
   starting from (R)-1-{(3S,4R,5R)-4-[4-(4-methoxy-butoxy)-phenyl]-5-[4-(3-methoxy-propyl)-3,4-dihydro-2H-benzo[1,4]oxazin-6-ylmethoxy]-piperidin-3-yloxy}-propan-2-ol (example 91 a).
108 (3S,4R,5R)-3-Hydroxymethyl-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidine-1-carboxylic acid 1-isobutyryloxy-ethyl ester
   starting from {(3S,4R,5R)-4-[4-((S)-3-methoxy-2-methyl-propoxymethyl)-phenyl]-5-[4-(3-methoxy-propyl)-3,4-dihydro-2H-benzo[1,4]oxazin-6-ylmethoxy]-piperidin-3-yl}-methanol (example 102a).
117 (2R,4R,5R)-2-(2-Hydroxy-3-methoxy-2-methyl-propyl)-4-(4-methoxy-phenyl)-5-[4-(3-methoxy-propyl)-3,4-dihydro-2H-benzo[1,4]oxazin-6-ylmethoxy]-piperidine-1-carboxylic acid 1-isobutyryloxy-ethyl ester (diastereomeric mixture)
   starting from (R,S)-1-methoxy-3-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2-methyl-propan-2-ol (diastereomeric mixture) (example 109a).
122 (2S,4R,5R)-4-(4-Methoxy-phenyl)-5-[4-(3-methoxy-propyl)-3,4-dihydro-2H-benzo[1,4]oxazin-6-ylmethoxy]-2-(2-methyl-2-methylcarbamoyl-propyl)-piperidine-1-carboxylic acid 1-isobutyryloxy-ethyl ester
   starting from 3-{(2S,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2,2,N-trimethyl-propionamide (example 118a).
127 (2S,4R,5R)-4-[4-(3-Methoxy-propoxy)-phenyl]-5-[4-(3-methoxy-propyl)-3,4-dihydro-2H-benzo[1,4]oxazin-6-ylmethoxy]-2-(2-methyl-2-methylcarbamoyl-propyl)-piperidine-1-carboxylic acid 1-isobutyryloxy-ethyl ester
   starting from 3-{(2S,4R,5R)-4-[4-(3-methoxy-propoxy)-phenyl]-5-[4-(3-methoxy-propyl)-3,4-dihydro-2H-benzo[1,4]oxazin-6-ylmethoxy]-piperidin-2-yl}-2,2,N-trimethyl-propionamide (example 123a).
132 (2S,4R,5R)-4-[4-(2-Methoxy-ethoxymethyl)-phenyl]-5-[4-(3-methoxy-propyl)-3,4-dihydro-2H-benzo[1,4]oxazin-6-ylmethoxy]-2-(2-methyl-2-methylcarbamoyl-propyl)-piperidine-1-carboxylic acid 1-isobutyryloxy-ethyl ester
   starting from 3-{(2S,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-2,2,N-trimethyl-propionamide (example 128a).
137 (2R,4R,5R)-4-[4-(2-Methoxy-ethoxymethyl)-phenyl]-5-[4-(3-methoxy-propyl)-3,4-dihydro-2H-benzo[1,4]oxazin-6-ylmethoxyl-2-(2-methyl-2-methylcarbamoyl-propyl)-piperidine-1-carboxylic acid 1-isobutvryloxy-ethyl ester
   starting from 3-{(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-2,2,N-trimethyl-propionamide (example 133a).
142 (2R,4R,5R)-4-(4-Methoxy-phenyl)-5-[4-(3-methoxy-propyl)-3,4-dihydro-2H-benzo[1,4]oxazin-6-ylmethoxyl-2-(2-methyl-2-methylcarbamoyl-propyl)-piperidine-1-carboxylic acid 1-isobutyryloxy-ethyl ester
   starting from 3-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-2,2,N-trimethyl-propionamide (example 138a).
147 (2R,4R,5R)-2-(2-Acetylamino-2-methyl-propyl)-4-[4-(2-methoxy-ethoxymethyl)-phenyl]-5-[4-(3-methoxy-propyl)-3,4-dihydro-2H-benzo[1,4]oxazin-6-ylmethoxy]-piperidine-1-carboxylic acid 1-isobutyryloxy-ethyl ester
   starting from N-(2-{(2R,4R,5R)-4-[4-(2-methoxy-ethoxymethyl)-phenyl]-5-[4-(3-methoxy-propyl)-3,4-dihydro-2H-benzo[1,4]oxazin-6-ylmethoxy]-piperidin-2-yl}-1,1-dimethyl-ethyl)-acetamide (example 143a).
152 (2R,4R,5R)-2-(2-Acetylamino-2-methyl-propyl)-4-(4-methoxy-phenyl)-5-[4-(3-methoxy-propyl)-3,4-dihydro-2H-benzo[1,4]oxazin-6-ylmethoxy]-piperidine-1-carboxylic acid 1-isobutyryloxy-ethyl ester
   starting from N-(2-{(2R,4R,5R)-4-(4-methoxy-phenyl)-5-[4-(3-methoxy-propyl)-3,4-dihydro-2H-benzo[1,4]oxazin-6-ylmethoxy]-piperidin-2-yl}-1,1-dimethyl-ethyl)-acetamide (example 148a).

## Claims

1. A compound of the formula 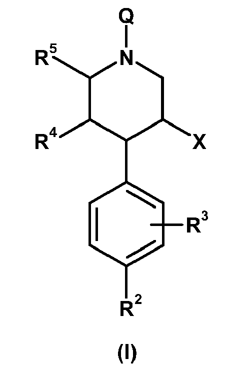 and salt thereof, preferably the pharmaceutically acceptable salt thereof; where
R¹ is aryl or heterocyclyl, especially benzoimidazolyl, benzo[1,3]dioxolyl, benzofuranyl, benzooxazolyl, benzothiazolyl, benzo[b]thienyl, 2H-chromenyl, dihydro-2H-benzo[1,4]oxazinyl, dihydro-3H-benzo[1,4]oxazinyl, dihydro-2H-benzo[1,4]thiazinyl, 2,3-dihydroindolyl, dihydro-1H-pyrido[2,3-b][1,4]oxazinyl, imidazo[1,2-a]pyridyl, imidazo[1,5-a]pyridyl, indazolyl, indolyl, isobenzofuranyl, isoquinolyl, [1,5]naphthyridyl, phenyl, phthalazinyl, pyridyl, pyrimidinyl, 1 H-pyrrolo[2,3-b]pyridyl, 1 H-pyrrolo[2,3-c]pyridyl, 1 H-pyrrolo[3,2-b]pyridyl, quinazolinyl, quinolyl, quinoxalinyl, tetrahydroimidazo[1,2-a]pyridyl, tetrahydroimidazo[1,5-a]pyridyl, tetrahydroisoquinolyl, tetrahydroquinolyl, tetrahydroquinoxalinyl, [1,2,3]triazolo[1,5-a]pyridyl or [1,2,4]triazolo[4,3-a]pyridyl, each of which is substituted by 1-4 acyl-C₁₋₈-alkoxy-C₁₋₈-alkoxy, acyl-C₁₋₈-alkoxy-C₁₋₈-alkyl, (N-acyl)-C₁₋₈-alkoxy-C₁₋₈-alkylamino, C₁₋₈-alkanoyl, C₁₋₈-alkoxy, C₁₋₈-alkoxy-C₁₋₈-alkanoyl, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, (N-C₁₋₈-alkoxy)-C₁₋₈-alkylaminocarbonyl-C₁₋₈-alkoxy, (N-C₁₋₈-alkoxy)-C₁₋₈-alkylaminocarbonyl-C₁₋₈-alkyl, C₁₋₈-alkoxy-C₁₋₈-alkyl-carbamoyl, C₁₋₈-alkoxy-C₁₋₈-alkyl-carbonylamino, C₁₋₈-alkoxy-C₁₋₈-alkyl-heterocyclyl, C₁₋₈-alkoxy-carbonyl, C₁₋₈-alkoxycarbonyl-C₁₋₈-alkoxy, C₁₋₈-alkoxycarbonyl-C₁₋₈-alkyl, C₁₋₈-alkoxycarbonylamino-C₂₋₈-alkoxy, C₁₋₈-alkoxycarbonylamino-C₁₋₈-alkyl, C₁₋₈-alkyl, (N-C₁₋₈-alkyl)-C₁₋₈-alkoxy-C₁₋₈-alkyl-carbamoyl, (N-C₁₋₈-alkyl)-C₁₋₈-alkoxy-C₁₋₈-alkylcarbonylamino, (N-C₁₋₈-alkyl)-C₁₋₈-alkoxy-carbonylamino, (N-C₁₋₈-alkyl)-C₁₋₈-alkylcarbonylamino-C₂₋₈-alkoxy, (N-C₁₋₈-alkyl)-C₁₋₈-alkylcarbonylamino-C₁₋₈-alkyl, (N-C₁₋₈-alkyl)-C₁₋₈-alkylsulfonylamino-C₂₋₈-alkoxy, (N-C₁₋₈-alkyl)-C₁₋₈-alkylsulfonylamino-C₁₋₈-alkyl, C₁₋₈-alkyl-amidinyl, C₁₋₈-alkylamino-C₂₋₈-alkoxy, di-C₁₋₈-alkylamino-C₂₋₈-alkoxy, C₁₋₈-alkylamino-C₁₋₈-alkyl, di-C₁₋₈-alkylamino-C₁₋₈-alkyl, C₁₋₈-alkylaminocarbonyl-C₁₋₈-alkoxy, di-C₁₋₈-alkylaminocarbonyl-C₁₋₈-alkoxy, C₁₋₈-alkylaminocarbonyl-C₁₋₈-alkoxy-C₁₋₈-alkyl, C₁₋₈-alkylaminocarbonyl-C₁₋₈-alkyl, di-C₁₋₈-alkylaminocarbonyl-C₁₋₈-alkyl, C₁₋₈-alkylaminocarbonylamino-C₂₋₈-alkoxy, C₁₋₈-alkylamino-carbonylamino-C₁₋₈-alkyl, C₁₋₈-alkylcarbonylamino, C₁₋₈-alkylcarbonylamino-C₂₋₈-alkoxy, C₁₋₈-alkylcarbonylamino-C₁₋₈-alkyl, C₁₋₈-alkylcarbonyloxy-C₂₋₈-alkoxy, C₁₋₈-alkylcarbonyloxy-C₁₋₈-alkyl, C₁₋₈-alkylsulfonyl, C₁₋₈-alkylsulfonyl-C₁₋₈-alkoxy, C₁₋₈-alkylsulfonyl-C₁₋₈-alkyl, C₁₋₈-alkylsulfonylamino-C₂₋₈-alkoxy, C₁₋₈-alkylsulfonylamino-C₁₋₈-alkyl, optionally N-mono- oder N,N-di-C₁₋₈-alkylated amino, aryl-C₀₋₈-alkoxy, aryl-C₀₋₈-alkyl, optionally N-mono- oder N,N-di-C₁₋₈-alkylated carbamoyl-C₀₋₈-alkoxy, carboxy-C₁₋₈-alkoxy, carboxy-C₁₋₈-alkoxy-C₁₋₈-alkyl, carboxy-C₁₋₈-alkyl, C₁₋₈-alkyl, cyano-C₂₋₈-alkoxy, cyano-C₁₋₈-alkyl, C₃₋₈-cycloalkyl-C₁₋₈-alkoxy, C₃₋₈-cycloalkyl-carbamoyl-C₀₋₈-alkyl, C₃₋₈-cycloalkylcarbonylamino-C₂₋₈-alkoxy, C₃₋₈-cycloalkyl-carbonylamino-C₁₋₈-alkyl, O,N-dimethylhydroxylamino-C₁₋₈-alkyl, halogen, halogen-C₁₋₈-alkoxy, halogen-C₁₋₈-alkyl, heterocydyl-C₀₋₈-alkoxy, heterocydyl-C₀₋₈-alkyl, heterocyclyl-carbonyl, hydroxy-C₂₋₈-alkoxy-C₁₋₈-alkoxy, hydroxy-C₂₋₈-alkoxy-C₁₋₈-alkyl, hydroxy-C₁₋₈-alkyl, O-methyloximyl-C₁₋₈-alkyl, oxide or oxo;
R² is C₂₋₈-alkenyloxy, C₂₋₈-alkenyloxy-C₁₋₈-alkoxy, C₂₋₈-alkenyloxy-C₁₋₈-alkyl, C₁₋₈-alkoxy, C₁₋₈-alkoxy-C₁₋₈-alkoxy, optionally halogen-substituted C₁₋₈-alkoxy-C₁₋₈-alkoxy-C₁₋₈-alkoxy, optionally halogen-substituted C₁₋₈-alkoxy-C₁₋₈-alkoxy-C₁₋₈-alkyl, C₁₋₈-alkoxy-C₁₋₈-alkyl, C₁₋₈-alkoxy-C₂₋₈-alkylamino-C₂₋₈-alkoxy, C₁₋₈-alkoxy-C₂₋₈-alkylamino-C₁₋₈-alkyl, C₁₋₈-alkoxy-C₀₋₈-alkyl-C₃₋₈-cycloalkyl-C₀₋₈-alkoxy-C₁₋₈-alkoxy, C₁₋₈-alkoxy-C₀₋₈-alkyl-C₃₋₈-cycloalkyl-C₀₋₈-alkoxy-C₁₋₈-alkyl, C₁₋₈-alkoxy-C₂₋₈-alkylsulfanyl, C₁₋₈-alkoxy-C₂₋₈-alkylsulfanyl-C₂₋₈-alkoxy, C₁₋₈-alkoxy-C₂₋₈-alkylsulfanyl-C₁₋₈-alkyl, C₁₋₈-alkyl, C₁₋₈-alkylsulfanyl-C₂₋₈-alkoxy, C₁₋₈-alkylsulfanyl-C₂₋₈-alkoxy-C₁₋₈-alkoxy, C₁₋₈-alkylsulfanyl-C₂₋₈-alkoxy-C₁₋₈-alkyl, C₁₋₈-alkylsulfanyl-C₁₋₈-alkyl, C₁₋₈-alkylsulfonyl-C₂₋₈-alkoxy-C₁₋₈-alkoxy, C₁₋₈-alkylsulfonyl-C₂₋₈-alkoxy-C₁₋₈-alkyl, aryl-C₀₋₈-alkoxy, aryl-C₀₋₈-alkoxy-C₁₋₈-alkoxy, aryl-C₀₋₈-alkoxy-C₁₋₈-alkyl, C₃₋₈-cycloalkyl-C₀₋₈-alkoxy-C₁₋₈-alkoxy, C₃₋₈-cycloalkyl-C₀₋₈-alkoxy-C₁₋₈-alkoxy-C₁₋₈-alkoxy, C₃₋₈-cycloalkyl-C₀₋₈-alkoxy-C₁₋₈-alkoxy-C₁₋₈-alkyl, C₃₋₈-cycloalkyl-C₀₋₈-alkoxy-C₁₋₈-alkyl, heterocyclyl-C₀₋₈-alkoxy, heterocyclyl-C₀₋₈-alkoxy-C₁₋₈-alkoxy, heterocyclyl-C₀₋₈-alkoxy-C₁₋₈-alkyl or heterocyclyl;
R³ is hydrogen or one or two halogen;
R⁴ is
(a) hydrogen
or, when R⁵ is hydrogen, is
(b) optionally halogen- and/or hydroxy-substituted C₁₋₈-alkoxy, optionally halogen- and/or hydroxy-substituted C₁₋₈-alkoxy-C₁₋₈-alkoxy, optionally N-mono- or N,N-di-C₁₋₈-alkylated amino-C₂₋₈-alkoxy, optionally N-mono- or N,N-di-C₁₋₈-alkylated or optionally hydroxy-substituted amino-C₀₋₈-alkylcarbonyl-C₁₋₈-alkoxy, hydroxy-C₁₋₈-alkylcarbonyl-C₀₋₈-alkoxy, C₁₋₈-alkoxy-C₀₋₈-alkylcarbonyl-C₀₋₈-alkoxy, optionally N-C₁₋₈-alkylated C₁₋₈-alkoxycarbonylamino-C₂₋₈-alkoxy, optionally N-C₁₋₈-alkylated C₁₋₈-alkoxy-C₂₋₈-alkylamino-C₂₋₈-alkoxy, optionally N-C₁₋₈-alkylated or optionally halogen-substituted C₁₋₈-alkylcarbonylamino-C₂₋₈-alkoxy, cyano-C₂₋₈-alkoxy, optionally N-C₁₋₈-alkylated or optionally halogen-substituted C₃₋₈-cycloalkyl-C₀₋₈-alkylcarbonylamino-C₂₋₈-alkoxy, optionally N-C₁₋₈-alkylated hydroxy-C₂₋₈-alkylamino-C₂₋₈-alkoxy, heterocyclylcarbonyl-C₀₋₈-alkylamino-C₂₋₈-alkoxy, C₁₋₈-alkoxycarbonylamino-C₂₋₈-alkoxy, optionally N-C₁₋₈-alkylated or optionally halogen-substituted heterocyclyl-C₀₋₈-alkylcarbonylamino-C₂₋₈-alkoxy, C₃₋₈-cycloalkyl-C₀₋₈-alkoxy, C₃₋₈-cycloalkyloxy-C₂₋₈-alkoxy, heterocyclyl-C₀₋₈-(optionally hydroxy-substituted)alkoxy, optionally N-C₁₋₈-alkylated heterocyclyl-C₀₋₈-alkylamino-C₀₋₈-alkylcarbonyl-C₀₋₈-alkoxy, C₁₋₈-alkylsulfonyl-C₁₋₈-alkoxy, C₃₋₈-alkynyloxy, heterocydyl-C₃₋₈-alkynyloxy, optionally N-mono- or N,N-di-C₁₋₈-alkylated amino-C₃₋₈-alkynyloxy, N-mono- or N,N-di-C₁₋₈-alkylated aminocarbonyl-C₃₋₈-alkynyloxy, heterocyclylcarbonyl-C₀₋₈-alkoxy, heterocydyloxy-C₂₋₈-alkoxy, optionally halogen- and/or hydroxy-substituted C₁₋₈-alkyl, optionally halogen- and/or hydroxy-substituted C₁₋₈-alkoxy-C₁₋₈-alkyl, optionally N-mono- or N,N-di-C₁₋₈-alkylated amino-C₁₋₈-alkyl, optionally N-mono- or N,N-di-C₁₋₈-alkylated or optionally hydroxy-substituted amino-C₀₋₈-alkylcarbonyl-C₁₋₈-alkyl, hydroxy-C₁₋₈-alkylcarbonyl-C₀₋₈-alkyl, C₁₋₈-alkoxy-C₀₋₈-alkylcarbonyl-C₀₋₈-alkyl, optionally N-C₁₋₈-alkylated C₁₋₈-alkoxycarbonylamino-C₁₋₈-alkyl, optionally N-C₁₋₈-alkylated C₁₋₈-alkoxy-C₂₋₈-alkylamino-C₁₋₈-alkyl, optionally N-C₁₋₈-alkylated or optionally halogen-substituted C₁₋₈-alkylcarbonylamino-C₁₋₈-alkyl, cyano-C₁₋₈-alkyl, optionally N-C₁₋₈-alkylated or optionally halogen-substituted C₃₋₈-cycloalkyl-C₀₋₈-alkylcarbonylamino-C₁₋₈-alkyl, optionally N-C₁₋₈-alkylated hydroxy-C₂₋₈-alkylamino-C₁₋₈-alkyl, heterocyclylcarbonyl-C₀₋₈-alkylamino-C₁₋₈-alkyl, C₁₋₈-alkoxycarbonylamino-C₁₋₈-alkyl, optionally N-C₁₋₈-alkylated or optionally halogen-substituted heterocyclyl-C₀₋₈-alkylcarbonylamino-C₁₋₈-alkyl, C₃₋₈-cycloalkyl-C₀₋₈-alkyl, C₃₋₈-cycloalkyloxy-C₁₋₈-alkyl, heterocyclyl-C₀₋₈-(optionally hydroxy-substituted)alkyl, optionally N-C₁₋₈-alkylated heterocydyl-C₀₋₈-alkylamino-C₀₋₈-alkylcarbonyl-C₀₋₈-alkyl, C₁₋₈-alkylsulfonyl-C₁₋₈-alkyl, C₂₋₈-alkynyl, heterocydyl-C₂₋₈-alkynyl, optionally N-mono- or N,N-di-C₁₋₈-alkylated amino-C₂₋₈-alkynyl, N-mono- or N,N-di-C₁₋₈-alkylated aminocarbonyl-C₂₋₈-alkynyl, heterocyclylcarbonyl-C₀₋₈-alkyl or heterocydyloxy-C₁₋₈-alkyl,
whereby
each of the residues mentioned above for R⁴ bearing a hydroxy-group is optionally substituted at that hydroxy-group by a radical (A) whereby an ester bond is formed;
R⁵ is
(a) hydrogen
or, when R⁴ is hydrogen, is
(b) C₂₋₈-alkenyl, C₁₋₈-alkyl, C₀₋₈-alkyl-carbonyl-amino-C₁₋₈-alkyl, C₁₋₈-alkyl-sulfonyl-C₁₋₈-alkyl, C₂₋₈-alkynyl, optionally N-mono- or N,N-di-C₁₋₈-alkylated carbamoyl-C₀₋₈-alkyl, optionally O-C₁₋₈-alkylated carboxyl-C₀₋₈-alkyl, C₃₋₈-cycloalkyl-C₀₋₈-alkyl, heterocyclylcarbonyl-C₀₋₈-alkyl or optionally N and/or N' mono-, di- or tri-C₁₋₈-alkylated ureido-C₁₋₈-alkyl, each of said radicals may be substituted, preferably by 1-4 C₁₋₈-alkoxy, C₁₋₈-alkoxy-C₁₋₈-alkoxy, C₁₋₈-alkoxycarbonyl-(N-C₁₋₈-alkyl)-amino, C₁₋₈-alkyl, C₁₋₈-alkyl-carbonyl-(N-C₁₋₈-alkyl)-amino, C₁₋₈-alkyl-sulfanyl, C₁₋₈-alkyl-sulfinyl, aryl-C₀₋₈-alkoxy, which is optionally substituted by 1 or 2 aryl or C₁₋₈-alkoxy radicals, aryl, which is optionally substituted by 1 or 2 aryl or C₁₋₈-alkoxy radicals, aryl-amino, cyano, C₃₋₈-cycloalkoxy, halogen, heterocyclyl-C₀₋₈-alkyl, heterocyclyl-C₀₋₈-alkoxy, heterocyclyl-C₀₋₈-alkyl-amino, heterocyclyl-carbonyl, hydroxyl, optionally N-mono-or N,N-di-C₁₋₈-alkylated amino, optionally N-mono- or N,N-di-C₁₋₈-alkylated carbamoyl-C₀₋₈-alkyl, optionally N-mono- or N,N-di-C₁₋₈-alkylated carbamoyloxy, optionally N-mono- or N,N-di-C₁₋₈-alkylated sulfamoyl, optionally N-arylated or N-heterocyclyl-substituted carbamoyl, oxo, trifluoromethoxy or trifluoromethyl,
whereby
each of the residues mentioned above for R⁵ bearing a hydroxy-group are optionally substituted at that hydroxy-group by a radical (A) whereby an ester bond is formed;
R⁶ is hydrogen or C₁₋₈-alkyl;
R⁷ is optionally carboxy- or hydroxy-substituted C₁₋₈-alkyl or optionally carboxy- or hydroxy-substituted aryl-C₀₋₈-alkyl;
R⁸ is C₁₋₈-alkyl;
X is -Alk-R¹, -O-Alk-R¹, -Alk-O-R¹, -O-Alk-O-R¹, -S-Alk-R¹, -Alk-S-R¹, -Alk-NR⁶-R¹, -NR⁶-Alk-R¹, -C(O)-NR⁶-R¹, -Alk-C(O)-NR⁶-R¹, -C(O)-NR⁶-Alk-R¹, -Alk-C(O)-NR⁶-Alk-R¹, -NR⁶-C(O)-R¹, -Alk-NR⁶-C(O)-R¹, -NR⁶-C(O)-Alk-R¹, -Alk-NR⁶-C(O)-Alk-R¹, -O-Alk-C(O)-NR⁶-R¹, -O-Alk-NR⁶-C(O)-R¹, -S(O)₂-NR⁶-R¹, -Alk-S(O)₂-NR⁶-R¹, -S(O)₂-NR⁶-Alk-R¹, -Alk-S(O)₂-NR⁶-Alk-R¹, - NR⁶-S(O)₂-R¹, -Alk-NR⁶-S(O)₂-R¹, -NR⁶-S(O)₂-Alk-R¹ or -Alk-NR⁶-S(O)₂-Alk-R¹, where Alk represents C₁₋₈-alkylene, which is optionally substituted with halogen;
Q is hydrogen or represents a radical (A) whereby an amide bond is formed or a group of the formula -C(O)OCHR⁷OC(O)R⁸;
(A) represents a mono- or di-peptidic residue of one or two of the 20 natural amino acids, said residue being formally obtained from the mono- or di-peptide by elimination of the OH-group from the C-terminal end;
and whereby
(i) a radical (A) is present in at least one of R⁴, R⁵ or Q; or else
(ii) at least Q is a group of the formula -C(O)OCHR⁷OC(O)R⁸.

2. A compound according to claim 1 of the general formula 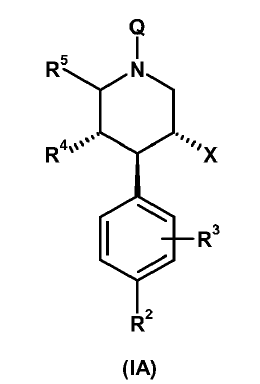 and the salts thereof, preferably the pharmaceutically acceptable salts thereof;
in which R¹, R², R³, R⁴, R⁵, Q and X have the meaning given in claim 1 for the compounds of the formula (I).

3. A compound accordng to claim 1 or 2 where
R¹ is aryl or heterocyclyl;
R² is C₂₋₈-alkenyloxy, C₁₋₈-alkoxy, C₁₋₈-alkoxy-C₁₋₈-alkoxy, C₁₋₈-alkoxy-C₁₋₈-alkyl, C₁₋₈-alkoxy-C₀₋₈-alkyl-C₃₋₈-cycloalkyl-C₀₋₈-alkoxy-C₁₋₈-alkoxy, C₁₋₈-alkoxy-C₀₋₈-alkyl-C₃₋₈-cycloalkyl-C₀₋₈-alkoxy-C₁₋₈-alkyl, C₁₋₈-alkoxy-C₂₋₈-alkylsulfanyl, C₁₋₈-alkoxy-C₂₋₈-alkylsulfanyl-C₂₋₈-alkoxy, C₁₋₈-alkoxy-C₂₋₈-alkylsulfanyl-C₁₋₈-alkyl, C₁₋₈-alkyl, C₁₋₈-alkylsulfanyl-C₂₋₈-alkoxy, C₁₋₈-alkylsulfanyl-C₂₋₈-alkoxy-C₁₋₈-alkoxy, C₁₋₈-alkylsulfanyl-C₂₋₈-alkoxy-C₁₋₈-alkyl, C₁₋₈-alkylsulfanyl-C₁₋₈-alkyl, aryl-C₀₋₈-alkoxy, aryl-C₀₋₈-alkoxy-C₁₋₈-alkoxy, aryl-C₀₋₈-alkoxy-C₁₋₈-alkyl, C₃₋₈-cycloalkyl-C₀₋₈-alkoxy-C₁₋₈-alkoxy, C₃₋₈-cycloalkyl-C₀₋₈-alkoxy-C₁₋₈-alkoxy, C₃₋₈-cycloalkyl-C₀₋₈-alkoxy-C₁₋₈-alkoxy-C₁₋₈-alkyl, C₃₋₈-cycloalkyl-C₀₋₈-alkoxy-C₁₋₈-alkyl, heterocydyl-C₀₋₈-alkoxy, heterocyclyl-C₀₋₈-alkoxy-C₁₋₈-alkoxy or heterocydyl-C₀₋₈-alkoxy-C₁₋₈-alkyl;
R³ is hydrogen or 1-2 halogen;
R⁴ is
(a) hydrogen
or, when R⁵ is hydrogen, is
(b) is optionally halogen- and/or hydroxy-substituted C₁₋₈-alkoxy, optionally halogen- and/or hydroxy-substituted C₁₋₈-alkoxy-C₁₋₈-alkoxy, optionally N-mono- or N,N-di-C₁₋₈-alkylated amino-C₂₋₈-alkoxy, optionally N-mono- or N,N-di-C₁₋₈-alkylated or optionally hydroxy-substituted amino-C₀₋₈-alkylcarbonyl-C₁₋₈-alkoxy, C₃₋₈-cycloalkyl-C₀₋₈-alkoxy, C₃₋₈-cyclo-alkyloxy-C₂₋₈-alkoxy, heterocyclyl-C₀₋₈-(optionally hydroxy-substituted)alkoxy, optionally N-C₁₋₈-alkylated heterocydyl-C₀₋₈-alkylamino-C₀₋₈-alkylcarbonyl-C₀₋₈-alkoxy, C₃₋₈-alkynyloxy, heterocyclylcarbonyl-C₀₋₈-alkoxy, heterocydyloxy-C₂₋₈-alkoxy, optionally halogen- and/or hydroxy-substituted C₁₋₈-alkyl, optionally halogen- and/or hydroxy-substituted C₁₋₈-alkoxy-C₁₋₈-alkyl, optionally N-mono- or N,N-di-C₁₋₈-alkylated amino-C₁₋₈-alkyl, optionally N-mono- or N,N-di-C₁₋₈-alkylated or optionally hydroxy-substituted amino-C₀₋₈-alkylcarbonyl-C₁₋₈-alkyl, optionally N-C₁₋₈-alkylated C₁₋₈-alkoxycarbonylamino-C₁₋₈-alkyl, optionally N-C₁₋₈-alkylated or optionally halogen-substituted C₁₋₈-alkylcarbonylamino-C₁₋₈-alkyl, optionally N-C₁₋₈-alkylated or optionally halogen-substituted C₃₋₈-cycloalkyl-C₀₋₈-alkylcarbonylamino-C₁₋₈-alkyl, C₁₋₈-alkoxycarbonylamino-C₁₋₈-alkyl, optionally N-C₁₋₈-alkylated or optionally halogen-substituted heterocydyl-C₀₋₈-alkylcarbonylamino-C₁₋₈-alkyl, C₃₋₈-cycloalkyl-C₀₋₈-alkyl, C₃₋₈-cycloalkyloxy-C₁₋₈-alkyl, heterocyclyl-C₀₋₈-(optionally hydroxy-substituted)alkyl, optionally N-C₁₋₈-alkylated heterocydyl-C₀₋₈-alkylamino-C₀₋₈-alkylcarbonyl-C₀₋₈-alkyl, heterocyclylcarbonyl-C₀₋₈-alkyl or heterocydyloxy-C₁₋₈-alkyl,
whereby
each of the residues, mentioned above for R⁴, bearing a hydroxy-group is optionally substituted at that hydroxy-group by a radical (A) whereby an ester bond is formed;
R⁵ is
(a) hydrogen
or, when R⁴ is hydrogen, is
(b) C₁₋₈-alkyl, C₀₋₈-alkyl-carbonyl-amino-C₁₋₈-alkyl, optionally N-mono- or N,N-di-C₁₋₈-alkylated carbamoyl-C₀₋₈-alkyl, C₃₋₈-cycloalkyl-C₀₋₈-alkyl, heterocydylcarbonyl-C₀₋₈-alkyl or optionally N and/or N' mono-, di- or tri-C₁₋₈-alkylated ureido-C₁₋₈-alkyl, each of said radicals may be substituted, preferably by 1-4 C₁₋₈-alkoxy, C₁₋₈-alkoxy-C₁₋₈-alkoxy, C₁₋₈-alkoxycarbonyl-(N-C_{1- 8}-alkyl)-amino, C₁₋₈-alkyl, C₁₋₈-alkyl-carbonyl-(N-C₁₋₈-alkyl)-amino, C₁₋₈-alkyl-sulfanyl, C₁₋₈-alkyl-sulfinyl, aryl-C₀₋₈-alkoxy, which is optionally substituted by 1 or 2 aryl or C₁₋₈-alkoxy radicals, aryl, which is optionally substituted by 1 or 2 aryl or C₁₋₈-alkoxy radicals, aryl-amino, cyano, C₃₋₈-cycloalkoxy, halogen, heterocydyl-C₀₋₈-alkyl, heterocydyl-C₀₋₈-alkoxy, heterocydyl-C₀₋₈-alkyl-amino, heterocyclyl-carbonyl, hydroxyl, optionally N-mono- or N,N-di-C₁₋₈-alkylated amino, optionally N-mono- or N,N-di-C₁₋₈-alkylated carbamoyl-C₀₋₈-alkyl, optionally N-mono- or N,N-di-C₁₋₈-alkylated carbamoyloxy or optionally N-arylated or N-heterocyclyl-substituted carbamoyl,
whereby
each of the residues, mentioned above for R⁵, bearing a hydroxy-group is optionally substituted at that hydroxy-group by a radical (A) whereby an ester bond is formed;
R⁶ is hydrogen or C₁₋₈-alkyl;
R⁷ is optionally carboxy- or hydroxy-substituted C₁₋₈-alkyl or optionally carboxy- or hydroxy-substituted aryl-C₀₋₈-alkyl;
R⁸ is C₁₋₈-alkyl;
(A) represents a mono- or di-peptidic residue of one or two of the 20 natural amino acids, said residue being formally obtained from the mono- or di-peptide by elimination of the OH-group from the C-terminal end;
X is -Alk-R¹, -O-Alk-R¹, -O-Alk-O-R¹, -C(O)-NR⁶-R¹, -Alk-C(O)-NR⁶-R¹, -C(O)-NR⁶-Alk-R¹, - Alk-C(O)-NR⁶-Alk-R¹, -Alk-NR⁶-C(O)-R¹, -Alk-NR⁶-C(O)-Alk-R¹, -O-Alk-C(O)-NR⁶-R¹, -O-Alk-NR⁶-C(O)-R¹, -Alk-S(O)₂-NR⁶-R¹, -S(O)₂-NR⁶-Alk-R¹, -Alk-S(O)₂-NR⁶-Alk-R¹, -Alk-NR⁶-S(O)₂-R¹ or -Alk-NR⁶-S(O)₂Alk-R¹, where Alk represents C₁₋₈-alkylene, which is optionally substituted with halogen;
Q is hydrogen or represents a radical (A); or a group of the formula -C(O)OCHR⁷OC(O)R⁸; and whereby
(i) a radical (A) is present in at least one of R⁴, R⁵ or Q; or else
(ii) at least Q is a group of the formula -C(O)OCHR⁷OC(O)R⁸.

4. A compound according to any one of claims 1 to 3 where
R¹ is phenyl substituted by 1-3 radicals selected from C₁₋₈-alkanoyl, C₁₋₈-alkoxy, C₁₋₈-alkoxy-C₁₋₈-alkanoyl, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkoxycarbonyl-C₁₋₈-alkoxy, C₁₋₈-alkoxycarbonyl-C₁₋₈-alkyl, C₁₋₈-alkoxycarbonylamino-C₂₋₈-alkoxy, C₁₋₈-alkoxycarbonylamino-C₁₋₈-alkyl, C₁₋₈-alkyl, (N-C₁₋₈-alkyl)-C₁₋₈-alkylcarbonylamino-C₂₋₈-alkoxy, (N-C₁₋₈-alkyl)-C₁₋₈-alkylcarbonylamino-C₁₋₈-alkyl, C₁₋₈-alkylaminocarbonyl-C₁₋₈-alkoxy, di-C₁₋₈-alkylaminocarbonyl-C₁₋₈-alkoxy, C₁₋₈-alkylaminocarbonyl-C₁₋₈-alkoxy-C₁₋₈-alkyl, C₁₋₈-alkylaminocarbonyl-C₁₋₈-alkyl, di-C₁₋₈-alkylaminocarbonyl-C₁₋₈-alkyl, C₁₋₈-alkyl-aminocarbonylamino-C₂₋₈-alkoxy, C₁₋₈-alkylaminocarbonylamino-C₁₋₈-alkyl, C₁₋₈-alkylcarbonylamino-C₂₋₈-alkoxy, C₁₋₈-alkylcarbonylamino-C₁₋₈-alkyl, C₁₋₈-alkylsulfonyl-C₁₋₈-alkoxy, C₁₋₈-alkylsulfonyl-C₁₋₈-alkyl, C₁₋₈-alkylsulfonylamino-C₂₋₈-alkoxy, C₁₋₈-alkylsulfonylamino-C₁₋₈-alkyl, aryl-C₀₋₈-alkoxy, aryl-C₀₋₈-alkyl, optionally N-mono- oder N,N-di-C₁₋₈-alkylated carbamoyl-C₀₋₈-alkoxy, carboxy-C₁₋₈-alkoxy, carboxy-C₁₋₈-alkoxy-C₁₋₈-alkyl, carboxy-C₁₋₈-alkyl, C₁₋₈-alkyl, cyano, cyano-C₂₋₈-alkoxy, cyano-C₁₋₈-alkyl, C₃₋₈-cycloalkyl-C₁₋₈-alkoxy, C₃₋₈-cycloalkyl-carbamoyl-C₀₋₈-alkyl, C₃₋₈-cycloalkylcarbonylamino-C₂₋₈-alkoxy, C₃₋₈-cycloalkylcarbonylamino-C₁₋₈-alkyl, halogen, halogen-C₁₋₈-alkoxy, halogen-C₁₋₈-alkyl, heterocydyl-C₀₋₈-alkoxy, heterocydyl-C₀₋₈-alkyl, hydroxy-C₂₋₈-alkoxy-C₁₋₈-alkoxy or hydroxy-C₂₋₈-alkoxy-C₁₋₈-alkyl.

5. A compound according to any one of claims 1 to 3 where R¹ is benzofuranyl, benzoimidazolyl, 4H-benzo[1,4]oxazinyl, benzooxazolyl, 2- or 5-benzo[b]thienyl, 3,4-dihydro-2H-benzo[1,4]oxazinyl, 1,3-dihydroindolyl, 2,3-dihydroindolyl, indazolyl, indolyl,1H-quinolinyl, 2H-chromenyl, 1,1a,2,7b-tetrahydro-cyclopropa[c]chromenyl, 1a,7b-dihydro-1H-cyclopropa[c]chromenyl, 6- or 7-isoquinolyl, pyridyl, pyrimidinyl, 6- or 7-quinazolinyl, 6- or 7-quinolyl, 6-quinoxalinyl, 6- or 7-tetrahydroisoquinolyl or 6- or 7-tetrahydroquinolyl, each of which is substituted by 1-3 radicals selected from C₁₋₈-alkanoyl, C₁₋₈-alkoxy, C₁₋₈-alkoxy-C₁₋₈-alkanoyl, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkoxycarbonyl-C₁₋₈-alkoxy, C₁₋₈-alkoxycarbonyl-C₁₋₈-alkyl, C₁₋₈-alkoxycarbonylamino-C₂₋₈-alkoxy, C₁₋₈-alkoxycarbonylamino-C₁₋₈-alkyl, C₁₋₈-alkyl, (N-C₁₋₈-alkyl)-C₁₋₈-alkylcarbonylamino-C₂₋₈-alkoxy, (N-C₁₋₈-alkyl)-C₁₋₈-alkylcarbonylamino-C₁₋₈-alkyl, C₁₋₈-alkylaminocarbonyl-C₁₋₈-alkoxy, di-C₁₋₈-alkylaminocarbonyl-C₁₋₈-alkoxy, C₁₋₈-alkylaminocarbonyl-C₁₋₈-alkoxy-C₁₋₈-alkyl, C₁₋₈-alkylaminocarbonyl-C₁₋₈-alkyl, di-C₁₋₈-alkylaminocarbonyl-C₁₋₈-alkyl, C₁₋₈-alkylamino-carbonylamino-C₂₋₈-alkoxy, C₁₋₈-alkylaminocarbonylamino-C₁₋₈-alkyl, C₁₋₈-alkylcarbonylamino-C₂₋₈-alkoxy, C₁₋₈-alkylcarbonylamino-C₁₋₈-alkyl, C₁₋₈-alkylsulfonyl-C₁₋₈-alkoxy, C₁₋₈-alkylsulfonyl-C₁₋₈-alkyl, C₁₋₈-alkylsulfonylamino-C₂₋₈-alkoxy, C₁₋₈-alkylsulfonylamino-C₁₋₈-alkyl, aryl-C₀₋₈-alkoxy, aryl-C₀₋₈-alkyl, optionally N-mono- oder N,N-di-C₁₋₈-alkylated carbamoyl-C₀₋₈-alkoxy, carboxy-C₁₋₈-alkoxy, carboxy-C₁₋₈-alkoxy-C₁₋₈-alkyl, carboxy-C₁₋₈-alkyl, C₁₋₈-alkyl, cyano, cyano-C₂₋₈-alkoxy, cyano-C₁₋₈-alkyl, C₃₋₈-cycloalkyl-C₁₋₈-alkoxy, C₃₋₈-cycloalkyl-carbamoyl-C₀₋₈-alkyl, C₃₋₈-cycloalkylcarbonylamino-C₂₋₈-alkoxy, C₃₋₈-cycloalkylcarbonylamino-C₁₋₈-alkyl, halogen, halogen-C₁₋₈-alkoxy, halogen-C₁₋₈-alkyl, heterocydyl-C₀₋₈-alkoxy, heterocydyl-C₀₋₈-alkyl, hydroxy-C₂₋₈-alkoxy-C₁₋₈-alkoxy, hydroxy-C₂₋₈-alkoxy-C₁₋₈-alkyl, oxide or oxo.

6. A compound according to any one of claims 1 to 3 where
R¹ is phenyl, 3,4-dihydro-2H-benzo[1,4]oxazin-6-yl or 3,3-dimethyl-1,3-dihydroindol-6-yl, which is substituted as specified in claim 1, especially by at least one substituent selected from C₁₋₈-alkoxy, C₁₋₈-alkoxy-C₁₋₈-alkoxy, C₁₋₈-alkoxy-C₁₋₈-alkyl, C₁₋₈-alkyl, C₀₋₆-alkylcarbonylamino-C₁₋₈-alkoxy, C₀₋₆-alkylcarbonylamino-C₁₋₈-alkyl and halogen, and, preferably, where, in the case that R¹ is a bicyclic heterocyclyl, the phenyl-ring of the bicyclic system is bonded to the rest of the molecule and at least the non-phenyl-ring is substituted as specified.

7. A compound according to any one of claims 1 to 6 where
X is -Alk-R¹, -O-Alk-, -O-Alk-O-R¹, -C(O)-NR⁶-R¹ or -O-Alk-C(O)-NR⁶-R¹, where Alk represents C₁₋₈-alkylene, which is optionally substituted with halogen.

8. A process for enhancing the membrane transportation of compounds of formula (I), or preferably of the formula (IA), and their pharmaceutically useable salts according to any one of claims 1 to 6 **characterized in that**
(i) a radical (A) is present in at least one of R⁴, R⁵ or Q; or else
(ii) at least Q is a group of the formula -C(O)OCHR⁶OC(O)R⁷.

9. Use of a compound according to any one of claims 1 to 6 for the preparation of a medication, in particular a medication for the treatment or prevention of high blood pressure, heart failure, glaucoma, myocardial infarction, renal failure, restenoses and stroke.

10. A pharmaceutical composition comprising a compound according to any one of claims 1 to 6 and optionally one or more agents having cardiovascular activity.
